# EUROPEAN PATENT APPLICATION

(11) **EP 3 251 662 A1**
(43) Date of publication of application: **06.12.2017**
(21) Application number: 16001501.2
(22) Date of filing: 06.07.2016
(51) Int. Cl.: A61K 9/51, A61K 39/00, A61K 38/17, A61P 37/06, A61P 37/08

(54) **MATRIX-EMBEDDED TOLERANCE-PROMOTION ADJUVANTS FOR SUBCUTANEOUS IMMUNOTHERAPY**

(30) Priority: 03.06.2016 EP 16001276
(71) Applicant: Tolerogenics S.à.r.l., 4354 Esch-sur-Alzette (LU); Luxembourg Institute of Health (LIH), 4354 Esch-sur-Alzette (LU)
(72) Inventor: Bredehorst, Reinhard, 20255 Hamburg (DE); Grunwald, Thomas, 22459 Hamburg (DE); Ollert, Markus, 2441 Luxembourg (LU)
(74) Representative: Jungblut & Seuss

(57) **Abstract**

The present invention relates to compositions and methods for the application of tolerance-promoting adjuvants embedded in alum-, microcrystalline tyrosine-, or hydrogel-based formulations for allergen- or autoantigen-specific immunotherapy.

## Description

### Field of the invention

The present invention relates to compositions and methods for the application of tolerance-promoting adjuvants embedded in alum-, microcrystalline tyrosine-, or hydrogel-based formulations for allergen- or autoantigen-specific immunotherapy.

### Background of the invention and state of the art

For the treatment of allergic diseases and autoimmune diseases such as type I diabetes, rheumatoid arthritis, and multiple sclerosis, allergen- or autoantigen-specific immunotherapy has the potential of restoring lasting immunological tolerance, but supporting tolerance-promoting strategies are needed to increase the therapeutic efficacy of this approach.

One promising approach is the application of tolerance-promoting adjuvants. However, currently available adjuvants such as oils and alum trigger either Th1-type or Th2-type immune responses or both (for reviews, see Leroux-Roels, 2010; Nicholls et al., 2010). Based on the assumption that the induction of tolerance requires T-cell receptor activation in the absence of decision signals for effector T cells, currently available adjuvants are not suitable for the induction of tolerance. Adjuvants are needed that support the induction of tolerance-promoting regulatory T cells (Tregs) and tolerogenic antigen-presenting cells including tolerogenic dendritic cells (DCs) and tolerogenic macrophages.

Tolerance induction by Tregs. The term Treg encompasses at least 5 subsets of cell populations including a) natural thymus-derived CD4(+)CD25(+)Foxp3(+) nTregs producing IL-10 and TGF-β, b) CD8 (+) CD25 (+) Foxp3 (+) Tregs producing IL-10, IL-17 and IFN-γ, c) peripherally induced CD4(+)Foxp3(+) iTregs producing IL-10 and TGF-β (IL-10- and TGF-β-producing Th3 cells belong to this subset), d) inducible CD4(+)CD25(+)-Foxp3(-) Tr1 cells producing IL-10, and e) CD4(+)Foxp3(+) Tregs producing IL-17 (for review, see Zhang et al., 2014). In addition to Tregs, a newly described population of regulatory B cells (Bregs) producing IL-10 and/or TGF-β also contributes to immunosuppression both directly and via enhancement of Treg function (for a review, see Grant et al., 2015).

Numerous studies have demonstrated that control of the development, survival, and function of Tregs is instrumental for effective control of immune responses and the induction of tolerance. Either low Treg numbers with normal function or normal Treg numbers with compromised suppressive function have been made responsible for allergic and autoimmune diseases. For example, allergic patients exhibit a deficit of allergen-specific IL-10 producing T cells in the peripheral blood (for a review, see Schmidt-Weber and Blaser, 2006). Therefore, the main principle of allergen-specific immunotherapy in allergic patients is to increase the number of Tregs capable of controlling allergen-specific effector T cells. Both nTregs and inducible Tr1 cells inhibit the development of allergy via several mechanisms, including suppression of effector Th1, Th2, and Th17 cells, suppression of eosinophils, mast cells and basophils, induction of antibody isotype change from IgE to IgG4, suppression of inflammatory dendritic cells (DC), and suppression of inflammatory cell migration to tissues (for a review, see Palomares et al., 2010).

The induction of Treg-suppressive activity is specific and requires allergenic or antigenic stimulation through the T cell receptor (TCR). The suppressive activity of Tregs, however, is not antigen- or allergen-specific. Therefore, a wide range of immune responses can be inhibited by Tregs via 'bystander' suppression.

The efficacy of bystander suppression has been demonstrated in several animal models in which transfer of antigen- or allergen-specific regulatory T cells suppressed or markedly reduced clinical signs of various allergic and autoimmune disease including murine models of allergic airway inflammation (Kearley et al., 2005; Xu et al., 2012) and murine models of various autoimmune diseases such as experimental autoimmune encephalomyelitis (EAE) (Kohm et al., 2002), experimental collagen-induced arthritis (CIA) (Morgan et al., 2005; Kelchtermans et al., 2009; Kong et al., 2012), experimental autoimmune diabetes (Tang et al., 2004; Jaeckel et al., 2005), experimental renal disease in lupus-prone NZB/NZW mice (Scalapino et al., 2006), and experimental inflammatory bowel disease (Mottet et al., 2003).

Tolerance induction by tolerogenic DCs. As important for the induction of tolerance are antigen-presenting cells (APCs) including dendritic cells (DCs) and macrophages. Both APCs are indispensable in the regulation of the delicate balance between immunity and tolerance. For example, DCs can prevent autoimmunity by inducing apoptosis of autoreactive T cells in the thymus (central tolerance) and by induction of anergy, deletion, or tolerance through cooperation with regulatory T cells (Treg) in the periphery (peripheral tolerance). Recent investigations have shown that antigen-specific tolerance can be induced in vivo via vaccination with antigen-pulsed ex vivo generated DCs, so called tolerogenic DCs (tolDCs). Such tolDCs are phenotypically immature DCs characterized by a low expression of MHC-II as well as co-stimulatory molecules such as CD40, CD80, CD86, and a reduced production of pro-inflammatory IL-12 and increased secretion of anti-inflammatory IL-10 (for reviews, see Thomas, 2013; Gross and Wiendl, 2013; Van Brussel et al., 2014; Mackern-Oberti et al., 2015).

The efficacy of vaccination with antigen-pulsed ex vivo generated tolDCs has been demonstrated in several animal models of autoimmune diseases including experimental collagen-induced arthritis (CIA) (Chorny et al., 2005; van Duivenvoorde et al., 2007; Stoop et al., 2010), experimental antigen-induced arthritis (AIA) (Martin et al., 2007), experimental autoimmune encephalomyelitis (EAE) (Xiao et a., 2004; Toscano et al., 2010; Papenfuss et al. 2011; Matsuda et al., 2012), and experimental autoimmune diabetes (Clare-Salzler et al., 1992; Fleili-Hariri et al., 1999; Machen et al., 2004). Furthermore, encouraging results with tolDCs were also obtained in clinical Phase I studies.

Tolerance induction by tolerogenic macrophages. Another promising tolerance-promoting strategy is the use of apoptotic cells. For example, infusion of peptides cross-linked to the surface of apoptotic splenic leukocytes using ethylene carbodiimide (EDC), has been demonstrated to be a highly efficient method for inducing antigen-specific T cell tolerance in animals with experimental autoimmune diseases (Jenkins and Schwartz, 1987; Getts et al., 2011). A single intravenous injection of syngeneic splenocytes coupled with encephalitogenic myelin peptides/proteins has been shown to induce antigen-specific tolerance in experimental autoimmune encephalomyelitis (e.g., Tan et al., 1991). Promising results were also obtained in a recent phase 1 clinical trial in multiple sclerosis patients using seven myelin peptides coupled with the chemical crosslinker EDC (1-ethyl-3-(3-dimethylaminopropyl)-carbodiimide) to autologous peripheral blood mononuclear cells (PBMCs) (Lutterotti et al., 2013).

The exact mechanism of tolerization with antigens coupled to the surface of cells with EDC is not fully understood, but there is evidence that several mechanisms are involved. On the one hand it has been shown that antigen-specific T cells encountering their cognate antigen/MHC complexes on EDC-treated cells are anergized as a result of failure to receive adequate CD28-mediated costimulation (Jenkins and Schwartz, 1987). On the other hand, a potentially more important mechanism is based on the fact that EDC efficiently induces apoptosis in treated cells. Experiments in animal models suggest that i.v. injected apoptotic EDC-treated cells are phagocytosed in the spleen within a few hours by antigen-presenting cells (monocytes/macrophages and/or immature dendritic cells) leading to the production of IL-10 and expression of PD-L1 (programmed cell death 1 ligand; CD274) on macrophages which appears to be regulated by IL-10 in possibly autocrine fashion (Getts et al., 2011). The production of IL-10 by macrophages upon phagocytosis of apoptotic cells is in agreement with results of a previous study which has shown that feeding of peripheral blood-derived macrophages with apoptotic cells triggers the production in IL-10 (Voll et al., 1997). PD-L1 mediates T cell-negative costimulation. The formation of PD-1 receptor/PD-L1 ligand complex transmits an inhibitory signal which reduces the proliferation of CD8(+) T cells at lymph nodes. Furthermore, PD-1 is also able to control the accumulation of foreign antigen-specific T cells in lymph nodes through apoptosis (Chemnitz et al., 2004).

Both the production of IL-10 and the expression of PD-L1 on macrophages are important factors for the induction of tolerance. IL-10 plays an important immune regulatory role, preventing inflammatory immune responses and the development of autoimmunity. Importantly, neither IL-10-deficient mice nor mice treated with anti-IL-10 can be tolerized with ethylene carbodiimide-fixed splenocytes (Getts et al., 2011).

Promotion of tolerance by phosphatidyl-L-serine-containing liposomes (PS-liposomes). Several studies demonstrate that PS-liposomes have the potential to mimick the anti-inflammatory effect of apoptotic cells and, therefore, could serve as tolerance-promoting adjuvant.

In viable cells, PS is kept exclusively on the inner leaflet of the lipid bilayer via ATP-dependent translocases. In apoptotic cells, the concentration of PS on the outer leaflet of the lipid bilayer is estimated to increase by more than 280-fold within only a few hours after induction of apoptosis. PS exposed on the surface of apoptotic cells represents the key signal for triggering phagocytosis by macrophages (for a review, see Hochreiter-Hufford and Ravichandran, 2013). This is indicated by the observation that phagocytosis of apoptotic lymphocytes by macrophages was inhibited in a dose-dependent manner by PS and PS-containing liposomes, but not by liposomes containing other anionic phospholipids including phosphatidyl-D-serine (Fadok et al., 1992).

PS-liposomes have been shown to exert inhibitory effects on certain macrophage functions in vitro (Kornbluth, 1994). For example, PS-liposomes inhibited the IFN-γ-mediated induction of anti-leishmanial activity in murine macrophages (Gilbreath et al., 1985). Furthermore, inhibitory effects of PS-liposomes on the production of nitric oxide (NO) by mouse peritoneal macrophages stimulated with LPS have been reported (Matsuno et al., 2001). Apparently, TGF-β is the factor produced by PS-liposomes that suppresses production of NO, and the ERK signaling pathway is intimately involved in TGF-β production by macrophages following treatment with PS-liposomes (Otsuka et al., 2004). As demonstrated in a recent study, expression of MyD88, which is essential for the signal transduction in lipopolysaccharide (LPS) stimulation, is suppressed when macrophages are treated with PS-liposomes (Tagasugi et al., 2013).

PS-liposomes have also been demonstrated to regulate the maturation of human DCs. Large unilamellar PS-liposomes inhibited the up-regulation of HLA-ABC, HLA-DR, CD80, CD86, CD40, and CD83, as well as the production of IL-12p70 in human DCs in response to LPS. DCs exposed to PS had diminished capacity to stimulate allogeneic T cell proliferation and to activate IFN-γ-producing CD4(+) T cells (Chen et al., 2004). A corresponding effect of PS-liposomes on the maturation and immuno-stimulatory functions of murine DCs in response to 1-chloro-2,4-dinitrobenze (DNCB) was observed in the study of Shi et al. (2007). After treatment with PS-liposomes, murine DCs displayed reduced expression of MHC II, CD80, CD86 and CD40, but increased programmed death ligand-1 (PD-L1 and PD-L2), increased IL-10 and inhibited IL-12 cytokine production. DCs treated with PS-liposomes exhibited normal endocytic function, but their ability to stimulate allogeneic T cells was reduced, similar to immature dendritic cell. Treatment of DCs with PS-liposomes also suppressed DNCB-induced CD4(+) T cell proliferation and IFN-γ production. Furthermore, DCs treated with PS-liposomes enhanced the ratio of CD4(+) CD25(high)Foxp3(+) T cells to CD4(+) T cells and PD-1 expression on CD4(+) T cells.

In vivo effects of PS and PS-liposomes have also been reported (Kornbluth, 1994). For example, intravenous injection of PS in mice has been demonstrated to reduce both T cell-dependent and T cell-independent antibody production (Ponzin et al., 1989) and intravenous injection of PS prior to the injection of bacterial LPS has been shown to reduce serum TNF-α levels (Monastra and Bruni, 1992). Comparable effects have been observed also after injection of PS-liposomes. For example, intraperitoneal injection of PS-liposomes ameliorated the course of extrinsic allergic encephalitis induced in mice by immunization with myelin basic protein (Monastra et al., 1993). Also, PS-liposomes specifically inhibited responses in mice to antigens as determined by decreased draining lymph node tissue mass, reduced numbers of total leukocytes and antigen-specific CD4+ T cells and decreased levels of antigen-specific IgG in blood. TGF-β appears to play a critical role in this inhibition, as the inhibitory effects of PS-liposomes were reversed by in vivo administration of anti-TGF-β antibodies (Hoffmann et al., 2005). A recent study demonstrated that after uptake of PS-liposomes in vitro and in vivo, macrophages secrete high levels of the anti-inflammatory cytokines TGF-β and IL-10 and upregulate the expression of CD206 (mannose receptor C type 1; MRC1), concomitant with downregulation of pro-inflammatory markers such as TNF-α and the surface marker CD86 (Harel-Adar et al., 2011). CD86 (also known as B7-2) is a protein expressed on antigen-presenting cells that provides costimulatory signals necessary for T cell activation and survival. In subsequent experiments, the authors demonstrated that modulation of cardiac macrophages by PS-liposomes improves infarct repair. Injection of PS-liposomes via the femoral vein in a rat model of acute myocardial infarction promoted angiogenesis and prevented ventricular dilatation and remodeling (Harel-Adar et al., 2011).

Based on these studies, PS-liposomes have the ability to modulate macrophages and DCs in a tolerogenic manner. However, the potential of PS-liposomes as tolerance-promoting adjuvant for current approaches of antigen- or allergen-specific immunotherapy is limited for several reasons. Most important for a tolerance-promoting effect of PS-liposomes at the subcutaneous site of antigen or allergen presentation is their presence in this local microenvironment at therapeutically effective concentrations for at least two days, since the development of immunologic memory requires the engagement of the T cell receptor (TCR) over 12-48 h and long lasting memory may even require repetitive exposure (Shakhar et al., 2005; Garcia et al., 2007; Obst et al., 2007). This requirement, however, pose serious problems since subcutaneously injected PS-containing liposomes will diffuse away from the injection site and repeated injections are not practical.

Furthermore, find-me signals in addition to surface-exposed PS may be required to trigger effective local phagocytosis by macrophages and DCs in the periphery at the subcutaneous site of antigen or allergen presentation and, thereby, to enhance the tolerance-promoting effect of PS-containing liposomes. The role of find me signals is to establish a chemotactic gradient stimulating the migration of phagocytes to apoptotic cells. To date, several proposed find-me signals released by dying cells have been reported including fractalkine (i.e., chemokine CXC3CL1, a membrane associated protein that is proteolytically released from apoptotic B cells), lysophosphatidylcholine (LPC, released from apoptotic cells by caspase-3-dependent activation of phospholipase A2), sphingosine-1-phosphate (S1P, produced by sphingosine kinase 1) and the nucleotides ATP and UTP, which function together in an additive or synergistic manner during the phagocyte attraction (for a review, see Hochreiter-Hufford and Ravichandran, 2013).

However, administration of low molecular weight find-me signals such as LCP, S1P, ATP and UTP at the site of allergen or antigen presentation cannot establish chemotactic gradients which are comparable to those established by the continuing release of find-me signals from apoptotic cells. Furthermore, especially low molecular weight find-me molecules such as ATP and UTP are rapidly degraded and removed from circulation.

Patent application EP14075015.9 discloses a PLGA-PEG-PLGA hydrogel-based method that solves some of the above mentioned problems in that the hydrogel provides a slow release system for embedded PS-liposomes and the find-me molecules ATP/UTP. However, PS-liposomes are released relatively slowly from the hydrogel (approx. 15% during the first 24 hours), whereas ATP and UTP are released rather rapidly from the hydrogel (approx. 60% during the first 24 hours). After 48 hours the release of PS-liposomes from PLGA-PEG-PLGA hydrogels continues for another 2-3 days, but in the absence of find-me molecules due to the almost complete release of ATP and/or UTP from injected hydrogels during the first 48 hours. Furthermore, APCs which have been attracted to the injected hydrogel by ATP and/or UTP, migrate to local lymph nodes and, therefore, will not be present at the hydrogel site after 48 hours.

Therefore, there is a need for a more practical solution that ensures the release of PS-liposomes in the presence of ATP and/or UTP for more than 2 days.

Promotion of tolerance by anionic PLGA spheres. Anionic poly(lactic-co-glycolic acid) (PLGA) spheres PLGA represent another potential tolerance-promoting adjuvant.

PLGA belongs to a family of biodegradable polymers that are highly biocompatible. In water, PLGA biodegrades by hydrolysis of its ester linkages, which leads to metabolite monomers, lactic acid and glycolic acid. Because these two monomers are endogenous and easily metabolized by the body via the Krebs cycle, a minimal systemic toxicity is associated with the use of PLGA for drug delivery (for a review, see Makadia and Siegel, 2011) or biomaterial applications (for a review, see Gentile et al., 2014). Due to these properties, PLGA has been approved by the US FDA and European Medicine Agency (EMA) in various drug delivery systems for humans.

Autoantigen-loaded PLGA nanoparticles have been demonstrated to induce tolerogenic effects in animals with experimental autoimmune diseases. For example, oral application of collagen II-loaded PLGA nanoparticles (approx. 300 nm in size) in mice with collagen-induced arthritis induced profound tolerogenic effects as indicated by a significantly reduced arthritis index, a reduced incidence of arthritis, a reduced number of arthritis limb, a reduced development of antibodies to collagen II, and a reduced stimulation index of isolated lymph node cells upon incubation with collagen II (Kim et al., 2002).

Tolerogenic effects have also been achieved with allergen-loaded PLGA nanoparticles (for a review, see De Souza Reboucas et al., 2012). For example, subcutaneous treatment of Bet v 1-sensitized mice with a single shot of Bet v 1-containing PLGA nanoparticles (470-750 nm in size) resulted in the development high levels of Bet v 1-specific IgG2a antibodies (reflecting Th1 bias), whereas IgG1 levels (Th2 marker) decreased significantly. Moreover, T cells from mice treated with PLGA-Bet v 1 showed IFN-γ and IL-10 production (Schöll et al., 2004). These data demonstrate that allergen-loaded PLGA nanoparticles can modulate an ongoing Th2 response in a murine allergy model as indicated by down-regulation of IgG1 and production of IFN-γ and IL-10.

A similar observation was obtained with ovalbumin (OVA)-containing PLGA nanoparticles. Nasal administration of OVA-containing PLGA nanoparticles was found to induce immuno-regulatory responses as shown by enhanced Foxp3 expression in the nasopharynx associated lymphoid tissue and cervical lymph nodes and functional suppression of an OVA-specific Th1-mediated delayed-type hypersensitivity reaction (Keijzer et al., 2011).

Recently, OVA-containing PLGA nanospheres (400 nm in size) were demonstrated to enhance retinaldehyde dehydrogenase enzyme activity in cervical lymph node-derived CD11c+ dendritic cells (DCs) (Keijzer et al., 2013). The metabolic conversion of retinol to retinoic acid in DCs, which drives Treg differentiation, requires retinaldehyde dehydrogenase. Using these DCs after treatment with OVA-containing PLGA nanospheres for in vitro activation of OVA-specific T cells, an enhanced induction of tolerance-promoting CD4(+)FoxP3(+) T-cells via a retinoic acid and TGF-β dependent mechanism was observed (Keijzer et al., 2013).

As demonstrated in several studies, even plain PLGA spheres have the capacity to promote tolerance (for a review, see Getts et al., 2015). For example, in the study of Kim et al. (2002), oral tolerance induction in mice with collagen-induced arthritis (CIA) was investigated using PLGA nanoparticles (approx. 300 nm in size) with or without entrapped collagen II. The data show that both plain and collagen II-loaded PLGA spheres exert tolerogenic effects, although the tolerogenic effects induced by plain PLGA spheres were less profound than those induced by collagen II-containing PLGA nanoparticles.

The observation that even plain PLGA spheres are tolerance-promoting was substantiated by the study of Jilek et al. (2004). Using a murine phospholipase A2-induced allergy model, the authors have demonstrated that plain PLGA microspheres (size range of 1-10 µm) can induce tolerance for as long as 6 months post-sensitization. In this study, the potential of plain cationic (polyethylenimine-coated) and anionic PLGA microspheres for modulating the allergic response triggered by bee venom phospholipase A2 (PLA2) sensitization over a 34-week time course was analyzed. Both cationic and anionic plain PLGA microspheres were capable to downregulate allergic responses in this model and mice were even protected against an otherwise lethal challenge with phospholipase A2. For both microsphere formulations, the peak of PLA2-specific IgG2a antibody (reflecting Th1 bias) preceded that of IgG1 (Th2 marker). PLA2-specific IgG1 and IgG2a production turned out to be 2 times higher using cationic microspheres compared to anionic microspheres. The initial Th1 bias observed at the level of immunoglobulin was sustained transiently, followed by a mixed Th1/Th2 response supported by a similar expression of IL-4 and IFN-γ. During the whole time frame a sustained production of IL-10 was observed in both groups. These data suggest a dual mechanism that does initially rely on a Th2 to Th1 immune deviation and then on IL-10-mediated suppression which can explain the protective effect against anaphylaxis.

Patent application WO 2014/160465 A2 (priority date March 13, 2013) describes amelioration of inflammatory immune responses by administration of negatively charged plain particles including plain anionic PLGA particles. In view of the previous publications this observation is not surprising, but the inventors could also demonstrate that the therapeutic efficacy of negatively charged PLGA particles is affected the extent of negative charge. In comparison to the impressive therapeutic efficacy of highly negative carboxylated PLGA particles, less negative particles prepared by coating of the same particles with plasma proteins (reduction of the negative charge from -50 mV of non-coated particles to -16 mV of coated particles) exhibited a related reduced therapeutic efficacy.

As described more in detail in a subsequent publication (Getts et al., 2014), infused highly negative carboxylated PLGA particles are taken up by inflammatory monocytes, in an opsonin-independent fashion, via the macrophage receptor with collagenous structure (MARCO). Subsequently, these monocytes no longer traffic to sites of inflammation, but are sequestered in the spleen through apoptotic cell clearance mechanisms and, ultimately, undergo caspase-3-mediated apoptosis. Infusion of highly negative carboxylated PLGA particles in mouse models of myocardial infarction, experimental autoimmune encephalomyelitis, dextran sodium sulfate-induced colitis, thioglycollate-induced peritonitis, and lethal flavivirus encephalitis markedly reduced monocyte accumulation at inflammatory foci, reduced disease symptoms, and promoted tissue repair.

Based on these studies, plain PLGA particles and in particular plain anionic PLGA spheres have the ability to modulate pathological immune responses in a tolerogenic manner. However, the potential of plain anionic PLGA spheres as tolerance-promoting adjuvant for current approaches of antigen- or allergen-specific immunotherapy is limited for the similar reasons as described for PS-liposomes.

As mentioned above, a tolerance-promoting effect of plain PLGA particles for allergen- or antigen-specific immunotherapy requires the presence of such particles at therapeutically effective concentrations in the local microenvironment of the site of antigen or allergen presentation for an extended period of time, at least as long as the development of immunologic memory requires upon antigen or allergen exposure. Using plain PLGA particles with a size of more than 200 nm as adjuvant for subcutaneous immunotherapy, the particles will remain at the site of antigen or allergen presentation for some time since particles with that size must be phagocytosed by local phagocytes before transport to the draining lymph nodes. However, effective phagocytosis of PLGA particles at subcutaneous sites requires find-me signals for DCs and macrophages which is problematic since especially low molecular weight find-me signals such as ATP and UTP are rapidly eliminated at the site of antigen or allergen presentation due to diffusion and fast degradation, and single subcutaneous injections of find-me signals at the injection site of PLGA particles cannot establish chemotactic gradients which are comparable to those established by the continuing release of find-me signals from apoptotic cells.

Patent US 6,287,588 B1 and continuation in part application US 2002/0076441 disclose a dual phase polymeric agent-delivery composition comprising a continuous biocompatible gel phase such as PLGA-PEG-PLGA hydrogels, and a discontinuous particulate phase such as PLGA micro- or nanoparticles. Such compositions solve some of the above mentioned problems in that the hydrogel can serve as depot for PLGA particles and low molecular weight find-me molecules such as ATP and/or UTP. However, application of such hydrogel-based compositions pose the problem that PLGA particles are released relatively slowly from the hydrogel, whereas ATP and UTP are released rather rapidly from the hydrogel. Due to the rapid release of ATP and/or UTP from injected hydrogels, the majority of PLGA particles would be released from PLGA-PEG-PLGA hydrogels in the absence of find-me molecules.

As evident from the foregoing, there is a need for suitable approaches that allow for a concomitant sustained release of PS-containing liposomes or PLGA particles and low molecular weight find-me molecules from hydrogels for a period of more than 2 days. The present invention solves this problem and discloses methods for a significant improvement of the therapeutic efficacy of current antigen- or allergen-specific immunotherapies using hydrogel-embedded tolerance-promoting adjuvants loaded with low molecular weight find-me molecules.

### SUMMARY OF THE INVENTION

In one embodiment, the present invention provides novel compositions and methods for restoring immunological tolerance by subcutaneous allergen- or autoantigen-specific immunotherapy. Said compositions are matrix-based compositions for subcutaneous administration comprising matrix-embedded allergens or autoantigens or fragments thereof, one or more tolerance-promoting adjuvants, optionally one or more low molecular weight find-me molecules, and optionally one or more tolerance-promoting active immune modulators.

In another embodiment, the present invention provides hydrogel-, alum- and micro crystalline tyrosine (MCT)-based compositions for subcutaneous administration comprising matrix-embedded allergens or autoantigens or fragments thereof, one or more tolerance-promoting adjuvants, optionally one or more low molecular weight find-me molecules, and optionally one or more tolerance-promoting active immune modulators. Preferred compositions are hydrogel-based compositions comprising embedded allergens or autoantigens or fragments derived thereof, one or more tolerance-promoting adjuvants, optionally one or more low molecular weight find-me molecules, and optionally one or more tolerance-promoting active immune modulators.

In another embodiment, the present invention discloses in situ-forming hydrogel systems suitable for the method of the present invention. Such in situ-forming gel systems undergo a sol-gel-sol transition, from a free flowing sol at room temperature to a non-flowing gel at body temperature. Preferred are injectable in situ-forming gel systems which are biodegradable. Most preferred are biodegradable PLGA-PEG-PLGA hydrogels. Compared to other biodegradable hydrogels, injectable thermo-gelling PLGA-PEG-PLGA polymers possess several advantages including easy preparation, a formulation process which is free of harmful organic solvents, application of building blocks which are approved for parenteral use in humans by the FDA, excellent biocompatibility, and well established procedures for the production of compositions comprising liposomes, PLGA particles and various proteinaceous components. Most important, PLGA-PEG-PLGA hydrogels do not mediate tolerance-interfering Th1-type or Th2-type immune responses.

In another embodiment, the present invention discloses PLGA particles as tolerance-promoting adjuvant. Tolerance-promoting PLGA particles suitable for the present invention include plain PLGA micro- and nanoparticles, PLGA micro- and nanoparticles coated with allergenic or autoimmunogenic proteins or fragments thereof, or PLGA micro- and nanoparticles containing encapsulated allergenic or autoimmunogenic proteins or fragments thereof. Preferred for the method of the present invention are plain, anionic PLGA nanospheres.

In another embodiment, the present invention discloses PS-liposomes as tolerance-promoting adjuvant. Tolerance-promoting PS-liposomes suitable for the present invention include PS-containing conventional liposomes, ethosomes, niosomes, and elastic liposomes (the initial formulation approach being termed transferosomes). Preferred for the method of the present invention are conventional PS-liposomes. Conventional, tolerance-promoting PS-liposomes suitable for the present invention include empty PS-liposomes, PS-liposomes coated with allergenic or auto-immunogenic proteins or fragments thereof, or PS-liposomes containing encapsulated allergenic or auto-immunogenic proteins or fragments thereof.

In another embodiment, the present invention discloses a combination of tolerance-promoting PLGA particles and tolerance-promoting PS-liposomes as adjuvants. Both tolerance-promoting adjuvants act synergistically, since they induce different tolerogenic cytokines. IL-10-mediated suppression of immune responses is an important tolerogenic mechanism of PLGA spheres, whereas TGF-β appears to play a critical role for the tolerogenic effects of PS-liposomes.

In another embodiment, the present invention discloses the application of plain anionic PLGA particles and/or PS-liposomes as tolerance-promoting adjuvants and for targeting of matrix-embedded allergenic or auto-immunogenic proteins or fragments thereof to antigen-presenting cells including dendritic cells and macrophages. The combined presence of allergens or autoantigens and PLGA spheres and/or PS-liposomes in hydrogel-, alum- or MCT-based compositions leads to adsorption of at least some of the allergenic or auto-immunogenic proteins or fragments thereof onto the surface of PLGA spheres and/or PS-liposomes. Upon their release from subcutaneously injected matrices, the tolerance-promoting PLGA spheres and PS-liposomes facilitate direct targeting of some of the matrix-embedded allergens or autoantigens or fragments thereof to local antigen-presenting cells (APCs) including macrophages and dendritic cells (DCs). PS-liposomes provide the additional advantage that phosphatidylserine (PS) is the most important eat-me signal for APCs and mediates preferred uptake of PS-liposomes by these cells. As a result, these allergens or autoantigen are presented to T cells by tolerogenic APCs due to the simultaneous uptake of a tolerance-promoting adjuvant. Furthermore, non-adsorbed allergenic or auto-immunogenic proteins are released from the subcutaneously injected matrices in an increasingly tolerogenic micro-environment.

In another embodiment, the present invention discloses low molecular weight find-me signals capable of triggering effective local phagocytosis at the site of allergen or autoantigen presentation, thereby enhancing the tolerance-promoting effect of PLGA spheres and PS-liposomes. Suitable low molecular weight find-me signals include but are not limited to lysophosphatidylcholine (LPC), sphingosine-1-phosphate (S1P) and the nucleotides ATP and UTP. Preferred are ATP and UTP as low molecular weight find-me molecules.

In another embodiment, tolerance-promoting PLGA particles and tolerance-promoting PS-liposomes are loaded with one or more low molecular weight find-me molecules. Loading of PLGA particles or PS-liposomes with low molecular weight find-me molecules such as ATP and/or UTP provides the important advantage that these tolerance-promoting adjuvants create a chemotactic gradient of find-me signals by themselves after their release from the injected matrices. To establish a sustained release of small amounts of encapsulated low molecular weight find-me molecules such as ATP and/or UTP, PLGA particles with appropriate degradation kinetics and PS-liposomes providing an appropriate leakiness are used. This novel approach guarantees efficient peripheral phagocytosis of the tolerance-promoting adjuvants and surface-attached allergenic or auto-immunogenic proteins.

In another embodiment, the present invention discloses allergens and autoantigens as well as formulations of allergens or autoantigens suitable for the method of the present invention. Suitable for the treatment of allergic diseases are allergen extracts, cross-linked allergen extracts (allergoids), depigmented allergoids, one or more purified natural allergens, one or more recombinant allergens or derivatives thereof, and one or more fragments of natural or recombinant allergens. Suitable for the treatment of autoimmune diseases are one or more purified natural autoantigens, one or more recombinant autoantigens or derivatives thereof, and one or more fragments of natural or recombinant autoantigens.

In another embodiment, the present invention discloses tolerance-promoting immune modulators (immune modulators) capable of a) inducing tolerogenic APCs (including DCs and macrophages) and tolerance-promoting Tregs, and b) inhibiting pro-inflammatory cytokines and pro-inflammatory complement factors at the site of autoantigen or allergen presentation.

In a preferred embodiment, immune modulators are employed for the method of the present invention which can be embedded in substantial quantities in PLGA-PEG-PLGA hydrogels, which are chemically and physically compatible with PLGA-PEG-PLGA hydrogels, and which are released from such matrices over a prolonged period of time. Suitable immune modulators include but are not limited to a) vitamin D3 and selected analogs such as calcipotriol, b) glucocorticoids such as dexamethasone phosphate, c) Janus kinase (JAK) inhibitors such as tofacitinib, d) antagonistic cytokine molecules such as Il-4/IL-13 muteins, e) salicylate-based therapeutics for the inhibition of TNFR1-mediated pathways, f) peptide- or peptidomimetic-based complement inhibitors, and g) aptamer-based inhibitors of pro-inflammatory cytokines. Preferred are low to medium molecular weight immune modulators which provide a relatively short serum half-life that is sufficient to be locally active upon their release from a depot at the site of autoantigen or allergen presentation, and which allows fast removal from circulation upon diffusion and transport away from the site of autoantigen or allergen presentation. Thereby, the induction of tolerogenic effects is restricted mainly to the site of autoantigen or allergen presentation and adverse effects due to interaction of immune modulators with targets distal from the site of autoantigen or allergen presentation are minimized.

In another embodiment, the present invention discloses the application of compositions for the treatment of patients suffering from allergy, allergic asthma or various autoimmune diseases including but not limited to rheumatoid arthritis, juvenile idiopathic arthritis, type 1 diabetes, multiple sclerosis, and autoimmune uveitis.

In another embodiment, the present invention discloses methods for incorporating compositions into pharmaceutical formulations suitable for administration.

In yet another embodiment, the present invention discloses therapeutic methods including therapeutic applications of suitable compositions, the determination of therapeutically effective doses, and modes of administration for the induction of allergen or autoantigen tolerance using the compositions of the present invention.

### BRIEF DESCRIPTION OF THE DRAWINGS

Figure 1: Analysis of PLGA-PEG-PLGA copolymer by gel permeation chromatography (GPC). For details see Example 1.1.4.
Figure 2: Concentration-dependent gelling temperature of PLGA-PEG-PLGA copolymer. For details see Example 1.1.5.
Figure 3: Subcutaneous formation of PLGA-PEG-PLGA gels in mice. For details see Example 1.1.6.
Figure 4: Effect of PS-liposomes on the gelation characteristics of PLGA-PEG-PLGA copolymers. For details see Example 3.2.
Figure 5: Release of PS-liposomes from PLGA-PEG-PLGA/PS-liposome compositions. For details see Example 3.3.3.
Figure 6: Release of find-me molecule ATP from PLGA-PEG-PLGA hydrogels. For details see Example 5.2.
Figure 7: Release of vitamin D3 from PLGA-PEG-PLGA hydrogels. For details see Example 6.1.
Figure 8: Release of TNFR1 inhibitor sodium salicylate (SA) from PLGA-PEG-PLGA hydrogels. For details see Example 6.3.
Figure 9: Release of IL-4 antagonist QY from PLGA-PEG-PLGA hydrogels. For details see Example 6.4.2.
Figure 10: Experimental setting for immunotherapy of OVA-sensitized mice with PLGA-PEG-PLGA hydrogel-embedded OVA. For details see Example 7.1.4.
Figure 11: Bronchoalveolar lavage analysis of OVA-sensitized mice after OVA-specific immunotherapy. For details see Example 7.1.7.
Figure 12: Experimental setting for the induction of tolerance towards PLA2 in mice with PLGA-PEG-PLGA hydrogel-embedded PLGA microspheres. For details see Example 7.3.3.

### DETAILED DESCRIPTION OF THE INVENTION

For the treatment of allergy, asthma and autoimmune diseases including but not limited to type I diabetes, rheumatoid arthritis, multiple sclerosis and autoimmune uveitis, allergen- or autoantigen-specific immunotherapy has the potential of restoring lasting immunological tolerance, but adjuvant strategies are needed to increase the efficacy of this approach.

One promising strategy for subcutaneous allergen- or autoantigen-specific immunotherapy is the use of matrix-embedded tolerance-promoting adjuvants including poly(lactic-co-glycolic acid) particles (PLGA particles) and/or phosphatidyl-L-serine-containing liposomes (PS-liposomes).

PLGA particles support the induction of tolerance and provide attractive properties including biodegradability, biocompatibility and FDA and European Medicine Agency (EMA) approval for parenteral administration.

PS-liposomes support also the induction of tolerance and provide attractive properties including biodegradability and biocompatibility. Furthermore, several liposomal formulations containing hydrophilic small drugs have been approved for parenteral administration by the FDA and EMA and are marketed as commercial products (for a review, see Eloy et al., 2014).

Both PLGA particles and PS-liposomes complement each other as tolerogenic adjuvants since they induce different tolerogenic cytokines. Apparently, IL-10-mediated suppression of immune responses is an important tolerogenic mechanism of PLGA spheres (Jilek et al., 2004), whereas TGF-β appears to play a critical role for the tolerogenic effects of PS-liposomes, as the inhibition of immune responses by PS-liposomes could be reversed by in vivo administration of anti-TGF-β antibodies (Hoffmann et al. (2005).

### 1. PLGA particles as tolerance-promoting adjuvant

In one embodiment of the present invention, PLGA particles are used as tolerance-promoting adjuvant.

Preferred size and shape of PLGA particles. For the method of the present invention, PLGA particles with a size of more than 100 nm are suitable. Nanoparticles with a size of less than 100 nm are not suitable since they tend to interact with cellular organelles, including the mitochondria and nucleus, and these interactions can trigger cellular respiratory and gene toxicity in cells (Park et al., 2011). This risk is reduced with increasing NP size, presumably because larger NPs tend to initiate phagocytosis, which effectively isolates particles from the more sensitive cytoplasmic environment (for a review, see Getts et al., 2015).

Preferred for the method of the present invention are PLGA particles with a size of more than 200 nm. Small particles with a size of less than 200 nm may drain freely from subcutaneous sites of application to local lymph nodes (Xiang et al., 2013), whereas particles with a size of more than 200 nm must be phagocytosed by local phagocytes before transport to the draining lymph nodes. In order to create a local tolerogenic micro-environment at the injection site of the hydrogel composition, phagocytosis of released PLGA particles close to the injected hydrogel composition is preferred. Thereby, PLGA-particle-induced secretion of the anti-inflammatory cytokines TGF-β and IL-10 by macrophages and DCs will create a tolerogenic environment in proximity of the injection site for the presentation of allergenic or auto-immunogenic proteins upon their release from the injected hydrogel composition.

Preferred for the method of the present invention are PLGA spheres. For antigen-presenting cells (APCs) such as macrophages and dendritic cells (DCs), uptake is also impacted by shape, with spherical nanoparticles having more favorable uptake kinetics than rodshaped nanoparticles, irrespective of nanoparticle size (Champion and Mitragotri, 2006).

PLGA nanospheres with a size of 300-500 nm are rapidly taken up by local APCs as demonstrated in a recent study (Nicolete et al., 2011). The data of this study show that plain PLGA nanospheres with an average size of 389 nm are rapidly phagocytosed by murine macrophages, whereas the uptake of plain PLGA microspheres with an average size of 6.5 µm by is slow.

Based on these observations, spherical PLGA particles with a size ranging from 200 nm to 10 µm are applicable for the method of the present invention. Preferred are PLGA spheres with a size ranging from 300 nm to 1 µm.

Preferred surface charge of PLGA nanoparticles. For the promotion of tolerance according to the method of the present invention, anionic PLGA nanoparticles are preferred. The preference for anionic PLGA nanospheres is supported by the observation that cationic nanoparticles can alter mitochondrial and endoplasmic reticulum function, triggering the production of reactive oxygen species and pro-inflammatory cytokines, as well as cell death (Hawang et al., 2015; Chiu et al., 2014; Xia et al., 2008), whereas anionic nanoparticles have been associated with little to no toxicity (Park et al., 2011).

Most important, however, cationic nanoparticles are likely to induce Th1-type immune responses rather than tolerance-promoting effects. Positively charged nanoparticles seem to be able to escape from lysosomes after being internalized and exhibit perinuclear localization, whereas negatively and neutrally charged nanoparticles prefer to colocalize with lysosomes (for a review, see Danhier et al., 2012). Escape of cationic nanoparticles from lysosomes into the cytosol, however, is likely to favor presentation of allergenic or auto-immunogenic proteins adsorbed onto the surface of these cationic nanoparticles in the hydrogel via MHC class I, which leads to Th1-type immune responses. This assumption is supported by the observation that positively charged antigen-loaded nanoparticles are significantly more effective at stimulating Th1 responses after either intradermal or mucosal (pulmonary) inoculation, whereas anionic particles stimulate T and B cell responses poorly under similar conditions (Fromen et al., 2015; Bal et al., 2011). This observation is further substantiated by a recent study in which the uptake of cationic particles by dendritic cells has been demonstrated to regulate positive costimulatory molecules in these cells (Koppolu and Zaharoff., 2013). In vitro, professional antigen-presenting cells such as DCs have been reported to engulf cationic microspheres in low acidic phagosomes, resulting in slow degradation that favors expression of the surface marker CD86, and production of TNF-α (Jilek et al., 2004b; Thiele et al., 2003).

In contrast, induction of allergen-specific tolerance has been achieved with allergen-loaded anionic PLGA nanoparticles (for a review, see De Souza Reboucas et al., 2012). For example, subcutaneous treatment of Bet v 1-sensitized mice with a single shot of Bet v 1-containing anionic PLGA nanoparticles (470-750 nm in size) resulted in the development high levels of Bet v 1-specific IgG2a antibodies (reflecting Th1 bias), whereas IgG1 levels (Th2 marker) decreased significantly. Moreover, T cells from mice treated with PLGA-Bet v 1 showed IFN-γ and IL-10 production (Schöll et al., 2004). These data demonstrate that allergen-loaded PLGA nanoparticles can modulate an ongoing Th2 response in a murine allergy model as indicated by down-regulation of IgG1 and production of IFN-γ and IL-10.

A similar observation was obtained with ovalbumin (OVA)-containing anionic PLGA nanoparticles (250-500 nm in size). Nasal administration of nanoparticles was found to induce immuno-regulatory responses as shown by enhanced Foxp3 expression in the nasopharynx associated lymphoid tissue and cervical lymph nodes and functional suppression of an OVA-specific Th1-mediated delayed-type hypersensitivity reaction. Furthermore, detailed analysis of the CD4(+) T cell response revealed that OVA-containing anionic PLGA nanoparticles induce both IL-10 and IFN-γ (Keijzer et al., 2011).

Recently, OVA-containing anionic PLGA nanospheres (400 nm in size) were demonstrated to enhance retinaldehyde dehydrogenase enzyme activity in cervical lymph node-derived CD11c+ dendritic cells (DCs) (Keijzer et al., 2013). The metabolic conversion of retinol to retinoic acid in DCs, which drives Treg differentiation, requires retinaldehyde dehydrogenase. Using these DCs after treatment with OVA-containing PLGA nanospheres for in vitro activation of OVA-specific T cells, an enhanced induction of tolerance-promoting CD4(+)FoxP3(+) T-cells via a retinoic acid- and TGF-β-dependent mechanism was observed (Keijzer et al., 2013).

Both plain anionic PLGA nanoparticles and antigen-containing anionic PLGA nanoparticles have also been used successfully for the induction of tolerance in mice with collagen-induced arthritis (CIA). Oral administration of collagen II-loaded anionic PLGA spheres (approx. 300 nm in size) induced profound tolerogenic effects as indicated by a significantly reduced arthritis index, a reduced incidence of arthritis, and a reduced stimulation index of isolated lymph node cells upon incubation with collagen II. Plain anionic PLGA spheres (approx. 300 nm in size) induced also tolerogenic effects although to lesser extent (Kim et al., 2002).

### 2. PS-Liposomes as tolerance-promoting adjuvant

In another embodiment of the present invention, phosphatidyl-L-serine (PS)-containing liposomes (PS-liposomes) are used as tolerance-promoting adjuvant.

Tolerance-promoting PS-liposomes applicable for the present invention include PS-containing conventional liposomes, ethosomes, niosomes, and elastic liposomes (the initial formulation approach being termed transferosomes). Preferred for the method of the present invention are conventional PS-liposomes.

Applicable lipid mixtures of PS-liposomes. Lipid mixtures of conventional PS-liposomes are composed of PS and other phospholipids such as phosphatidylcholine (PC) from soybean or egg yolk, with or without cholesterol (CH). Cholesterol is used to stabilize conventional liposomes.

The most common applied PS is derived from bovine brain, but other PS sources and synthetic PS preparations such as 1-palmitoyl-2-oleyl-sn-3-glycerophospho-L-serine or 1,2-distearoyl-sn-3-glycero-phospho-L-serine are also suitable.

For the preparation of conventional PS-liposomes without cholesterol various lipid mixtures containing PS and PC are applicable including but not limited to lipid mixtures comprising molar ratios of PS:PC of 30:70 (Gilbreath et al., 1985) or 50:50 (Fadok et al., 2001).

For the preparation of conventional cholesterol-containing PS-liposomes also various lipid mixtures containing PS, PC and CH are applicable including but not limited to lipid mixtures comprising molar ratios of PS:PC:CH of 30:30:40 (Hoffmann et al., 2005; Harel-Adar et al., 2011).

The percentage of PS in lipid mixtures which are applicable for the method of the present invention ranges from 5 to 50%. As demonstrated recently, efficient uptake by macrophages can also be achieved with liposomes containing PS as low as 6 mol% (Geelen et al., 2012).

Preparation procedures of PS-liposomes. Conventional liposomes can be prepared in several ways. Most frequently, a film hydration method is employed, where a thin layer of lipid is deposited on the walls of a container by evaporation of a volatile solvent. An aqueous solution containing the molecule to be entrapped is added at a temperature above the transition temperature of the lipids, resulting in the formation of multilamellar vesicles. These systems contain several lipid bilayers surrounding the aqueous core. Further processing by sonication or filter extrusion generates large unilamellar vesicles (LUV, 1-5 µm diameter), or small unilamellar vesicles (LUV, 0.1-0.5 µm diameter).

For the method of the present invention, both multilamellar and unilamellar PS-liposomes are applicable. Preferred are unilamellar PS-liposomes. Unilamellar PS-liposomes with 1 µm diameter have been shown to trigger efficient uptake by macrophages (Harel-Adar et al., 2011).

### 3. Low molecular weight find-me molecules for enhanced peripheral phagocytosis

Apoptotic cells are quickly recognized and removed by phagocytes, which can be either neighboring healthy cells or professional phagocytes recruited to the site of apoptotic cell death. Phagocytes are extremely efficient in sensing and detecting the dying cells at the earliest stages of apoptosis and in the recent past, several find-me signals released from apoptotic cells have been identified (for a review, see Ravichandran, 2011).

In one embodiment, the present invention utilizes find-me signals capable of triggering effective local phagocytosis. Suitable find-me signals include but are not limited to low molecular weight molecules such as lysophosphatidylcholine (LPC), sphingosine-1-phosphate (S1P) and the nucleotides ATP and UTP.

ATP and/or UTP are preferred for the method of the present invention. Both nucleotides have been described as non-redundant find-me signals released by apoptotic cells (Elliott et al., 2009). UTP acts only on P2Y-family receptors and UDP produced via degradation of released UTP by extracellular enzymes has been shown to promote phagocytic activity via the P2Y6 nucleotide receptor. In contrast, ATP acts on P2X- and P2Y-family receptors, whereas ADP produced via degradation of released ATP by extracellular enzymes acts only on P2Y-family receptors (for a review, see Gombault et al., 2013).

In a more preferred embodiment, equimolar quantities of ATP and UTP are employed as 'find me' signals. Using a transwell migration assay, both nucleotides have been demonstrated to effect maximal migration (approximately a threefold increase) of phagocytes at a concentration of about 100 nM (Elliott et al., 2009). For the method of the present invention it is important to restrict the concentration of ATP and UTP to the lower nanomolar range since extracellular nucleotides at higher concentrations (more than 1 µM, for example, by necrotic cells) are considered pro-inflammatory (Kono and Rock, 2008).

Adjustment of degradation and release kinetics of PLGA nanospheres. For the method of the present invention, preferred PLGA nanospheres loaded with ATP and/or UTP are those which release both nucleotides within a period of 2 to 5 days.

Since the release kinetics of both nucleotides are determined largely by the degradation kinetics of the PLGA nanospheres, preferred PLGA nanospheres provide corresponding degradation kinetics. In water, poly(lactic-co-glycolic acid) (PLGA) biodegrades by hydrolysis of its ester linkages. In general, polymer degradation is accelerated by greater hydrophilicity in the backbone or end groups, lesser crystallinity, lower average molecular weight, and smaller size of the finished device.

The polymer composition is the most important factor to determine the hydrophilicity and rate of degradation (for a review, see Makadia and Siegel, 2011). For PLGA nanospheres the amount of glycolic acid is the critical parameter in tuning the hydrophilicity of the matrix and thus the degradation and release rate of loaded low molecular weight find-me molecules. An increase in glycolic acid percentage in the oligomers accelerates the weight loss of polymer. For example, PLGA 50:50 (PLA/PGA) exhibits a faster degradation than PLGA 65:35 due to preferential degradation of the hydrophilic glycolic acid proportion. Accordingly, PLGA 65:35 shows faster degradation than PLGA 75:25 and PLGA 75:25 a faster degradation than PLGA 85:15. PLGA 75/25 exhibits a half-life of 10 weeks compared with 20 weeks for PLGA 85/25 (for a review, see Alexis, 2005).

Thus the degradation rate and the release rate of loaded low molecular weight 'find-me' molecules increases with the glycolic acid proportion. A detailed analysis by gel permeation chromatography of the decrease in molecular weight of PLGA microspheres as a function of time has been performed by Park (1995). According to this study, the molecular weight of PLGA microspheres with a ratio of 50:50 (PLA/PGA) decreased rapidly within 2 days from approx. 2 x 10⁴ to approx. 8 x 10². A similar decrease in molecular weight was observed for PLGA microspheres with a ratio of 70:30 (PLA/PGA), whereas the decrease in molecular weight for PLGA microspheres with a ratio of 80:20 (PLA/PGA) was significantly delayed, exhibiting an almost linear decrease over 20 days from approx. 2 x 10⁴ to approx. 3 x 10².

Based on these data, applicable for the method of the present invention are anionic PLGA nanospheres with a glycolic acid proportion ranging from 10% to 50%. Preferred are anionic PLGA nanospheres with a glycolic acid proportion ranging from 20% to 50%. More preferred are anionic PLGA nanospheres with a glycolic acid proportion ranging from 30% to 50%.

Encapsulation of low molecular weight find-me molecules in anionic PLGA nanospheres. Various techniques are availble for manufacturing PLGA nanospheres loaded with low molecular weight find-me molecules (for a review, see Makadia and Siegel, 2011).

The most common method used for the preparation of solid, polymeric nanospheres is the emulsification solvent evaporation technique, and this method is primarily used in encapsulation of hydrophobic molecules such as hydrophobic low molecular weight find-me molecules (e.g., lysophosphatidylcholine (LPC) and sphingosine-1-phosphate (SIP)).

A modification on this procedure called the double or multiple emulsion technique has become the favored protocol for encapsulating hydrophilic compounds such as hydrophilic low molecular weight find-me molecules (e.g., ATP and UTP)(Mao et al., 2007).

Adjustment of leakiness of PS-liposomes loaded with ATP and/or UTP. For the method of the present invention it is important that PS-liposomes loaded with low molecular 'find-me' molecules such as ATP and UTP are leaky to some extent to allow for a controlled release of these find-me molecules in order to establish a chemotactic gradient for phagocytes.

In one embodiment of the present invention, a varying extent of cholesterol in the bilayer of PS-liposomes is used to establish a controlled leakiness for encapsulated ATP and/or UTP, suitable for generating chemotactic gradients for phagocytes.

The presence of cholesterol exerts a profound influence on the properties of the lipid bilayers of the liposomes. For example, liposomes made from 100% unsaturated lipid in fluid phase are leaky and cannot hold encapsulated water soluble compounds. The addition of cholesterol to a fluid phase bilayer comprising mainly unsaturated lipids, decreases its permeability to water. Cholesterol molecules fill in the free space that was formed due to the kink in the chain of the unsaturated lipids and this will decrease the flexibility of the surrounding lipid chains. This interaction also increases the mechanical rigidity of fluid bilayers and decreases their lateral diffusion.

In contrast, the addition of cholesterol to gel phase bilayers comprising mainly saturated lipids, disrupts local packing orders and increases the diffusion coefficient and decreases the elastic modulus. Liposomes made from 100% saturated lipids are leak-proof in the absence of the cholesterol.

The effect of cholesterol on the stability of liposomes and the release of encapsulated hydrophilic small molecular weight compounds has been investigated in various studies. For example, in the study of Kirby and Gregoriadis (1981), the release of radiolabelled sucrose from small unilamellar liposomes comprising either egg phosphatidylcholine (cholesterol-free liposomes), or egg phosphatidylcholine and cholesterol at a molar ratio of 40 (PC):11.4 (CH) (cholesterol-poor liposomes), or egg phosphatidylcholine and cholesterol at a molar ratio of 40 (PC):40 (CH) (cholesterol-rich liposomes), has been investigated in the presence of mouse plasma (liposome/plasma ratio 1:4). After 30 min incubation at 37°C, released sucrose from cholesterol-free liposomes was 80.6%, from cholesterol-poor liposomes 42.2%, and from cholesterol-rich liposomes only 4.1%. These data demonstrate that both cholesterol and serum proteins affect the stability of liposomes and the release rate of encapsulated hydrophilic small molecular weight compounds.

Certain serum proteins are known to react with liposomes and to cause leakage (for a review, see Bonté and Juliano, 1986). For the method of the present invention, however, interactions of serum proteins with PS-liposomes are likely to play a minor role since most of the surface of hydrogel-embedded PS-liposomes will be coated with allergenic or auto-immunogenic proteins, leaving little surface space for interactions with serum proteins.

The extent of leakiness of PS-liposomes containing an aqueous solution of ATP and/or UTP is further influenced by several factors including buffer species, temperature, and pH of the aqueous solution (Kensil and Dennis, 1981; Grit et al., 1989). Several studies have demonstrated that aqueous formulations of drug products tend to be less stable since the presence of excess or bulk water leads to rapid hydrolytic degradation in lipid preparations (Kensil and Dennis, 1981; Grit et al., 1989).

In one specific embodiment, lipids from biological sources (e.g., egg, bovine liver, bovine brain, or soybean) are used for the preparation of PS-liposomes containing encapsulated ATP and/or UTP (compare Fadok et al., 2001). Lipids from biological sources typically contain significant levels of polyunsaturated fatty acids and therefore are inherently less stable than their synthetic counterparts. The degree of leakiness for ATP and/or UTP is adjusted by the addition of varying amounts of cholesterol to the lipid mixtures.

In another specific embodiment, synthetic monounsaturated phosphatidyl compounds containing oleic acid or phosphatidyl compounds containing both monounsaturated oleic acid and saturated palmitic acid such as 1-palmitoyl-2-oleoyl-sn-3-glycerphosphorylcholine (POPC) and 1-palmitoyl-2-oleoyl-sn-3-glycerphospho-L-serine (POP-L-S) are used for the preparation of PS-liposomes containing encapsulated ATP and/or UTP (compare Hoffmann et al., 2005). Since these phosphatidyl compounds are monounsaturated, they provide a lesser degree of leakiness for ATP and/or UTP than polyunsaturated compounds, which may be further decreased by the addition of cholesterol.

### 4. Hydrogels for sustained delivery of active components and adjuvants

Effective supportive promotion of tolerance by PLGA particles and/or PS-liposomes at the site of allergen or autoantigen presentation requires a sustained local delivery of these tolerance-promoting adjuvants for a period of at least 2 to 3 days which is important for efficient tolerogenic priming of APCs, since the development of immunologic memory requires the engagement of the T cell receptor (TCR) over 12-48 h and long lasting memory may even require repetitive exposure (Shakhar et al., 2005; Garcia et al., 2007; Obst et al., 2007).

In one embodiment, the present invention discloses suitable hydrogels for sustained local delivery of tolerance-promoting adjuvants, optionally loaded with find-me molecules, along with a prolonged presentation of allergens or autoantigens or fragments thereof, and optionally for sustained delivery of tolerance-promoting active immune modulators. Preferred hydrogels a) can serve as depot for sufficient quantities of allergens or autoantigens or fragments thereof, tolerance-promoting adjuvants (optionally loaded with find-me molecules) and optionally tolerance-promoting active immune modulators, b) allow the release of sufficient quantities of the embedded components over a prolonged period of at least 2 to 3 days, and c) are chemically and physically compatible with all embedded components.

Hydrogels which are applicable for the present invention include biodegradable and non-biodegradable thermogelling hydrogels.

Non-biodegradable thermogelling hydrogels. Poloxamers (trade name Pluronics) are nonionic triblock copolymers composed of a central hydrophobic chain of poly(propylene oxide) (PPO) flanked by two hydrophilic chains of poly (ethylene oxide) (PEO) (Gilbert et al., 1987). Poloxamers exhibit a sol-gel transition behavior in aqueous solutions and have been used for sustained delivery of several therapeutic agents. However, poloxamers are not biodegradable and can accumulate in the body which may lead to toxic side effects. Thus, the application of poloxamers in biomedical fields has been greatly restricted. In a recent study, however, Pluronic F127 (100-unit PEO chain surrounding one 65-unit PPO) has been used to form composite thermo-sensitive hydrogels with PEG-PCL-PEG (PECE) (Gong et al., 2009b). Based on the results of this study Pluronic F127/PECE composite hydrogels are biocompatible with low cell cytotoxicity and, therefore, are applicable for the method of the present invention.

Biodegradable thermogelling hydrogels. In a preferred specific embodiment, injectable in situ-forming gel systems which are biodegradable, are used for the method of the present invention (for a review, see Ruel-Gariepy and Leroux, 2004). In situ-forming gel systems (thermogelling hydrogels) undergo a sol-gel-sol transition, which is a free flowing sol at room temperature and a non-flowing gel at body temperature.

In a more preferred specific embodiment of the invention, biodegradable thermogelling hydrogels are used which are composed of FDA-approved biodegradable polymers. Preferred biodegradable polymers approved by the FDA and used in a clinical trial, include but are not limited to poly(D,L-lactic acid), poly(lactic-co-glycolic acid) (PLGA), and copolymers of L-lactide and D,L-lactide. All FDA approved polymers have been studied extensively for their biocompatibility, toxicology, and degradation kinetics. Furthermore, these polymers have been shown to release embedded therapeutics for several hours up to several weeks in vivo.

Useful for the method of the present invention are biodegradable thermogelling block polymers which are based on monomethoxy poly(ethylene glycol) (MPEG) including but not limited to a) diblock copolymers consisting of MPEG and poly(ε-caprolactone) (PCL) (Hyun et al., 2007), b) MPEG-*b-*(PCL-ran-PLLA) diblock copolymers (Kang et *al.,* 2010), and c) diblock copolymers consisting of MPEG and PLGA (Peng et al., 2010). MPEG copolymers containing PCL provide the advantage that they do not create an acidic environment upon biodegradation in contrast to MPEG copolymers containing PLLA and PLGA (Hyun et al., 2007).

Useful for the method of the present invention are also biodegradable thermo-gelling diblock and triblock copolymers which consist of polyethylene oxide (PEO) and a biodegradable polyester such as poly-L-lactic acid (PLLA) (Jeong et al., 1997). These block copolymers, however, are a free flowing sol at a higher temperature and form a gel at a lower temperature. For example, a 23% aqueous solution of PEO-PLLA-PEO (Mᵣ 5,000-2,040-5,000) is a sol at 45°C and becomes a gel at 37°C. By changing the biodegradable block length, the sol-gel transition temperature can be manipulated, e.g., increasing the PLLA block length increases the aggregation tendency of a block copolymer in water, resulting in a steepening of the gel-sol transition curve slopes and the onset of gelation at lower concentrations. The sol-gel transition temperature is a function of concentration as well as composition of a block polymer.

Useful for the method of the present invention are also thermo-sensitive hydrogels formulated on the basis of trimethylated chitosan derivatives (Wu et al., 2012) are used for the sustained delivery of suitable active substances and one or more antigens or allergens. In addition to its application as a vaccine delivery system, the cationic polysaccharide has shown promising results as an adjuvant. For example, application of a chitosan solution for subcutaneous vaccination has been demonstrated to enhance both humoral and cell-mediated immune responses (Zaharoff et al., 2007). However, the unfavorable pH-dependent solubility and charge density of chitosan is a limiting factor. In contrast, trimethylated chitosan is well soluble in aqueous solution at neutral pH and provides excellent biocompatibility and mucoadhesive nature. Trimethylated chitosan derivatives are best characterized by the degree of quarternization, the degree of O-methylation and the degree of acetylation (Hagenaars et al., 2010). Trimethylated chitosan derivatives are frequently formulated into particles or spray powder (Alhalaweh et al., 2009). Very recently, the trimethylated chitosan derivative N[(2-hydroxy-3-trimethylammonium) propyl] chitosan chloride has also been formulated together with α,β-glycerophosphate into a thermal-sensitive hydrogel (Wu et al., 2012). This hydrogel was shown a) to significantly prolong the antigen residence time in the nasal cavity, b) to enhance the transepithelial transport via the paracellular routes, and c) to induce in mice a high mucosal immunity (sIgA) and systemic immune response (IgG1 and IgG2a).

Preferred for the method of the present invention are biodegradable thermogelling triblock polymers including but not limited to a) PLGA-PEG-PLGA (Qiao et al., 2005), b) PEG-PLGA-PEG (Zhang et al., 2006), and c) PEG-PCL-PEG (PECE) (Gong et al., 2009a). Various biodegradable thermogelling triblock polymers made up of PLGA and PEG are disclosed in patent application WO 99/18142. At lower temperatures, hydrogen bonding between hydrophilic PEG segments of the copolymer chains and water molecules dominate in aqueous solutions, resulting in the dissolution of these copolymers in water. As the temperature increases, the hydrogen bonding becomes weaker, while hydrophobic forces of the hydrophobic segments such as PLGA segments are getting stronger, leading to sol-gel transition. PEG, PLGA and PCL are well-known FDA-approved biodegradable and biocompatible materials which have been widely used in the biomedical field.

More preferred for the method of the present invention are biodegradable thermogelling PLGA-PEG-PLGA triblock polymers. Compared to other biodegradable hydrogels, injectable thermo-gelling PLGA-PEG-PLGA polymers possess several advantages including easy preparation, a formulation process which is free of harmful organic solvents, application of building blocks which are approved for parenteral use in humans by the FDA, excellent biocompatibility, and well established procedures for the production of composits comprising liposomes and PLGA particles. Furthermore, PLGA-PEG-PLGA hydrogels provide another important advantage in that tolerance-interfering Th1-type or Th2-type immune responses are avoided.

PLGA particle-thermogelling hydrogel formulations. US patent 6,287,588B1 and continuation in part application US 2002/0076441 disclose a dual phase polymeric agent-delivery composition comprising a continuous biocompatible gel phase such as PLGA-PEG-PLGA hydrogels, and a discontinuous particulate phase such as PLGA micro- or nanoparticles. A distinct advantage of using a thermogelling hydrogel such as PLGA-PEG-PLGA as matrix for the preparation of PLGA particle-hydrogel formulations lies in the ability of PLGA-PEG-PLGA hydrogels to wet and suspend PLGA particles. The combination of hydrophobic A-blocks (PLGA) and hydrophilic B-blocks (PEG) renders the block copolymer amphiphilic in nature. In that regard, it functions as a wetting agent. The viscosity of PLGA-PEG-PLGA hydrogels is slightly higher than normal saline, thus it can also been seen as a thickening agent. The combination of these two properties makes thermogelling hydrogels such as PLGA-PEG-PLGA an excellent suspending agent for hydrophobic PLGA particles.

PS-liposome-thermogelling hydrogel formulations. For sustained local delivery of PS-liposomes at the site of autoantigen or allergen presentation according to the method of the present invention, different liposome-hydrogel formulations are suitable (e.g., Xing et al., 2013; Nie et al., 2011).

In a preferred specific embodiment, thermo-sensitive hydrogels comprising PLGA-PEG-PLGA are employed for sustained local delivery of PS-liposomes, optionally loaded with one or more low molecular weight find-me molecules, at the site of autoantigen or allergen presentation. As demonstrated in a recent study, liposome-loaded PLGA-PEG-PLGA hydrogels exhibit still reversible thermo-sensitive properties (Xing et al., 2013). However, its sol-gel and gel-precipitate transition temperatures decreased with increasing liposome concentrations. Most important, the particle size of free liposomes and those released from the PLGA-PEG-PLGA hydrogels were found to be close regardless of particle size of liposomes, indicating that the liposomes were stable in the hydrogel and intact liposomes could be released (Xing et al., 2013).

Modification of the degradation kinetics of thermogelling hydrogels. The various biodegradable thermo-gelling polymers provide different stability characteristics. For example, poloxamer triblock polymers provide excellent thermo-sensitivity, but due to weak hydrophobicity of the PPO block such copolymers form fast eroding gels which have been reported to persist in vivo a few hours at most. Exposure of poloxamer gels to phosphate-buffered saline under in vitro conditions demonstrated gel erosion within 2 days (Hyun et al., 2007). Similar results were observed when the polymer solutions were subcutaneously injected into rats. Remaining poloxamer gels could not be observed after 2 days (Hyun et al., 2007).

Different results were obtained with MPEG-PCL gels. Under in vitro conditions MPEG-PCL gels maintained their structural integrity for more than 28 days and after subcutaneous injection into rats MPEG-PCL gels maintained their structural integrity longer than 30 days. The stability of MPEG-PCL gels, however, may also create problems since the rate of degradation of PCL *in vivo* is rather slow (2-3 years) compared to that of PLA, PGA or PLGA (for a review, see Sinha et al., 2004). Therefore, most preferred biodegradable thermogelling polymers for the method of the present invention are those which maintain their structural integrity for a few days but do not remain in the body for more than a month.

Useful for the method of the present invention are biodegradable thermos-gelling polymers which allow modification of their degradation kinetics. For example, PLLA segments can be incorporated into the PCL segment of MPEG-PCL copolymers, since PLLA provides better accessibility of water to the ester bonds of PLLA which enhances the hydrolytic degradation of the copolymer (Kang et al., 2010).

In another example, the rate of PLGA-PEG-PLGA hydrogel erosion can be modified by altering the molar ratio of DL-lactide/glycolide in the PLGA segment. The DL-lactide moiety is more hydrophobic than the glycolide moiety. Therefore, by increasing the molar ratio of DL-lactide/glycolide in the PLGA segment of PLGA-PEG-PLGA triblock copolymers, more stable hydrogels are formed due to stronger hydrophobic interactions among the copolymer molecules (Qiao et al. 2005).

### 5. Suitable allergens and autoantigens

In still another embodiment, the present invention discloses allergens and autoantigens or fragments thereof applicable for the method of the present invention.

### 5.1. Allergens and allergenic formulations for the treatment of allergic diseases.

Applicable for the treatment of allergic diseases according to the method of the present invention are allergen extracts, mixtures of allergen extracts obtained from different sources, allergen extracts enriched with one or more purified natural allergens, cross-linked allergen extracts (allergoids), depigmented allergoids, one or more purified natural allergens obtained from the same source or different sources, one or more recombinant allergens or derivatives thereof, and one or more fragments of natural or recombinant allergens.

Suitable allergenic proteins or fragments thereof include but are not limited to those which are present in plant pollen derived from grass (including but not limited to ryegrass, timothy-grass), weeds (including but not limited to ragweed, plantago, nettle, Artemisia vulgaris, Chenopodium album, sorrel), and trees (including but not limited to birch, alder, hazel, hornbeam, Aesculus, willow, poplar, Platanus, Tilia, Olea, Ashe juniper, Alstonia scholaris)). Applicable allergenic proteins or fragments thereof include also those which are present in animal products (e.g., derived from dust mite excretion (feces and chitin), fur and dander, Fel d 1, cockroach calyx, and wool), food (including but not limited to those derived from legumes (peanuts, beans, peas, and soybeans), tree nuts (pecans, and almonds), fruit (e.g., pumpkin, egg-plant), eggs (typically albumen, the white), milk, fish (e.g., shellfish), wheat and their derivatives, sesame seeds, mustard, celery, celeriac, and corn or maize), and insect venoms (derived from e.g., wasps, honey bees, mosquitos, and fire ants). Applicable allergenic proteins or fragments thereof include also airborne fungal allergens (e.g., the basidospore family which produces the dominant airborne fungal allergens and includes mushrooms, rusts, smuts, brackets, and puffballs).

### 5.2. Autoantigens for the treatment of autoimmune diseases.

Applicable for the treatment of autoimmune diseases according to the method of the present invention are one or more purified natural autoantigens, one or more recombinant autoantigens or derivatives thereof, and one or more fragments of natural or recombinant autoantigens.

Target autoantigens in rheumatoid arthritis (RA). Some of the described autoantigens in RA are joint-derived proteins, such as type II collagen and human cartilage-derived glycoprotein HCgp39 (Tsark et al., 2002). Other autoantigens are stress-associated proteins, including grp78/BiP, which is an intracellular chaperone involved in endoplasmic reticulum stress and angiogenesis in proliferative RA synovial tissue (Blass et al., 2001; Yoo et al., 2012).

Citrullinated autoantigens have emerged as a major group of post-translationally modified autoantigens in RA. Citrullination or deimination is the conversion of the amino acid arginine in a protein into the amino acid citrulline. Enzymes called peptidyl arginine deiminases (PADs) replace the primary ketimine group (=HN) by a ketone group (=O). PADs are are Ca²⁺-dependent enzymes and are activated upon intracellular Ca²⁺ flux into inflammatory settings. Arginine is positively charged at neutral pH, whereas citrulline is uncharged. As ca result, citrullination can substantially affect the protein structure and function.

Approximately 70% of RA patient sera contain autoantibodies reactive to a variety of citrullinated peptide antigens (ACPA) (Vossenaar & Venrooij, 2004). These autoantigens include vimentin, fibrinogen, collagen type II, α-enolase, clusterin, histones and peptidyl arginine deiminase-4 itself (Anzilotti et al., 2010; Masson-Bessiere et al., 2001; Sokolove et al., 2012). Citrullinated autoproteins are found in inflamed RA joints, but are not specific to RA. Anti-citrullinated protein antibodies (ACPA) may predate the onset of clinical RA by up to 15 years.

The major histocompatibility complex (MHC) contributes about one-third of the genetic susceptibility to RA. Specific RA-associated human leukocyte antigen (HLA) DR alleles encode a conserved amino acid sequence in the HLA-DR antigen-binding groove, known as the shared epitope (SE). Antibodies to citrullinated peptide antigens (ACPA), reflecting autoreactivity to citrullinated autoantigens, are much more likely to occur in patients with the HLA-DR SE (van der Helm-van Mil et al., 2007).

Several studies have demonstrated citrullinated autoantigen-specific T-cell autoimmunity in RA patients carrying HLA susceptibility alleles (Law et al., 2012; von Delwig et al., 2010; Snir et al., 2011). It was found that pro-inflammatory cytokines were secreted by peripheral blood (PB) CD4⁺ T cells of RA patients and healthy controls, in response to citrullinated but not unmodified peptides in the context of the HLA-SE sequence. RA patient T cells secreted a broader range of cytokines than healthy control T cells. Of the peptides tested, citrullinated aggrecan was most immunogenic (Law et al., 2012).

Autoantigens used in tolerance trials include type II bovine or chicken collagen, HCgp39, lyophilised Escherichia coli extract, dnaJp1 peptide (derived from heat shock protein dnaJ), and citrullinated peptides (for a review, see Thomas, 2013).

Oral administration of chicken or bovine type II collagen was safe when administered to patients with RA. While some clinical improvement was noted in open-labelled studies, placebo-controlled trials found no statistically significant improvement in collagen-fed patients, including among patients with early RA.

Oral administration of human gp39 was also trialled by two companies, but with little evidence of efficacy.

Oral administration of lyophilised E. coli extract containing several bacterial heat shock proteins with immunomodulatory properties, showed in a placebo-controlled trial clinical efficacy equivalent to D-penicillamine. This extract is more likely to be a nonspecific immunomodulator than to induce antigen-specific tolerance.

Oral administration of the 15-mer synthetic peptide dnaJp1 (QKRAAYDQYGAAFE), derived from HSP dnaJ (Albani et al., 1995), was trialled in phase I and phase II clinical trials in RA. This bacterial heat shock protein sequence has been proposed to be cross-reactive with corresponding self-peptides in RA because it is homologous with the HLA-DR SE sequence. Oral dnaJpl had an excellent safety profile and demonstrated immune modulatory effects. Whereas patients generally made Th1-type T-cell cytokine and proliferative responses to dnaJp1 at baseline, dnaJ-specific proliferation and IFN-γ decreased and IL-4 and IL-10 increased after the treatment. In a placebo-controlled phase II clinical trial, the dnaJp1 treated group showed statistically significant improvement in American College of Rheumatology scores after 6 months of daily oral dnaJp1 peptide, associated with increased expression of regulatory molecules and decreased secretion of TNF in PB (Koffeman et al., 2009).

Recently, a Phase I clinical trial in RA patients has evaluated the feasibility and safety of autologous tolDC therapy. Autologous tolDCs were generated by treatment with the tolerogenic agent BAY11-7082, an NFκB inhibitor, and exposure to several citrullinated peptides citrullinated peptides (cit-vimentin, cit-fibrinogen, cit-fibrinogen, cit-collagen type II). RA patients received only one intradermic dose of 1 x 10⁷ tolDCs. Only mild adverse effects such as headache and minimal changes in hematology parameters were reported while the expected therapeutic effect could already be observed in some patients (Thomas et al., 2011).

Based on these studies, the 15-mer synthetic peptide dnaJp1 and citrullinated peptides derived from cit-vimentin, cit-fibrinogen, cit-fibrinogen, and cit-collagen type II, represent preferred target autoantigens for the treatment of RA according to the method of the present invention.

Target auto-antigens in type 1 diabetes (T1D). Triggers of islet autoimmunity have not been identified. The only markers of islet autoimmunity are islet autoantibodies to insulin, GAD65 (glutamic acid decarboxylase), IA-2 (islet antigen 2; tyrosine phosphatase), and the ZnT8 transporter (zink transporter 8, localized on the membrane of insulin secretory granules) (for a review, see Lenmark and Larsson, 2013).

The first trial ever attempted to preserve β-cell function was based on intensive insulin therapy with continuous insulin infusion delivered by an external artificial pancreas, termed the 'Biostator' (Shah et al., 1989). Compared to the conventionally-treated group, the experimental group showed preservation of β-cell function with an increase in C-peptide levels at 1 year post-treatment; however, all T1D individuals remained insulin-dependent (Shah et al., 1989).

Subsequent insulin therapy trials include the DPT-1 trial, in which parental insulin was administered to individuals with positive autoantibodies, and the ENDIT trial in which individuals with positive autoantibodies were enrolled and received nicotinamide. Both trials failed to show any significant effect on halting the progression of the disease (Gale et al., 2004; Schatz and Bingley, 2001; Skyler et al., 2005).

The administration of oral insulin to first- and second-degree relatives of individuals with T1D at high genetic risk for developing T1D, showed some beneficial (Skyler et al., 2005).

Therapy of T1D individuals with immunomodulatory peptide DiaPep277 (derived from hsp60 protein) resulted in preservation of stimulated C-peptide for 10 months post-treatment. Furthermore, the DiaPep277 group required less exogenous insulin than the placebo group (Raz et al, 2001). Two other randomized trials using DiaPep277 reported stability of C-peptide levels compared to placebo (Schloot et al., 2007). DiaPep277 phase III or known as DIA-AID 1 trial, showed some preservation of β-cell function and an improved glycemic control in T1D individuals (Hegele et al., 2013).

Therapy with GAD-alum (glutamic acid decarboxylase formulated in alum) in subjects with T1D within 6 months from diagnosis also showed preservation of stimulated C-peptide levels (Ludvigsson et al., 2008). However, phase 2 and 3 trials of GAD-alum therapy failed to confirm the preliminary observation (Wherrett et al., 2011; Ludvigsson et al., 2012).

Therapy with DNA plasmid encoding proinsulin (BHT-3021) showed promising results. Proinsulin is a major target of the adaptive immune response in T1D. 80 subjects over 18 years of age who were diagnosed with T1D within the past 5 years were randomized 2:1 to receive intramuscular injections of BHT-3021 or BHT-placebo, weekly for 12 weeks, and then monitored for safety and immune responses in a blinded fashion. No serious adverse events related to BHT-3021 were observed. C-peptide levels improved relative to placebo at all doses. Proinsulin-reactive CD8⁺ T cells, but not T cells against unrelated islet or foreign molecules, declined (Roep et al., 2013).

Based on these studies, proinsulin, GAD and HSP60-derived peptides represent preferred target autoantigens for the treatment of T1D according to the method of the present invention.

Target autoantigens in multiple sclerosis (MS). In MS, the primary target antigens are not known for certain, but it is well accepted that proteins within the myelin sheath, such as myelin basic protein (MBP), myelin oligodendrocyte protein (MOG), and proteolipid protein (PLP), are important targets of the autoreactive immune response.

However, the target epitopes of myelin proteins differ between MS patients, and it is likely that the myelin-specific T cell reactivity may change over time. In relapsing-remitting (RR) animal models of MS, chronic demyelination leads to the generation of new T cell responses against multiple endogenous antigens, a process called epitope spreading, and these newly generated T cells are able to induce relapses, which can be inhibited by tolerance to the spread epitope.

In a recent successful Phase 1 trial, 7 myelin peptides (MBP₁₃₋₃₂, MBP₈₃₋₉₉, MBP₁₁₁₋₁₂₉, MBP_{146-170,} MOG₁₋₂₀, MOG_{35-55,} and PLP₁₃₉₋₁₅₄), which were previously identified as important targets of autoreactive T cells in MS, were coupled to the surface of apoptotic PBMCs (Lutterotti et al., 2013).

In another recent successful double-blind, placebo-controlled trial, 3 myelin peptides (MBP₈₅₋₉₉, PLP₁₃₉₋₁₅₁, and MOG₃₅₋₅₅) were applied transdermally as a skin patch in 30 patients with relapsing-remitting MS over the course of 1 year (Walczak et al., 2013).

Still another study has demonstrated that human DC pulsed with 7 myelin peptides (MBP₁₃₋₃₂, MBP₈₃₋₉₉, MBP₁₁₁₋₁₂₉, MBP₁₄₆₋₁₇₀, MOG₁₋₂₀, MOG₃₅₋₅₅, and PLP₁₃₉₋₁₅₄₎, can induce anergy in myelin-specific T cells obtained from relapsing-remitting MS patients (Raich-Regue et al., 2012). In this study, monocyte-derived DCs (MMDCs) from RR-MS patients were treated with a proinflammatory cytokine cocktail in the presence of 1α,25- dihydroxyvitamin-D3.

Based on these studies, 7 myelin peptides including MBP₁₃₋₃₂, MBP_{83-99,} MBP₁₁₁₋₁₂₉, MBP_{146-170,} MOG₁₋₂₀, MOG₃₅₋₅₅, and PLP₁₃₉₋₁₅₄ represent preferred target autoantigens for the treatment of MS according to the method of the present invention.

Target auto-antigens in autoimmune uveitis (AU). AU is a rare autoimmune disease and qualifies for orphan designation. AU is a sight-threatening inflammation inside the eye and is the fourth most common cause of blindness among the working-age population in the developed world. The prevalence of AU is estimated at 38 cases per 100,000 people.

Several major autoantigens for human autoimmune uveitis have been identified including S-antigen (A-Ag) (Wacker et al., 1977), interphotoreceptor retinoid binding protein (IRBP) (Caspi et al., 1988), cellular retinaldehyde binding protein (CRALBP) (Deeg et al., 2007), and arrestin (Kyger et al., 2013).

Both experimental and clinical autoantigen-specific immunotherapeutic trials have been performed. Recently, effective arrestin-specific immunotherapy of experimental autoimmune uveitis in humanized leukocyte antigen (HLA-DR3) transgenic mice has been reported using subcutaneously injected recombinant T cell receptor ligands with covalently tethered arrestin peptide 291-310 (RTL351). The protective effect of RTL351 was attributed to a significant decrease in Th1/Th17-mediated inflammation (Kyger et al., 2013).

Clinical trials have been performed with peptide B27PD (Optiquel, Enzo Biochem; USA) which is a human leukocyte antigen class I antigen that mimics ocular S-antigen (Thurau et al., 2004). Retinal S-antigen, a 45 kDa glycoprotein, is the most characterized retinal autoantigen (Wacker et al., 1977).

In a small pilot trial, eight patients with chronic non-infectious uveitis (NIU) were treated orally with peptide B27PD 4 mg three times daily for 12 weeks and followed for five years. The average corticosteroid dosage decreased from 10.4 mg/day prior to oral tolerance induction to 2.9 mg/day at year 2 and 1.9 mg/day at year 5. Although relapses occurred in some patients, those who were retreated with oral peptide B27PD for uveitis recurrence responded to treatment, exhibiting decreased inflammation.

A 52-week, phase 1/2 study assessed the efficacy of peptide B27PD as corticosteroid-sparing therapy in 31 patients with chronic NIU (NCT01195948). Peptide B27PD was administered at oral doses of 1 and 4 mg three times daily, while a third group received placebo. The primary endpoint was the time to recurrence of uveitis during or after tapering the corticosteroid dose to 7.5 mg/day (Optiquel as corticosteroid-sparing therapy for chronic non-infectious uveitis. Available at: https://clinicaltrials. gov/show/NCT01195948. Accessed June 18, 2015).

Based on these studies, S-antigen, interphotoreceptor retinoid binding protein, cellular retinaldehyde binding protein, arrestin, and peptides derived of these autoantigens such as arrestin peptide 291-310 represent preferred target autoantigens for the treatment of AU according to the method of the present invention.

### 6. Adsorption of allergens or autoantigens onto the surface of anionic PLGA nanospheres and PS-liposomes

Delivery of allergenic or auto-immunogenic proteins to APCs together with tolerance-promoting anionic PLGA nanospheres represents a promising strategy for the induction of tolerance as demonstrated in several experimental studies (for a review, see De Souza Reboucas et al., 2012). However, the successful application of allergen- or autoantigen-containing PLGA particles has been demonstrated only for PLGA particles containing either one encapsulated individual allergen (e.g., OVA or Bet v 1), or one encapsulated individual autoantigen (e.g., collagen II). Due to the limited encapsulation efficiency of macromolecules in PLGA nanospheres, encapsulation of complete allergen extracts or a composition comprising a variety of different allergenic or auto-immunogenic proteins poses serious problems.

This problem is solved by the present invention in that plain anionic PLGA nanospheres, optionally loaded with one or more low molecular weight find-me molecules, are co-embedded in a suitable matrix including but not limited to PLGA-PEG-PLGA hydrogels with compositions comprising a variety of different allergenic or auto-immunogenic proteins or fragments thereof. The combined presence of allergens or autoantigens or fragments thereof and PLGA spheres in the injected matrix leads to adsorption of at least some of the allergenic or auto-immunogenic proteins or fragments thereof onto the surface of the PLGA spheres. Anionic PLGA particles have surface functional groups such as aliphatic chains and ester linkages in addition to ionizable groups such as carboxyls and hydroxyls that will carry a net negative charge at physiologic pH. As a result, PLGA particles can adsorb proteins or fragments thereof on their surface through a combination of electrostatic and hydrophobic attraction.

The novel combination of matrix-embedded plain anionic PLGA spheres and compositions comprising a variety of different allergenic or auto-immunogenic proteins or fragments thereof provides an important advantage in that suitable matrices and especially PLGA-PEG-PLGA hydrogels allow embedment of larger amounts of allergenic or auto-immunogenic proteins than those that can be encapsulated in PLGA spheres. Furthermore, PLGA spheres with adsorbed allergens or autoantigens facilitate direct targeting of some of the matrix-embedded allergens or autoantigens or fragments thereof to local antigen-presenting cells (APCs) including macrophages and dendritic cells (DCs). Of course, not all of the proteins embedded in the injected matrix-composition such as negatively charged proteins will adsorb to matrix-embedded anionic PLGA nanospheres. However, non-adsorbed proteins are released from the injected matrices in an increasingly tolerogenic micro-environment due to the concomitant release and uptake by APCs of tolerance-promoting PLGA nanospheres with adsorbed allergens or autoantigens.

Similar considerations apply to the application of matrix-embedded PS-liposomes as tolerance-promoting adjuvant for allergen- or autoantigen-specific immunotherapy. The combined presence of allergens or autoantigens or fragments thereof and PS-liposomes in PLGA-PEG-PLGA hydrogels or in other suitable matrices will also lead to adsorption of at least some of the allergenic or auto-immunogenic proteins or fragments thereof onto the surface of the PS-liposomes. PS-liposomes provide an additional advantage in that PS on the liposomal surface mediates direct targeting and uptake of released protein-coated liposomes by antigen-presenting cells (APCs) including macrophages and dendritic cells (DCs).

### 7. Additional tolerance-promoting immune modulators

In another embodiment, the present invention provides also compositions for allergen-specific SCIT and autoantigen-based immunotherapy comprising one or more additional tolerance-promoting immune modulators.

Suitable compositions for additional tolerance-promoting immune modulators according to the method of the present invention include but not limited to hydrogel-, alum- and micro crystalline tyrosine-based compositions.

Preferred compositions are hydrogel-based compositions comprising embedded allergens or autoantigens or fragments derived thereof, one or more tolerance-promoting adjuvants, optionally one or more low molecular weight find-me molecules, and one or more tolerance-promoting active immune modulators. Most preferred compositions are based on PLGA-PEG-PLGA hydrogels since such hydrogels are suitable for sustained delivery of several components and are compatible with a large number of tolerance-promoting therapeutic immune modulators.

In one embodiment, the present invention discloses low or medium molecular weight tolerance-promoting active immune modulators (immune modulators) capable of a) inducing tolerogenic APCs (including DCs and macrophages) and tolerance-promoting Tregs, b) suppressing effector T cell-mediated responses, and c) inhibiting pro-inflammatory cytokines and pro-inflammatory complement factors at the site of autoantigen or allergen presentation.

In a preferred specific embodiment, immune modulators are employed for the method of the present invention which can be embedded in substantial quantities in PLGA-PEG-PLGA hydrogels, which are chemically and physically compatible with PLGA-PEG-PLGA hydrogels, and which are released from such matrices over a prolonged period of time. Preferred are low to medium molecular weight immune modulators which provide a relatively short serum half-life that is sufficient to be locally active upon their release from a depot at the site of autoantigen or allergen presentation, and which allows fast removal from circulation upon diffusion and transport away from the site of autoantigen or allergen presentation. Thereby, the induction of tolerogenic effects is restricted mainly to the site of autoantigen or allergen presentation and adverse effects due to interaction of immune modulators with targets distal from the site of autoantigen or allergen presentation are minimized.

Applicable immune modulators include but are not limited to a) vitamin D3 and selected analogs such as calcipotriol, b) glucocorticoids such as dexamethasone phosphate, c) Janus kinase inhibitors, also known as JAK inhibitors or jakinibs, such as tofacitinib, d) antagonistic cytokine molecules such as I1-4/IL-13 muteins, e) salicylate-based therapeutics for the inhibition of TNFR1-mediated pathways, f) peptide- or peptidomimetica-based complement inhibitors, and g) aptamer-based inhibitors of pro-inflammatory cytokines.

### 7.1. Vitamin D3 and selected analogs.

In one embodiment of the invention, vitamin D3 and selected analogs thereof are used for the method of the present invention.

Vitamin D, in particular the biologically active metabolite 1α,25-dihydroxyvitamin D3 (calcitriol; 1,25-(OH)₂D3), is a pleiotropic hormone exerting a variety of biological effects including the regulation of calcium, bone and mineral metabolism, as well as the modulation of immune responses by promoting both directly and indirectly regulatory T cell populations (for reviews, see Hart et al., 2011; Chambers and Hawrylowicz, 2011; Fletcher et al., 2012).

Vitamin D3, also known as cholecalciferol, is synthesized from 7-dehydrocholesterol in the skin upon exposure to ultraviolet light, then transported to the liver, where it is converted to 25-hydroxyvitamin D3 (25(OH)D3; calcidiol) by cytochrome P450 enzymes. Calcidiol is a circulating metabolite that is converted to the active hormone 1,25-(OH)₂D3 (calcitriol) in the mitochondria of the kidney by 25-hydroxyvitamin 1α-hydroxylase (CYP27B1). However, numerous other cell populations including keratinocytes in the epidermis possess the enzymes capable of processing vitamin D3 to active metabolites (Anderson et al., 2008). Macrophages, dendritic cells (DCs) as well as T and B cells express CYP27B1 when they are activated and, thereby, have the ability to produce the active hormone calcitriol (Bikle, 2009).

Calcitriol binds to the vitamin D receptor (VDR), a member of the superfamily of nuclear hormone receptors, followed by binding to the retinoic X receptor (RXR) and formation of a heterodimer. The heterodimer translocates to the nucleus where it can bind to specific DNA sequence elements, so-called vitamin D response elements (VDREs) identified as direct repeats of PuG(G/T)TCA motifs, thereby promoting transcription of vitamin D-responsive genes. The VDR-RXR heterodimer can also bind to a negative vitamin D response element, thereby preventing gene transcription. Alternatively, the VDR-RXR heterodimer can bind to transcription factors present in the nucleus which prevents binding of these transcription factors to their target gene promoters. VDR is expressed by many cells of the immune system including activated B and T cells, monocytes and DCs (for a review, see Chambers and Hawrylowicz, 2011).

### Suppression of adaptive immune responses by vitamin D3.

Several studies have demonstrated that locally synthesized calcitriol activates innate immune responses, but can also suppress adaptive immune responses (for reviews, see Bouillon et al., 2008; Hewison, 2010). Suppressive effects of 1,25-(OH)₂D3 on adaptive immune responses include a) antigen-presenting cell functions, b) effector T cell responses, and c) the induction of regulatory T cells (Tregs).

For the application of vitamin D3 as additional immune modulator it is important that vitamin D3 affects effector T cell responses also via direct inhibition of T cell responses. For example, calcitriol has been shown a) to inhibit Th1 cytokine release with a large reduction in IFN-γ from human peripheral CD3+CD4+ T cells (Reichel et al., 1987), and b) to hinder cytokine production by Th17 cells (Tang et al., 2009). Lower concentrations of calcitriol have been demonstrated to inhibit the expression of IL-4 and other Th2 cytokines in human T cells (Pichler et al., 2002).

Induction of Treg populations by vitamin D3. Concomitant with the inhibition of effector T cell responses by calcitriol is the induction of Treg populations. For example, in bulk cultures of human CD4(+) CD25(-) T cells and putative naive T cells, calcitriol increased in the presence of IL-2 the frequency of activation-induced FoxP3(+) T cells expressing high levels of the inhibitory receptor CTLA-4 (cytotoxic T lymphocyte antigen 4). Furthermore, a significant reduction in the pro-inflammatory cytokines IFN-γ and IL-17 was observed in this study (Jeffery et al., 2009). In another study, treatment of human peripheral blood CD4(+) T cells with calcitriol induced IL-10(+) Tregs (Urry et al., 2009). The capacity of calcitriol to promote tolerogenic T cell functions in humans is further supported by the observation that vitamin D supplementation and pharmacologic treatment with biologically active vitamin D increased the level of serum- or T cell-associated TGF-β and IL-10 (Mahon et al., 2003; Urry et al., 2009).

In a recent study, the molecular and cellular events underlying the immunosuppressive effects of calcitriol have been elucidated more in detail (van der Aar et al., 2011). Calcitriol promotes tolerogenic epidermal Langerhans cells (LCs) and dermal dendritic cells (DDCs). Both subsets are able to generate Treg cells with different effector functions. Treatment of epidermal LCs with calcitriol generates functional Foxp3(+) Tregs through a mechanism that is dependent on keratinocyte-derived TGF-β. In contrast, treatment of DDCs with calcitriol generates functional IL-10(+) FoxP3(-) T_{R}1 cells in an IL-10-dependent fashion.

Modulation of dendritic cells (DCs) by vitamin D3. DCs play a central role in regulating immune activation and responses to self. DC maturation is central to the outcome of antigen presentation to T cells. Maturation of DCs is inhibited by physiological levels of calcitriol. Conditioning of bone marrow cultures with 10⁻¹⁰ M calcitriol resulted in accumulation of immature DCs with reduced IL-12 secretion and without induction of transforming growth factor β1. These DCs retained an immature phenotype after withdrawal of calcitriol and exhibited blunted responses to maturing stimuli (CD40 ligation, macrophage products, or lipopolysaccharide) (Griffin et al., 2001). Furthermore, DCs treated with calcitriol show an intermediate expression of molecules involved in T cell activation such as MHC-II and CD86, are resistant to maturation by pro-inflammatory stimulation without affecting IL-10 production, express significant high levels of PD-L1 (programmed death-1 ligand) upon activation, and possess the capacity to convert CD4(+) T cells into IL-10-secreting Treg capable of potently suppressing the proliferation of responder T cells (Xing et al., 2002; Unger et al., 2009).

Applicable vitamin D3 metabolites and derivatives thereof. In one specific embodiment, the active vitamin D3 metabolite 1,25-(OH)₂D3 (calcitriol) is used for the method of the present invention. In another specific embodiment, the relatively biologically inactive 25-OH-D3 (calcidiol) is used for the method of the present invention. In still another specific embodiment, vitamin D analogues with modifications in the side chain are used for the method of the present invention including but not limited to those reviewed by Plum and DeLuca (2010) such as alfacalcidol, paricalcitol, oxacalcitriol, doxercalciferol, falecalcitriol, calcipotriol, and tacalcitol, as well as those reviewed by Fletcher et al. (2012).

Calcipotriol. In a preferred specific embodiment, calcipotriol is used for the method of the present invention. As compared to calcitriol, calcipotriol has 100-200 times less effect on calcium metabolism including activation of calcium absorption and bone calcium mobilization (Kissmeyer and Binderup, 1991). An important advantage for the method of the present invention is the short half-life of calcipotriol in circulation which is measured in minutes (Kragballe, 1995). The rate of clearance (half-life of 4 min) was approximately 140 times higher for calcipotriol than for calcitriol. Furthermore, calcipotriol is rapidly metabolized and effects of the metabolites have been demonstrated to be 100 times weaker than those of the parent compound (Kissmeyer and Binderup, 1991). Due to these characteristics, calcipotriol has been used clinically for more than 10 years for topical treatment of psoriasis without systemic toxicity (for a review, see Plum and DeLuca, 2010).

Most important, the tolerance-promoting effects of calcipotriol are comparable to those of calcitriol (e.g., Al-Jaderi et al., 2013). In a recent study in mice, topical pretreatment with calcipotriol for 1 day followed by transcutaneous immunization within the treated skin area (stratum corneum was removed by tape stripping) with ovalbumin in the presence of a CpG oligonucleotide adjuvant (TLR9 agonist), priming of antigen-specific CD8(+) T cells was abolished and CD4(+)CD25(+) Tregs were induced (Ghoreishi et al., 2009). The suppressive effect of calcipotriol was found to be a local effect, as application of antigen to an area untreated with calcipotriol did not result in suppression.

### 7.2. Glucocorticoids

In another embodiment, the present invention utilizes glucocorticoids as additional tolerance-promoting immune modulators. Glucocorticoids, such as dexamethasone and prednisone, are widely used for their anti-inflammatory and immunosuppressive properties. These agents interact with the steroid receptor to down-regulate the expression of specific genes that regulate the inflammatory process. There are several proposed mechanisms to explain the inhibitory effects of glucocorticoids.

Within the cell, glucocorticoids act in three ways. First, the glucocorticoid-glucocorticoid receptor (GR) complex moves to the nucleus, where it binds as a homodimer to DNA sequences called glucocorticoid-responsive elements. The resulting complex recruits either coactivator or corepressor proteins that modify the structure of chromatin, thereby facilitating or inhibiting assembly of the basal transcription machinery and the initiation of transcription by RNA polymerase II. Second, regulation of other glucocorticoid-responsive genes involves interactions between the glucocorticoid-GR complex and other transcription factors, such as NF-κB. Glucocorticoids such as dexamethasone induce the expression of IκBα which binds to NF-κB in the cytosol and, thereby, blocks the nuclear localization signal. NF-κB is thus maintained in an inactive cytoplasmic complex so that the expression of genes involved in the pathogenesis of the immune response is reduced. The third mechanism is glucocorticoid signalling through membrane-associated receptors and second messengers (so-called nongenomic pathways) Evidence indicates that the glucocorticoid receptor inhibits inflammation through all three mechanisms: direct and indirect genomic effects and nongenomic mechanisms.

Glucocorticoids and the glucocorticoid receptor regulate a complex network that inhibits a variety of inflammatory pathways via several mechanisms such as expression of anti-inflammatory proteins, induction and inhibition of cytokines, inhibition of inflammatory receptors, reduced expression of adhesion molecules, and the induction of Tregs (for reviews, see Barnes, 2001; Rhen and Cidlowski, 2005; Longui, 2007; Robinson, 2010).

For the application of glucocorticoids as additional immune modulator it is an advantage that glucocorticoids exert immunosuppressive and anti-inflammatory effects in a variety of immune cells including dendritic cells, macrophages, T cells, eosinophils, mast cells, and neutrophils. However, there are marked differences in the response of different cells and of different cytokines to the inhibitory action of glucocorticoids, and these differences may depend on the relative abundance of transcription factors within different cell types.

The immunosuppressive and anti-inflammatory effects of glucocorticoids include:
a) Inhibition of NF-κB and AP-1. The repression of NF-κB- and activator protein-1 (AP-1)-dependent transcription is a major component of the glucocorticoid-mediated inhibition of gene expression. Both transcription factors play an important role in the induction of pro-inflammatory factors.
   NF-κB stimulates the transcription of cytokines, chemokines, cell-adhesion molecules, complement factors, and receptors for these molecules. NF-κB also induces the transcription of cyclooxygenase 2, an enzyme essential for prostaglandin production. Prostaglandins are derived from arachidonic acid. They both sustain homeostatic functions and mediate pathogenic mechanisms, including the inflammatory response.
   AP-1 (activator protein-1) is a transcription factor which is a heterodimeric protein composed of proteins belonging to the c-Fos, c-Jun, ATF (activating transcription factor) and JDP (jun dimerization partner) families. AP-1, functioning as a proinflammatory transcription factor, trans-activates a number of genes that are expressed in inflammation.
   The glucocorticoid receptor (GR) inhibits AP-1 target gene transcription. The cross-talk between the glucocorticoid receptor and AP-1 is mutual in that c-Fos, c-Jun, and JunD are blocked by ligand activated glucocorticoid receptor and, vice versa, glucocorticoid receptor function is impaired by overexpressed c-Fos and c-Jun.
b) Expression of anti-inflammatory proteins. Glucocorticoids suppress inflammation by increasing the synthesis of anti-inflammatory proteins. For example, corticosteroids increase in several cells the synthesis of annexin I (also called lipocortin-1), a 37-kDa protein that has an inhibitory effect on phospholipase A2 (PLA2) and, thereby, inhibits the production of arachidonic acid-derived eicosanoids (i.e., prostaglandins, thromboxanes, prostacyclins, and leukotrienes). Other anti-inflammatory proteins induced by glucocorticoids include IL-1 receptor antagonist (which inhibits the binding of IL-1 to its receptor), SLPI (which inhibits proteases, such as tryptase), neutral endopeptidase (which degrades bronchoactive peptides such as kinins), CC-10 (an immunomodulatory protein), the inhibitor of NF-κB (IκB-α), and MAPK phosphatase 1. Glucocorticoid-induced MAPK phosphatase 1 dephosphorylates and inactivates Jun N-terminal kinase, thereby inhibiting c-Jun-mediated transcription. Phosphorylated c-Jun homodimers and c-Jun-Fos heterodimers bind DNA sequences called activator protein 1 response elements and induce the transcription of inflammatory and immune genes.
c) Induction and inhibition of cytokines. Glucocorticoids are able to inhibit the transcription of many pro-inflammatory cytokines, such as IL-2 and IL-12, INF-γ and TNF-α. These inhibitory effects are due, at least in part, to an inhibitory effect on the transcription factors that regulate induction of these cytokine genes, including AP-1 and NF-κB. For example, eotaxin, which is important in selective attraction of eosinophils from the circulation into the airways, is regulated in part by NF-kB, and its expression in airway epithelial cells is inhibited by glucocorticoids. Furthermore, glucocorticoids inhibit IL-5, at least in part, by inhibiting the AP-1 component of NF-AT (nuclear transcription factor of activated T cells).
   On the other side, glucocorticoids induce transforming growth factor (TGF-β) secretion, which is able to reduce lymphocyte T activation and cell proliferation, and the expression of IL-10 (an anti-inflammatory cytokine). Glucocorticoid-induced expression of IL-10 is important for the treatment of allergic diseases, since in macrophages from asthmatic patients the expression of IL-10 is decreased, resulting in an increased expression of several inflammatory genes.
d) Inhibition of adhesion molecules. The expression of adhesion molecules which are important for the trafficking of inflammatory cells to sites of inflammation, such as vascular adhesion molecules (VCAM-1), intercellular adhesion molecules (ICAM), and E-selectin, are inhibited by glucocorticoids at the level of gene transcription.
e) Inhibition of inflammatory receptors. A number of receptors involved in the regulation of inflammatory gene, are also inhibited by glucocorticoids. For example, the gene for the NK₁-receptor, which mediates the inflammatory effects of tachykinins in the airways, has an increased expression in asthma and is inhibited by corticosteroids, probably via an inhibitory effect on AP-1. Glucocorticoids also inhibit the transcription of the NK₂-receptor, which mediates the bronchoconstrictor effects of tachykinins. Furthermore, glucocorticoids inhibit the expression of the inducible bradykinin B₁-receptor and bradykinin B₂-receptor.
f) Induction of apoptosis. The immunosuppressive and anti-inflammatory effects of glucocorticoids are also based in part on the induction of apoptosis of certain inflammatory cells. For example, dexamethasone was shown to preferentially deplete CD4(+) effector cells while sparing Treg cells (Chen et al., 2004b). Dexamethasone-induced apoptosis was also observed in B cells (Andreau et al., 1998), preferentially in B-2 cells, while B-1 cells are spared (Chen et al., 2014). Furthermore, glucocorticoids induce apoptosis in eosinophils. Eosinophil survival is dependent on the presence of certain cytokines, such as IL-5 and GM-CSF. Exposure to glucocorticoids blocks the effects of these cytokines and leads to apoptosis. In contrast, glucocorticoids decrease apoptosis in neutrophils and thus extend their survival (for a review, see Barnes, 2001) .
g) Promotion of peripheral Treg cell production and upregulation of FoxP3 and IL-10 expression in Treg cells. For the method of the present invention it is important that glucocorticoids also promote or initiate differentiation of naive T cells toward regulatory T cells and upregulate FoxP3 and IL-10 expression in Treg cells (for a review, see Robinson, 2010).
   In asthmatic patients receiving glucocorticoid treatment, FoxP3 mRNA expression was significantly increased and correlated tightly with IL-10 mRNA expression. The frequency of CD25(+) memory CD4(+) T cells and transient FoxP3 mRNA expression by CD4(+) T cells significantly increased after systemic glucocorticoid treatment (Karagiannidis et al., 2004).
   Recently, it was shown that glucocorticoid-induced leucine zipper (GILZ) promotes peripheral Treg cell production (Bereshchenko et al., 2014). This function involves the regulation of TGF-β signaling which is a requisite for the peripheral induction of FoxP3 in naive T cells and generation of peripheral Treg cells (Josefowicz et al., 2012; Yadav et al., 2013). Thus, GILZ mediates glucocorticoid-induced Treg generation by promoting TGF-β signaling.
h) Modulation of dendritic cells (DCs) by glcuocorticoids. DCs treated with dexamethasone show a stable, semi-mature phenotype with intermediate expression of molecules involved in T cell activation such as MHC-II and CD86, are resistant to maturation by pro-inflammatory stimulation without affecting IL-10 production, and possess the capacity to convert CD4(+) T cells into IL-10-secreting Treg capable of potently suppressing the proliferation of responder T cells (Xing et al., 2002; Unger et al., 2009).

Selection of suitable glucocorticoids. For the method of the present invention it is of interest, that the immunosuppressive and anti-inflammatory effects of glucocorticoids can be locally restricted to the site of allergen or autoantigen presentation in order to minimize adverse systemic side effects. Therefore, a short plasma half-life is advantageous. Glucocorticoids exhibiting a short plasma half-life (ranging between 30 min and 2 hours) and a relatively short biological half-life of 8-12 hours include cortisone and hydrocortisone, glucocorticoids exhibiting an intermediate plasma half-life (ranging between 2.5 and 5 hours) and an intermediate biological half-life of 18-36 hours include prednisone, prednisolone, methylprednisolone and triamcinolone, and glucocorticoids exhibiting a long plasma half-life (up to 5 hours) and a relatively long biological half-life of 36-54 hours include dexamethasone, betamethasone and fludrocortisone (for a review, see Longui, 2007).

However, most important for the method of the present invention is the glucocorticoid potency, which defines the capacity to elevate glycemia and which is proportional to the anti-inflammatory potency. In this respect cortisone and hydrocortisone exhibit a rather low potency, prednisone, prednisolone, methylprednisolone and triamcinolone an intermediate potency, whereas dexamethasone and betamethasone exhibit a rather high potency, which is 25-30-fold higher than that of cortisone or hydrocortisone (for a review, see Longui, 2007). Therefore, glucocorticoids with a high anti-inflammatory potency such as dexamethasone are preferred for the method of the present invention despite their relatively long plasma and biological half-lives.

### 7.3. Janus kinase inhibitors

Janus kinase inhibitors, also known as JAK inhibitors or jakinibs, inhibit the activity of one or more of the Janus kinase family of enzymes, thereby interfering with the JAK-STAT signaling pathway.

The biological significance of cytokine signaling through different JAK combinations (reviewed by Cutolo, 2013). In mammals, the Janus kinase family comprises four members: JAK1, JAK2, JAK3, and TYK2 (tyrosine kinase 2). JAK activation occurs upon ligand-mediated receptor oligomerization because two JAKs are brought into close proximity, allowing transphosphorylation.
JAK1/JAK3. Interleukin (IL)-2, IL-4, IL-7, IL-9, IL-15 and IL-21 use JAK1 and JAK3, and are involved in a) growth and maturation of lymphoid cells, b) differentiation and homeostasis of T cells and NK cells, c) B cell class switching, and d) inflammatory processes.
JAK1/JAK3. Interferon-γ (IFN-γ) uses JAK1 and JAK3, and is involved in a) antiviral responses, and b) inflammatory processes.
JAK1/TYK2. IL-10, IL-20, IL-22 and IFNs use JAK1 and TYK2, and are involved in a) antiviral and antitumor responses, and b) inflammatory processes.
JAK1/TYK2. IL-12 and IL-23 use JAK1 and TYK2, and are involved in a) differentiation and proliferation of Th17 cells, b) innate immunity, and c) inflammatory processes.
JAK1/JAK2/TYK2. IL-6, IL-11, IL-27 and G-CSF (granulocyte-colony stimulating factor) use JAK1 und JAK2, and are involved in a) T cell homeostasis, b) naive T cell differentiation, c) granulopoiesis, and d) inflammatory processes.
JAK2/JAK2. IL-3, IL-5, EPO (erythropoietin), TPO (thyreoperoxidase), GH (growth hormone) and PRL (prolactin) use JAK2, and are involved in a) erythropoiesis and myelopoiesis, b) megakaryocyte and platelet production, c) mammary development, and d) growth.

Differential inhibition of JAK-signalling by tofacitinib. The first JAK inhibitor to reach clinical trials was tofacitinib. Tofacitinib is a specific inhibitor of JAK3 and JAK1 and has little effect on other kinases (Karaman et al., 2008).

Using cell-free assays, the inhibitory potency of tofacitinib for JAK3 (1 nM) has been described as being 20-fold selective relative to JAK2 and 100-fold selective relative to JAK1 (Changelian et al., 2003).

In recent enzyme assays, however, the inhibitory potency of tofacitinib for recombinant human kinase protein JAK1 was only 2-fold lower (IC₅₀=3.2 nM) and for JAK2 2.5-fold lower (IC₅₀=4.1 nM) than for JAK3 (IC₅₀=1.6 nM) (Flanagan et al., 2010; Meyer et al., 2010).

Tofacitinib has little effect on TYK2 (IC₅₀=260 nM) (Ghoreschi et al., 2011), although in enzyme assays the inhibitory potency of tofacitinib for recombinant human kinase protein TYK2 was significantly higher (IC₅₀=34 nM) (Meyer et al., 2010).

Based on the different inhibitory potency of tofacitinib for the four members of the Janus kinase family in enzyme assays, lower concentrations of tofacitinib inhibit signalling via JAK1 and JAK3 (IL-2, IL-4, IL-7, IL-9, IL-15, IL-21, and IFN-γ), whereas higher concentrations of tofacitinib inhibit also signalling via JAK1 and TYK2 (IL-10, IL-12, IL-20, IL-22, IL-23 and IFNs), and via JAK1, JAK2 and TYK2 (IL-6, IL-11, IL-27 and G-CSF).

For the induction of tolerance according to the method of the present invention it is important, to inhibit JAK1/JAK3-mediated signalling, but to avoid inhibition of JAK1/TYK2-mediated signalling since IL-10 is essential for the induction of tolerance.

Therapeutic effects of JAK1/JAK3 inhibition. As a result of specific inhibition of JAK3 and JAK1 by tofacitinib, Th2 cell differentiation is blocked and, therefore, tofacitinib is effective in treating allergic diseases. Furthermore, Janus kinase (JAK) pathways are also key mediators in the immunopathogenesis of other immune diseases such as psoriasis and rheumatoid arthritis (RA). Several clinical studies have shown convincing therapeutic efficacy of tofacitinib in the treatment of these diseases. The therapeutic efficacy of tofacitinib is a result of its direct suppressive effect on the production of cytokines and proliferation of T cells (Maeshima et al., 2012).

Functionally, tofacitinib affects both innate and adaptive immune responses by inhibiting pathogenic Th17 cells and Th1 and Th2 cell differentiation (Ghoreschi et al., 2011). As demonstrated in a recent study, tofacitinib is also capble of inducing a tolerogenic phenotype in dendritic cells, thereby reducing the T cell stimulatory of DCs (Kubo et al., 2014). This effect of tofacitinib represents an important supporting mechanism for the induction of tolerance by allergen- or autoantigen-specific immunotherapy.

Hydrogel-based formulations of tofacitinib. In one embodiment, the present invention discloses hydrogel-based formulations of tofacitinib. Based on the solubility of tofacitinib citrate (MW 504.5) in water (2.9 mg/mL; 5.7 mM), tofacitinib citrate is used for the preparation of PLGA-PEG-PLGA hydrogel-based compositions.

Hydrogel-embedded tofacitinib formulations offer the possibility to use tofacitinib as supporting tolerance-promoting immune modulator at concentrations which provide therapeutic efficacy at the site of allergen or autoantigen presentation but minimize potential tofacitinib-mediated adverse effects. The hydrogel serves as sustained delivery system for tofacitinib at the site of allergen or autoantigen presentation and, thereby, eliminates peak serum levels of tofacitinib as observed after oral administrations. Furthermore, the short in vivo half-life of tofacitinib (2-3 h) minimizes systemic effects of tofacitinib upon diffusion and transport away from injected hydrogel-based compositions. As important is the application of find-me molecules in said compositions for attracting peripheral antigen-presenting cells including dendritic cells (DCs) and macrophages to s.c. injected hydrogel-based compositions. Tolerogenic modification of DCs in close proximity of such compositions requires lower amounts of tofacitinib than tolerogenic modification of distant DCs. Last not least, the application of tolerance-promoting adjuvants in said compositions supports the tolerance-inducing effect of tofacitinib by different mechanisms and will reduce the therapeutically effective dose of tofacitinib in said compositions.

Lowering the dose of tofacitinib without affecting its therapeutic efficacy is important since currently administered doses of tofacitinib for systemic treatment of arthritis is associated with potential serious adverse effects. For example, in a phase 3, randomized, double-blind, placebo-controlled clinical study with rheumatoid arthritis patients, there was an increased rate of serious infections during oral therapy with tofacitinib (at doses of 5 mg or 10 mg twice daily) as compared with placebo (Fleischmann et al., 2012a). In months 0 to 3 of treatment, a total of 330 patients (54.1%) had 701 adverse events, with similar frequencies across the patients treated with 5 mg or 10 mg tofacitinib. The most common of these adverse events were upper respiratory tract infection, headache, and diarrhea. Twelve patients (2.0%) discontinued the study drug during this period of treatment owing to serious adverse events, with similar frequencies across the two tofacitinib-treated patient groups (Fleischmann et al., 2012a). More serious immunologic and hematological adverse effects have also been noted resulting in lymphopenia, neutropenia, anemia, and increased risk of cancer and infection (https://pubchem.ncbi.nlm.nih.gov).

### 7.4. Antagonistic IL-4/IL-13 muteins.

In another embodiment of the invention, interleukin variants capable of antagonizing the activity of IL-4 and IL-13 are used as additional tolerance-promoting immune modulators. Any interleukin variant that functions as IL-4, IL-13 or IL-4/IL-13 antagonist and is suitable for administration in accordance with the method of the present invention may be employed. Interleukin-based antagonists do not need to completely abolish IL-4- or IL-13-induced biological activity to be useful. Rather, a given antagonist may reduce a biological activity of IL-4 and/or IL-13.

Antagonistic IL-4 mutants have been described (Kruse et al., 1992; Muller et al., 1994; US patent 6,028,176; US patent 5,723,118). Within the four helix bundle of human IL-4, tyrosine (Y124) on the D helix forms an important contact with the common γ-chain (γ_{c}). When Y124 is mutated to aspartic acid (Y124D), the resulting molecule binds to IL-4Rα with the same affinity as the wild-type IL-4, but has only 0.5% of the agonistic activity (Letzelter et al., 1998). Similarly, the arginine at position 121 of human IL-4 interacts with IL-13Rα1. Mutations of R121 generates mutants that can bind to IL-4Rα, but are antagonists of both IL-4- and IL-13-induced responses (Kruse et al., 1993). However, other or additional mutations of IL-4 may also be considered for generating IL-4 variants suitable for the method of the present invention. For example, by mutation of serine at position 125 of IL-4 mutants have been generated that can bind to IL-4Rα, but are antagonists of both IL-4- and IL-13-induced responses (Kruse et al., 1993). Suitable for the method of the present invention are antagonistic interleukin-4 variants with single, double and triple mutations, single mutations including but not limited to amino acid positions R121, Y124, and S125; double and triple mutations including but not limited to combinations of the above listed amino acid positions. Preferred are antagonistic IL-4 variants with a relatively short half-life to limit adverse effects distant from the site of allergen presentation.

The double mutant of human IL-4 (R121D/Y124D) has been shown to protect allergic cynomolgus monkeys from allergen-induced airways hyper-responsiveness when administered either subcutaneously or via nebulization. Furthermore, subcutaneous administration of this double mutant in monkeys for 6 weeks or more also reduced the cutaneous wheal response and circulating concentrations of allergen-specific IgE. The effect of the double mutant of IL-4 (R121D/Y124D) on late phase asthmatic response to allergen challenge in asthmatic patients has been evaluated recently in clinical studies (Wenzel et al., 2007). The studies demonstrate that dual inhibition of IL-4 and II-13 can positively affect the course of late asthmatic response after experimental allergen challenge. Treatment with the double mutant was associated with few adverse events, whether administered by subcutaneous injection (up to 30 mg for up to 13 weeks) or by inhalation (up to 60 mg for up to 4 weeks) in participants with atopic asthma or atopic eczema. However, it should be stressed that application of this double mutant for allergy immunotherapy according to the method of the present invention has not been disclosed.

More than 15 years ago animal experiments have demonstrated that inhibition of the IL-4/I1-13 receptor system can completely abrogate humoral immune response to allergen and development of allergic symptoms *in vivo* (Grunewald et al., 1998). Treatment of BALB/c mice with the murine IL-4 mutant Q116D/Y119D (the murine equivalent of the human IL-4 double mutant R121D/Y124D) before and after immunization with ovalbumin (OVA) completely inhibited synthesis of OVA-specific IgE and IgG1. In addition, the synthesis of specific IgG2a, IgG2b and IgG3 was suppressed, which may indicate the development of tolerance toward OVA. Immunization was performed by i.p. injection of 10 µg OVA in the presence of alum as an adjuvant. At day 0, the murine IL-4 mutant was applied by i.p. injection 2 hours pre- and post-OVA-immunization as a 50 µg dose each. From day 1 to 8 treatment was continued with 30 µg IL-4 mutant twice a day by i.p. injection. The authors concluded that the murine IL-4 mutant Q116D/Y119D inhibits antigen-specific humoral immune responses and allergic symptoms mediated either by IgE or IgG1 in vivo and, therefore, should be advantageous for therapy of atopic disorders and other Th2-dominated diseases. Although the results of this study are close to the method and the therapeutic aim of the present invention, the study of Grunewald et al. (1998) does not disclose how to provide a high local concentration of inhibitors for a prolonged period of time at the site of allergen presentation associated with a limited radius of inhibitory activity away from the site of allergen presentation due to rapid clearance. Grunewald et al. (1998) report the apparent induction of tolerance to the OVA allergen by starting the application of the murine IL-4 mutant Q116D/Y119D before immunization with OVA, which is not comparable to immunotherapy of patients with an existing allergy. For the induction of tolerance to allergens in individuals with a Th2-type immune phenotype only the method of the present inventions provides appropriate means to ensure a sufficient induction of Tregs in a local micro-environment that is free of Th1- or Th2-driving factors despite the presence of allergens.

IL-13 mutants useful for the method of the present invention include but are not limited to the IL-13 mutant E13K which is a powerful antagonist of human IL-13 and capable of partially inhibiting the responses of human IL-4 as well (Kioi et al., 2004). Glutamate at position 13 in IL-13 associates with IL-4Rα and a mutation to lysine decreases its binding to IL-4Rα. Although IL-13(E13K) prevents binding of IL-4Rα to IL-13Rα1, full inhibition of IL-4 responses cannot be achieved due to the availability of the alternative IL-4R complex, IL-4Rα : γ_{c}.

In a preferred embodiment, the human IL-4 double mutant R121D/Y124D is used for inhibition of IL-4- and IL-13-mediated pathways in accordance with the method of the present invention. The human IL-4 double mutant R121D/Y124D is able to antagonize the activity of IL-4 as well as that of IL-13 (Grunewald et al., 1998; Tony et al., 1994).

### 7.5. Inhibition of TNFR1-mediated effects by salicylates.

In another embodiment, salicylates including but not limited to aspirin (acetylsalicylic acid; ASA) and salicylic acid (SA) are used as inhibitors of TNFR1-mediated effects for the methods of the present invention.

Several studies have demonstrated that ASA and related salicylates have a spectrum of biochemical and pharmacological effects that are not related to COX inhibition. High doses of ASA and SA have been shown to interfere with the activation of critical transcription factors such as NF-κB (Koop and Ghosh, 1994) and activator protein 1 (AP-1; Dong et al., 1997). The inhibitory effect of salicylates is caused by activation of the p38 mitogen-activated kinase which leads to inhibition of TNF-α-induced IκBα phosphorylation and degradation. As a result of NF-κB inhibition, high doses of salicylates can interfere with Th1 cell differentiation and effector responses (e.g., Mazzeo et al., 1998).

An important finding was the observation that TNFR1-mediated activation of NF-κB is inhibited by high concentrations of sodium salicylate, but not TNFR2-mediated activation of NF-κB (Thommesen and Laegreid, 2005). Apparently, TNFR2 signaling involved in NF-κB activation proceeds independently of salicylate-sensitive signaling components, indicating distinct signaling pathways not shared with TNFR1. Inhibition of TNFR1-mediated activation of NF-κB requires high doses of salicylate (10-20 mM), but the inhibitory effect is highly reproducible (Thommesen and Laegreid, 2005).

Another major finding was the discovery that ASA and AS reduce IL-4 secretion and RNA expression in human CD4(+) T cells (Cianferoni et al., 2001). IL-4 inhibition is important for the induction of Tregs since GATA3 induced by IL-4 inhibits the expression of FOXP3, which is a requirement for inducible Treg differentiation (Mantel et al., 2007). The inhibitory effect on IL-4 expression occurs at the transcriptional level and is due to interference with the binding of a calcium-inducible factor to a proximal IL-4 promoter element upstream of, but not overlapping, the NF-κB-binding P1 element. IL-4 gene inhibition by ASA is not associated with reduced NF-κB activation (Cianferoni et al., 2001). As important is the observation that salicylate-mediated inhibition of IL-4 expression does not affect IL-2 expression (Cianferoni et al., 2001), an essential cytokine for up-regulating TNFR2 expression on Tregs (Chen and Oppenheim, 2010). Since TNF-α has been shown to selectively up-regulate the expression of the IL-2 receptor alpha-chain (CD25) on Tregs (Chen et al., 2007), both TNF-α and IL-2 generate a powerful mechanism for receptor amplification and synergistically up-regulate Treg suppressive activity.

The various inhibitory effects of ASA and related salicylates are concentration-dependent. A 50% inhibition by ASA requires a concentration of approx. 2 x 10⁻⁶ M for COX-1, appprox. 3 x 10⁻⁴ M for COX-2, approx. 1 x 10⁻³ M for IL-4 gene transcription, and approx. 3 x 10⁻³ M for NF-κB translocation (Cianferoni et al., 2001). Selective inhibition of TNFR1-mediated activation of NF-κB requires even higher doses of salicylates in the range of 1-2 x 10⁻² M (Thommesen and Laegreid, 2005).

However, the therapeutic range for ASA and SA has been restricted to 0.8-1.7 10⁻³ M, since salicylate-related toxicity (e.g., tinnitus) can occur with salicylate plasma levels as low as 1.2 x 10⁻³ M (Furst et al., 1987). Therefore, exploitation of COX-independent effects of salicylates for the induction of regulatory T cells requires novel application techniques that allow for high local concentrations of ASA and related salicylates well above the therapeutic range of systemically administered salicylates without the risk of increased side effects.

The present invention solves this problem by disclosing suitable matrices for sustained local delivery of ASA and related salicylates. Thereby, selective inhibition of TNFR1-mediated activation of NF-κB and effective inhibition of IL-4 gene transcription can be achieved at the site of antigen or allergen presentation, while upon diffusion away from the delivery site the salicylate concentration is rapidly decreased to systemically tolerable levels.

In a preferred specific embodiment, sodium salicylate, salicylamide or choline magnesium trisalicylate is used as inhibitor of TNFR1-mediated effects according to the methods of the present invention for patients suffering from aspirin-induced asthma (also known as aspirin-triad or aspirin-intolerant asthma, AIA). In these patients application of ASA for selective inhibition of TNFR1-mediated activation of NF-κB and inhibition of IL-4 gene transcription is contra-indicated. AIA refers to the development of acute bronchoconstriction, profuse rhinorrhea and skin flushing in asthmatic individuals following the ingestion of aspirin. AIA is likely to be mediated by a deviation of the arachidonic acid metabolic pathway toward excessive leukotriene production. Both cyclooxygenase and lipoxygenase are involved in the arachidonic acid metabolic pathway. The prevalence of AIA is 4.3% in Poland, 8.8% in Finland and 10.5% in Australia (for a review, see Gohil et al., 2010). While ASA is contra-indicated for patients with ASA, these patients can safely take sodium salicylate, salicylamide and choline magnesium trisalisylate, since these drugs are weak inhibitors of COX.

### 7.6. Inhibition of complement with peptides and peptidomimetics

In another embodiment, peptide and peptidomimetic complement inhibitors are used as additional immune modulators for the method of the present invention.

As a central component of the innate immune system, complement is a key player in the body's defense against invading microorganisms, but it is also involved in the clearance of autoantigens and apoptotic cells, and it is a vital participant in the induction of adaptive responses (for a review, see Ricklin et al., 2010).

Several studies have demonstrated that C3a and C5a can modulate DC and macrophage activation and function (for a review, see Zhou, 2012b). For example, C3aR-mediated signalling has been associated with the inhibition of TH2 responses by promoting IL-12 (a Th1-driving interleukin) production in DCs. Accordingly, in C3aR deficient mice the immune response upon epicutaneous introduction of antigen shifted towards TH2 responses (Kawamoto et al., 2004). In contrast, deficiency of C5 or C5aR inhibited TH2 immune responses in asthma, thus providing protection to antigen-challenged mice (Drouin et al., 2006; Köh1 et al., 2006).

Furthermore, using murine model of allergic asthma it has been demonstrated that C3a induced elevated levels of IL-17(+)CD4(+) cells in the lungs resulting in an IL-17-mediated late-phase asthmatic response and airway hyper-responsiveness via neutrophilic inflammation (Mizutani et al., 2012). Multiple treatments with a C3a receptor antagonist or anti-C3a mAb during the challenges inhibited the increase in IL-17(+) CD4(+) cells and suppressed the late-phase increase in airway resistance (AHR) and infiltration by neutrophils in bronchoalveolar lavage fluid (Mizutani et al., 2012).

C5a has also been demonstrated to drive Th17 cell differentiation. In genetically autoimmune-prone SKG mice (a mutant of the gene encoding ZAP-70 on the BALB/c background, which spontaneously develop CD4(+) T cell-mediated autoimmune arthritis), administration of mannan or -glucan, both of which activate serum complement, evoked Th17 cell-mediated chronic autoimmune arthritis (Hashimoto et al., 2010). In vitro, C5a produced via all three complement pathways (i.e., lectin, classical, and alternative), stimulated tissue-resident macrophages, but not dendritic cells, to produce inflammatory cytokines including IL-6. In vivo, C5a receptor (C5aR) deficiency in SKG mice inhibited the differentiation/expansion of Th17 cells after mannan or -glucan treatment, and consequently suppressed the development of arthritis. In vivo macrophage depletion also inhibited disease development in SKG mice (Hashimoto et al., 2010).

In another study, coincidental activation of complement and several TLRs in mice has been shown to promoted Th17 differentiation from anti-CD3/CD28 or antigen-stimulated T cells. The complement effect required C5a receptor, was evident at physiologically relevant levels of C5a, and could be demonstrated in cultured peritoneal macrophages as well as in the setting of antigen immunization. The Th17 cell-promoting activity in the serum correlated with IL-6 induction, since antibody neutralization of IL-6 abrogated the complement effect (Fang et al., 2009).

Complement-promoted Th17 cells were functionally competent in causing autoimmunity in an adoptive transfer model of experimental autoimmune encephalomyelitis (Fang et al., 2009). Therefore, blockade of C5aR is likely to be beneficial for controlling Th17-mediated inflammation and autoimmune disease.

Important are also the effects of C3aR and C5aR signalling on the induction of Tregs. DCs are able to expand antigen-specific CD4(+)CD25(+) regulatory T cells (Yamazaki et al., 2003; Kretschmer et al., 2005) and Peng et al. (2006) have demonstrated that C3^{+/+} and C3^{-/-} DCs (which are unable to generate C3a or C5a) have different abilities to elicit the development of Tregs. Co-culturing of CD4(+) T cells with allogeneic C3 deficient (C3^{-/-}) DCs for 9 days yielded a 4-fold higher level in T cells expressing the foxp3 gene (used as marker for CD4(+)CD25(+) regulatory T cells) as compared to those stimulated with C3^{+/+} DCs. In a very recent study, Strainic et al. (2013) have analyzed the phenotype of T cell responses when C3aR- and C5aR-mediated signals are absent on DCs, CD4(+) T cells or both. In vitro activation of naive CD4(+) T cells from mice deficient in both C3aR and C5aR resulted in a lower abundance of the pro-inflammatory cytokines IL-6 and IFN-γ, but a greater abundance of the anti-inflammatory cytokines IL-10 and TGF-β. Furthermore, the frequency of Foxp3(+) T cells proved to be much greater in cultures of C3ar1^{-/-}C5ar1^{-l-} T cells than in cultures derived from C3ar1^{-/-} mice, C5ar1^{-/-} mice or wild-type mice. These data indicate that signalling via the complement factors C3a and C5a regulates effector T cell responses and that the absence of local complement activation paves the way for a default pathway leading to Foxp3(+) Tregs.

Important for the method of the present invention is inhibition of locally synthesized complement component C3 at the site of allergen/autoantigen presentation. The liver is the primary source for the synthesis of C3. However, many other specialized cells have the capacity to synthesize C3 including activated macrophages, dendritic cells and epithelial cells (Pratt et al., 2002; Verschoor et al., 2003; Caroll, 2004; Peng et al., 2006; Strainic et al., 2008; Raedler et al., 2009). These cells synthesize C3 spontaneously or in response to cytokine stimulation.

Macrophages secrete all components of the complement system and antigens or inflammatory stimuli activate macrophages to produce substantial amounts of C3 (Morgan and Gasque, 1997). In particular, pro-inflammatory cytokines such as IL-6, tumor necrosis factor and interferon-γ stimulate macrophages to express the individual components.

Dendritic cells also synthesize and secrete C3 (Peng et al., 2006). However, in the absence of inflammation or danger signals DCs appear to produce only a small amount of C3, which is in accordance with the limited ability of DCs to stimulate an immune response under these conditions. In contrast, in some pathological conditions DC synthesis of C3 may be up-regulated by microbial factors and by non-specific inflammatory stimuli. Based on the observation that DC synthesis of C3 is essential for full T cell activation (Peng et al., 2006), DC synthesis of C3 appears to be an important characteristic of DC maturation and up-regulation of DC synthesis of C3 during inflammation and infection could enhance the antigen-presenting capacity of DCs.

Suitable inhibitors of the complement system for the present invention are low molecular weight inhibitors derived from peptides (including peptidomimetics), aptamers and structures selected from compound libraries and optimized derivatives thereof (for a review, see Qu et al., 2009). Preferred low molecular weight inhibitors of the complement system include but are not limited to inhibitors targeting a) complement protein-protein interactions such as C1q- and C3-specific inhibitors, b) serine proteases such as inhibitors of C1s, factor B and C2, and c) anaphylatoxin receptors such as antagonists of C3aR and C5aR (for reviews, see Qu et al., 2009; Wagner and Frank, 2010). Most preferred for the method of the present invention are low molecular weight inhibitors targeting complement component C3 and the anaphylatoxin receptors C3aR and C5aR.

Local intervention at the central level of C3. Intervention at the central level of C3 is an attractive strategy because this approach can effectively modulate the production of all the critical complement mediators.

One preferred low molecular weight inhibitor targeting complement component C3 is the 13-residue cyclic peptide (H-I [CWQDWGHHRC] T-NH₂) which is able to bind selectively to primate C3 and its C3b and C3c fragments, and to inhibit cleavage of C3 by C3 convertases of both the classical and the alternative pathway (Sahu et al., 1996; Ricklin and Lambris, 2008). The cyclic peptide, named Compstatin, is more efficacious in inhibiting the alternative pathway than the classical pathway which is due to the fact that it binds to both C3 and C3b in the alternative pathway C3 convertase, whereas it cannot bind to the classical pathway C3 convertase that involves C4b.

Another preferred low molecular weight inhibitors targeting complement component C3 are derivatives of the 13-residue cyclic peptide (H-I[CVVQDWGHHRC] T-NH₂). As revealed by analysis of alanine substitution analogues, substitution of valine⁴, histidine⁹, histidine¹⁰, and arginine¹¹ result in minimal change in the activity (Morikis et al., 1998). Substitution of valine⁴ for tryptophan has been shown to establish stronger hydrophobic interactions between the peptide and C3c. Methylation of the tryptophan indole nitrogen further strengthened this hydrophobic interaction (Katragadda et al., 2006).

Compstatin and its derivatives have been shown to be safe and effective in a series of ex vivo and in vivo experiments in which the effect of complement inhibition under different conditions was studied including transplantation (Fiane et al., 1999; Tjernberg et al., 2008), bio-incompatibility (Nilsson et al., 1998; Schmidt et al., 2003; Lappegard et al., 2005; Lappegard et al., 2008), and inflammation (Soulika et al., 2000: Mollnes et al., 2002).

To investigate the impact of anti-complement therapy on human T-cell activation, Compstatin was used to inhibit C3 activation. The study demonstrated that inhibition of C3 reduces Th1/Th17 polarization. The frequency of cells producing IFN-γ (Th1), IL-4 (Th2), IL-17 (Th17), IL-2 and TNF-α was significantly reduced among activated CD4(+) cells in the presence of Compstatin. Compstatin treatment decreased the proliferation of both CD4(+) and CD8(+) T cells upon TCR stimulation. However, Compstatin did not affect the production of IL-2 and TNF-α in activated CD8(+) T cells. Furthermore, the differentiation of CD8(+) T cells into distinct memory and effector subsets remained intact (Ma et al, 2014).

Recently, a Compstatin derivative successfully completed a phase I clinical study under the name POT-4 (Potentia Pharmaceuticals, Inc.) for the treatment of age-related macular degeneration (Francois et al., 2009).

Inhibition of C3a-receptor signalling. Therapeutic intervention of C3a/C3aR interactions is attractive, since C3a has been linked to various pro-inflammatory processes including chemotaxis, increases of vascular permeability, release of vasoactive amines, and activation of leukocytes such as macrophages, mast cells and eosinophils (for a review, see Gerad and Gerad, 2002). Furthermore, C3aR knock-out mice are characterized by decreased airway hyper-responsiveness (Drouin et al., 2002) and KO guinea pigs show decreased allergic responses (Bautsch et al., 2000). In humans, asthmatic patients have elevated levels of C3a in the broncho-alveolar fluid (Humbles et al., 2000) and in the plasma (Nakano et al., 2003). As demonstrated in a very recent study, signalling via C3a and C5a regulates effector T cell responses (Strainic et al., 2013).

Preferred low molecular weight C3aR antagonists include but are not limited to those with scaffolds featuring a) an arginine moiety (arginine derivatives) or b) an amino-piperidine linker and a pyridine moiety (amino-piperidine derivatives). Arginine derivatives include but are not limited to N²-[(2,2-dipheneylethoxy)acetyl]-L-arginine (SB290157; Ames et al., 2001), and those described by Denonne et al. (2007a). Amino-piperidine derivatives include but are not limited to those described by Denonne et al. (2007b).

In a preferred embodiment, the arginine derivative SB290157 is used for the method of the present invention. SB290157 has been shown to inhibit C3a-induced calcium mobilization in human neutrophils (IC₅₀: 28 nM) and C3a-induced receptor internalization in human neutrophils. The in vivo therapeutic efficacy of SB290157 has been demonstrated in several disease models including lung inflammation in guinea pigs (Ames et al., 2001), arthritis in rats (Ames et al., 2001), intestinal ischaemia/reperfusion injury in rats (Proctor et al., 2004), lupus nephritis in mice (Bao et al., 2005a), and allergic asthma in mice (Baelder et al., 2005). SB290157 has a short in vivo half-life which is favorable for the method of the present invention.

Inhibition of C5a-receptor signalling. Therapeutic intervention of C5a/C5aR interactions can be beneficial for various pathological conditions including inflammatory, autoimmune, and neurodegenerative disorders (for a review, see Lee et al., 2008).

Suitable low molecular weight C5aR antagonists include but are not limited to a) peptidomimetics such as linear C089 (Konteatis et al., 1994), cyclic PMX53 (Finch et al., 1999), cyclic PMX205 (March et al., 2004), and linear JPE1375 (Schnatbaum et al., 2006), b) low molecular weight antagonists that are not based on peptides such as W-54011 (Sumichika et al., 2002), NDT9520492 (Waters et al., 2005), NGD2000-1 (Lee et al., 2008), CP-447,697 (Blagg et al., 2008), and NDT9513727 (Brodbeck et al., 2008), and c) nucleic acid-based aptamers such as those described by Biesicker et al. (1999).

In a preferred embodiment, peptidomimetic C5aR antagonists including C089, PMX53, PMX205 and JPE1357 are employed for the method of the present invention. PMX53, PMX205 and JPE1375 are analogs of the linear hexapeptide C089 (NMeFKPdChaWdR) which was derived from the C-terminus of C5a. Residues in position 1, 4 and 6 appear to be important for binding, while position 5 is responsible for the antagonist activity. The sequence P-dXaa is critical for the reverse turn structure of these peptidometic C5aR antagonists, which is further stabilized by a lactam ring in the cyclic analogs PMX53 and PMX205. Hydrophobic substitutions at the C-terminus led to JPE1375 which provides increased receptor selectivity as compared to PMX53.

The in vivo therapeutic efficacy of these peptidomimetic C5aR antagonists has been demonstrated in various disease models (for a review, see Qu et al, 2009). For example, the in vivo therapeutic efficacy of C089 has been demonstrated in disease models of allergic asthma in rats (Abe et al., 2001) and thrombotic glomerulonephritis in rats (Kondo et al., 2001). The in vivo therapeutic efficacy of PMX53 has been demonstrated in several disease models including sepsis in mice (Huber-Lang et al., 2002), renal ischaemia/reperfusion injury in rats (Arumugam et al., 2003), hepatic ischaemia/reperfusion injury in rats (Arumugam et al., 2004), intestinal ischaemia/reperfusion injury in rats (Proctor et al., 2004), ruptured abdominal aortic aneurysm in rats (Harkin et al., 2004), inflammatory bowel disease in rats (Woodruff et al., 2005), lupus nephritis in mice (Bao et al., 2005b), 3-nitropropionic acid-induced Huntington's disease in rats (Woodruff et al., 2006), abdominal pain in mice and rats (Ting et al., 2008), and tumor growth in mice (Markiewski et al., 2008). Furthermore, PMX53 has been shown to be safe and well tolerated in Phase 1 clinical studies for rheumatoid arthritis and psoriasis (Köhl, 2006b).

### 7.7. Aptamer-based inhibitors of pro-inflammatory cytokines.

In another specific embodiment, aptamer-based inhibitors of pro-iflammatory cytokines are used for the method of the present invention.

In allergic and autoimmune diseases interleukins play an important role. For example, allergens are recognized and processed by dendritic cells that drive Th2 differentiation and secretion of multiple interleukins (IL) including but not limited to IL-4, IL-5, IL-9, IL-13, IL-17, and IL-25. The many functions of these interleukins include induction of B cells by IL-4 and IL-13 to produce IgE, promotion of development, recruitment and survival of eosinophils by IL-5, activation of mast cells and induction of mucus hypersecretion by IL-9, and promotion of influx of lymphocytes and neutrophils by IL-17 family members. Furthermore, results of studies in mouse models and in humans have identified a key role of IL-17 family members in the pathogenesis of autoimmunity, including rheumatoid arthritis (RA) and multiple sclerosis (MS).

The demonstration that these interleukins contribute to local and systemic aspects of the pathogenesis of allergic and autoimmune diseases, suggest that local inhibition of these interleukins at the site of allergen/autoantigen presentation will improve the efficacy of allergen- or autoantigen-specific immunotherapy (for reviews, see; Catley et al., 2011; Miossec and Kolls, 2012; Petersen and Lukacz, 2012; Polosa and Casale, 2012).

However, considering the complicated interactions of multiple interleukins in allergic and autoimmune diseases, inhibition of one interleukin will not be sufficient for efficient support of allergen- or autoantigen-specific immunotherapy. There are multiple pro- and anti-inflammatory cytokines, which exhibit overlapping actions and redundancy, making the overall effect extremely difficult to predict (for a review, see Gibeon and Menzies-Gow; 2012). Therefore, for the method of the present invention, a combined anti-interleukin therapy at the site of allergen/autoantigen presentation is preferred to guarantee significant efficacy of this adjuvant approach.

For the application of interleukin inhibitors as additional immune modulators it is important that the interleukin inhibitors exhibit a short plasma half-life to minimize potential adverse effects mediated by interaction of the therapeutics with targets away from the site of allergen or autoantigen presentation. This requirement is even more important for a combined anti-interleukin therapy, since potential adverse effects mediated by interaction of the therapeutics with targets away from the site of allergen or autoantigen presentation are likely to be potentiated by a combination of anti-interleukin therapeutics staying in circulation for a long time.

In one embodiment, nucleic acid-based aptamers are employed as interleukin inhibitors for the method of the present invention. Aptamers are nucleic acid binding species (typically 12-80 nucleotides long) generated by iterative rounds of in vitro selection, a process that is called SELEX (systematic evolution of ligands by exponential enrichment) (for a review, see Meyer et al., 2011). Typically, selected aptamers bind very tightly (up to the picomolar range) and specifically to their targets. Aptamers can also discriminate between closely related protein targets.

Aptamers have several key features that make them particularly well suited as reagents for the method of the present invention. First, aptamers are relatively small and can readily access sites which are difficult to target with large molecules such as the immunological synapse that is formed between the antigen presenting cells and the T cells. Another advantage for the present invention is the possibility to engineer the stability of aptamers towards degrading nucleases, thereby determining the period of their inhibitory activity. Furthermore, aptamers are selected entirely in vitro, their synthesis has been automated, and they can easily be chemically modified. In addition, they can be stored and shipped without problems, because the stability of DNA aptamers, in particular, is almost infinite. Importantly, they are not immunogenic (for reviews, see Keefe et al., 2010; Zhou et al., 2012; Shigdar et al., 2013).

Preferred interleukin inhibitors for the present invention are non-modified or slightly modified nucleic acid-based aptamers including RNA aptamers and DNA aptamers. Most preferred are nucleic acid-based aptamers with short plasma half-lives, ranging between 1 hour and 6 hours, although aptamers with a shorter and a longer half-life are also applicable for the present invention.

DNA and RNA aptamers are functionally similar. The basis of the specific three-dimensional interaction is the structure of the aptamer, which may include stem, internal loop, hairpin, bulges, pseudoknot, kissing complex, or G-quadruplex motifs. Compared to DNA aptamer, RNA aptamer could form more diverse three-dimensional structures, probably providing strong binding affinity. Although DNA aptamers have better chemical stability, RNA aptamers have better conformational stability due to strong intra-strand RNA-RNA interactions.

Compared to RNA aptamer, a major benefit of DNA aptamers is that they can be generated in a large scale by solid-phase chemical synthesis with a cheaper price. Commercial solid-phase synthesis provides RNAs of less than 60 nucleotides (nt), but for DNA up to about 400 nt can be synthesized with minimal side reactions.

Both RNA and DNA aptamers are inherently susceptible to nucleases present in human serum. While unmodified RNA has a half-life of seconds in human serum, DNA aptamers generally have a half-life of approximately 60 min (White et al., 2000). The main difference between DNA and RNA is the sugar present in the molecules. Desoxyribose sugar in DNA is less reactive due to C-H bonds, and its smaller grooves structures make it more stable and harder for enzymes to attack DNA. In contrast, ribose sugar in RNA is more reactive and not stable.

Useful strategies to delay enzymatic degradation include but are not limited to the use of phosphorothioate nucleotides and protective modifications at the 2'-position of the sugar (e.g., incorporation of amino, fluoro, alkyl and thio groups). For example, 2'-aminopyrimidine-modified RNA has been shown to remain stable for days. Modifications can also be applied to the ends of aptamers to confer greater stability. For example, a 3'-3' linkage can be added to prevent 3'-exonuclease degradation. Furthermore, protective modifications include also the use of inverted terminal cap structures. The structure of locked RNA aptamers is locked in place through a methylene bridge. It has been demonstrated that these types of modification are capable to increase serum stability of RNA aptamers to more than 81 h (Adler et al., 2008).

Aptamer-mediated inhibition of IL-5 or the IL-5 receptor α. In one specific embodiment, nucleic acid-based aptamers are employed as inhibitors of IL-5 or the IL-5 receptor α for the method of the present invention.

Eosinophils in the sputum are a hallmark of asthma. Eosinophil maturation, recruitment into the airways, and eosinophil survival are under the influence of IL-3, IL-5, and GM-CSF. However, it is IL-5 on which most attention has been focused for inducing and maintaining airway eosinophilia in asthmatic patients. Studies in many antigen-challenged animal models, including non-human primates, have indicated a sentinel role for IL-5 in the airway eosinophilic response (for a review, see Holgate, 2011).

Aptamer-mediated inhibition of IL-4 and IL-13 or the shared IL-4Rα/IL-13Rα1 complex. In another specific embodiment, nucleic acid-based aptamers are employed as inhibitors of IL-4 and IL-13 or the shared IL-4Rα/IL-13Rα1 complex for the method of the present invention.

IL-4 and IL-13 have pivotal roles in the development and regulation of T_{H}2 cells (IL-4 only), B-cell IgE isotype switching, mast cell IgE receptor expression, vascular cell adhesion molecule regulation involved in the microvascular recruitment of eosinophils and basophils, mucous metaplasia, and fibrosis. Both IL-4 and IL-13 induce their effects by signaling through the IL-4 receptor (IL-4R) α/IL-13 receptor (IL-13R) α1 complex and activation of signal transducer and activator of transcription 6. A truncated IL-13α2 receptor has only limited signaling capacity, although it does serve as a decoy to regulate IL-4 signaling (for a review, see Holgate, 2011).

Aptamer-mediated inhibition of IL-17A and IL-17F or the receptor units 17RA and IL-17RC. In another specific embodiment, nucleic acid-based aptamers are employed as inhibitors of IL-17A and IL-17F or the receptor subunits IL-17RA and IL-17RC for the method of the present invention.

IL-17A and IL-17F are both covalent homodimers. They also form IL-17A/IL-17F heterodimers. IL-17A, IL-17F and IL-17A/IL-17F heterodimers signal through the same receptor subunits including IL-17RA and IL-17RC (also known as IL-17RL), which together form a heteromeric complex.

Both IL-17A and IL-17F are expressed by a variety of cells including Th17 cells, CD8(+) T cells, CD4(+) T cells, γδ T cells, NK cells, and NKT cells (for a review, see Gaffen, 2009).

Cytokines that promote the induction of IL-17 include IL-23, IL-1 and IL-6 (Jones et al., 2012). IL-23 is a heterodimeric cytokine composed of an IL-12p40 subunit that is shared with IL-12 and the IL-23p19 subunit. Recently, bronchial epithelial cells have been identified as a novel cell source of IL-17F in response to IL-33 (Fujita et al., 2012).

IL-17A and IL-17F drive inflammation (neutrophil recruitment), immunity to extracellular pathogens and autoimmunity (IL-17A has a more important role in driving autoimmunity than IL-17F). However, IL-17A and IL-17F have distinct biological effects, probably due to the more potent strength of signalling by IL-17A. IL-17F-induced responses are 10- to 30-fold weaker in terms of downstream gene activation than those of IL-17A, with IL-17A/IL-17F heterodimers acting at an intermediate level. As IL-17RA and IL-17RC have different affinities for IL-17A and IL-17F, it is plausible that different receptor complexes exist with varying ratios of IL-17RA and IL-17RC that have different ligand preferences (for a review, see Gaffen, 2009).

Evidence shows that IL-17A is highly up-regulated at sites of inflammatory tissues of autoimmune diseases and amplifies the inflammation through synergy with other cytokines, such as TNF-α. Furthermore, several studies have demonstrated a critical role for IL-17A in the pathogenesis of variety of inflammatory autoimmune diseases, such as rheumatoid arthritis (RA) and multiple sclerosis (MS).

Involvement of IL-17A in the pathogenesis of autoimmune arthritis has been demonstrated in several studies (Lubberts, 2003; Miossoec, 2003). IL-17-deficient mice showed a suppression of immune induction of collagen-induced arthritis (Nakae et al., 2003). Furthermore, IL-17 receptor deficiency has been demonstrated to result in impaired synovial expression of IL-1 and matrix metalloproteinases 3, 9, and 13 and to prevent cartilage destruction during chronic reactivated streptococcal cell wall-induced arthritis (Koenders et al., 2005). Neutralizing endogenous IL-17 with the use of an extracellular domain of IL-17R and Fc fusion protein or with anti-IL-17 antibody treatment suppressed the early onset, as well as a later stage, of collagen-induced arthritis (Lubberts et al., 2001; Lubberts et al., 2004). In summary, these data indicate an important role of IL-17 in the sensitization stage of autoimmune arthritis and suggest the involvement of IL-17 in the progression of arthritis.

Furthermore, the development of experimental autoimmune encephalomyelitis (EAE) is significantly suppressed in IL-17^{-/-} mice. These animals exhibit delayed onset, reduced maximum severity scores, ameliorated histological changes, and early recovery (Komiyama et al., 2006). A monoclonal Ab (mAb) with specificity for IL-17A also prevented EAE development (Uyttenhove and Van Snick, 2006), and therapeutic neutralization of IL-17 with IL-17-receptor-Fc-protein in acute EAE ameliorated clinical symptoms (Hofstetter et al., 2005).

Importantly, promising results have been shown also in initial clinical trials of monoclonal antibodies against IL-17 or its receptor (IL-17R) to block IL-17-mediated function in treating autoimmune patients with psoriasis, RA and MS (for a review, see Zhu and Qian, 2012).

Elevated IL-17A concentrations have been found in the lung and blood of allergic asthma patients and linked to the severity of asthma. Furthermore, poly-sensitized allergic rhinitis patients (61 patients and 30 controls) exhibit higher IL-17A-producing CD4(+) T cell levels and eosinophil counts (Tsvetkova-Vicheva et al., 2014).

Both IL-17A and IL-33 contribute to IgE-mediated neutrophilic inflammation in mice. Compared with intra-tracheal administration of IL-33 or IL-17A alone, the combination exacerbated neutrophilic inflammation, airway hyper-responsiveness (AHR), and infiltration by alveolar macrophages expressing CXCR2 (Mizutani et al., 2014).

IL-17F is expressed in the airway of asthmatics and its expression level correlates with disease severity. IL-17F is also involved in neutrophilic airway inflammation (Ota et al., 2014). Pulmonary transfer of an IL-17F expression construct in mice resulted in a significant increase in the number of neutrophils in bronchoalveolar lavage fluids (Oda et al., 2005). Mucosal transfer of the IL-17F expression construct in OVA-sensitized mice enhanced the levels of pulmonary neutrophilia, but not eosinophilia.

Aptamer-mediated inhibition of IL-23 or the IL-23 receptor. In another specific embodiment, nucleic acid-based aptamers are employed as inhibitors of IL-23 or the IL-23 receptor for the method of the present invention.

IL-23 is a heterodimeric cytokine composed of an IL-12p40 subunit that is shared with IL-12 and the IL-23p19 subunit. The IL-23 receptor is composed of IL-12R β1 and IL-23R.

IL-23 is a member of the IL-12 family of cytokines with pro-inflammatory properties. IL-23 promotes the induction of IL-17. Its ability to potently enhance the expansion of T helper type 17 (Th17) cells indicates the responsibility for many of the inflammatory autoimmune responses (for a review, see Tang et al., 2012).

IL-23 is one of the critical cytokines in experimental autoimmune encephalomyelitis (EAE) development and is currently believed to be involved in the maintenance of encephalitogenic responses during the tissue damage effector phase of the disease (Thakker et al., 2007). Anti-IL-23p19 treatment reduced the serum level of IL-17 as well as CNS expression of IFN-γ, IL-10, IL-17, IL-6, and TNF mRNA. In addition, therapeutic treatment with anti-IL-23p19 during active disease inhibited proteolipid protein (PLP) epitope spreading and prevented subsequent disease relapse (Chen et al., 2006).

IL-23 regulates the proliferation of Th17 cells and induces IL-17 production from Th17 cells. Then IL-17 stimulates IL-1, TNF-α and receptor activator of NF-κB ligand (RANKL) expression, leading to the aggravation of the synovial inflammation, and joint destruction. IL-23 specific targeting by active immunization has been shown to improve collagen-induced arthritis (CIA) (Assier et al., 2010).

Aptamer-mediated inhibition of IL-25 (IL-17E) or the receptor units IL-17RA and IL-17RB. In another specific embodiment, nucleic acid-based aptamers are employed as inhibitors of IL-25 or the receptor units IL-17RA and IL-17RB for the method of the present invention.

IL-25 is a homodimer, binds a receptor complex composed of IL-17RB (also known as IL-25R or IL-17Rhl) and IL-17RA, and activates the NF-kB pathway. IL-25 is expressed by a variety of cells including intraepithelial lymphocytes, lung epithelial cells, alveolar macrophages, eosinophils, basophils, mast cells, Th2 cells, and NKT cells (for a review, see Gaffen, 2009).

IL-25 is a Th2 cell-promoting cytokine, induces TH2 cell differentiation and response with the up-regulation of Th2 cytokines as IL-4, IL-5, and IL-13. IL-25 also suppresses Th17 cell responses and development by the induction of IL-13 in dendritic cells or by inhibiting IL-23 production in macrophages (Gaffen, 2009; Barlow and McKenzie, 2009).

A recent study in mice demonstrated that chronic airways exposure to IL-25 alone is sufficient to induce allergen- and IgE-independent asthma-like airways inflammation, re-modelling and hyper-responsiveness (Yao et al., 2014). Apparently, IL-25 is a key molecular target in asthma, irrespective of the coexistence of IgE-dependent mechanisms.

In an OVA-induced pulmonary inflammation model, IL-25 mRNA expression increased in the lung after aerosolized OVA challenge, and antagonism of IL-25 using either a soluble IL-17RB-Fc protein or an antagonistic Ab to IL-25 inhibited many of the components of the allergen-induced Th2-type pulmonary inflammation (Tamachi et al., 2006; Ballentyne et al., 2007).

Several studies have demonstrated increased expression of IL-17E and its receptor in the human asthmatic bronchial mucosa at baseline and following allergen challenge (e.g., Corrigan et al., 2011). Furthermore, the expression of IL-17RA and IL-17RB on eosinophils in allergic asthma patients is increased suggesting that IL-25 may activate eosinophils during allergic inflammation (Tang et al., 2014).

Aptamer-mediated inhibition of IL-33 or the receptor units IL-1RL1 and IL-IRAcP. In another specific embodiment, nucleic acid-based aptamers are employed as inhibitors of IL-33 or the receptor units IL-1RL1 (also called ST2) and IL-1 receptor accessory protein (IL-IRAcP) for the method of the present invention.

The gene sequence and structure of IL-33 are similar to those of IL-1β and IL-18 which belong to the IL-1 family. The IL-33 receptor is a heterodimer comprised of IL-1RL1 (also called ST2) and IL-1 receptor accessory protein (IL-IRAcP).

IL-33 is expressed by many cells and tissues, including the stomach, brain, spleen, heart, bronchial epithelial cells, fibroblasts, smooth muscle cells, keratinocytes, macrophages and DCs (Schmitz et al., 2005). The ST2 receptor is highly expressed on mast cells, macrophages, hematopoietic stem cells, NK cells, NKT cells, eosinophils, basophils, and fibroblasts (for a review, see Farahani et al., 2014).

IL-33 is one of the most important cytokines responsible for Th2 immune deviation. IL-33 induces the differentiation of naive CD4 (+) T cells to IL-5 (+) IL-4 (-) CD4 (+) Th cells, independently of IL-4, STAT-6 and GATA-3, which are important factors for the typical Th2 cell differentiation (Kurowska-Stolarska et al., 2008). Similarly, differentiation of human CD4(+) cells in vitro in the presence of IL-33 enhanced antigen-dependent IL-5 and IL-13 production (Smithgall et al., 2008). In addition, IL-33 is a chemo-attractant for Th2 cells, recruiting Th2 cells to lymph nodes and tissue (Komai-Koma et al., 2007). IL-33 can also influence DC maturation and activity, leading to their enhanced expression of major histocompatibility complex-II, CD86 and IL-6. These activated DCs, when cultured with naive CD4 + T cells, lead to their differentiation in a fashion characterized by production of IL-5 and IL-13 (Rank et al., 2009).

Recent studies reported the correlation of IL-33 with rheumatic diseases, and most of them found that the IL-33 expression levels were consistent with disease activity and development. Evidence has indicated that IL-33-related treatment may ameliorate the pathogenic conditions and attenuate disease progression of rheumatic diseases (for a review, see Duan et al., 2013).

In experimental autoimmune encephalomyelitis, IL-33 attenuated the disease by switching a predominantly pathogenic Th17/Th1 response to Th2 activity, and by polarization lymph node and splenic macrophages to anti-inflammatory M2 macrophages (Jiang et al., 2012).

Exposure of mice to exogenous IL-33 results in airway hyper-responsiveness and airway goblet-cell hyperplasia in a lymphocyte-independent process Direct exposure to IL-33 results in epithelial hypertrophy and mucus accumulation in bronchial structures.

Blockade of IL-33 (anti-IL-33) and soluble ST2 meliorated airway inflammation in a murine OVA model of allergic asthma. Both treatments reduced total cell counts and eosinophil counts in BAL fluid and AHR to methacholine. The Th2 cytokines such as IL-4, IL-5, and IL-13 in BAL fluid were also significantly decreased after both treatments. However, there was no change in the level of TNF-β and IL-10 after each treatment (Lee et al., 2014). Furthermore, a recent study has demonstrated that IL-17A promotes the exacerbation of IL-33-induced airway hyper-responsiveness by enhancing neutrophilic inflammation via CXCR2 signaling in mice (Mizutani et al., 2014).

### 8. Liposomal formulations of additional tolerance-promoting immune modulators

In another embodiment, the present invention provides also compositions for allergen-specific SCIT and autoantigen-based immunotherapy comprising one or more additional liposomal tolerance-promoting immune modulator. Preferred are phosphatidyl-L-serine (PS)-liposomes containing one or more tolerance-promoting immune modulator.

Applicable compositions for additional liposomal tolerance-promoting immune modulators according to the method of the present invention include but not limited to hydrogel-, alum-and micro crystalline tyrosine-based compositions. Preferred compositions are based on PLGA-PEG-PLGA hydrogels.

Experimental studies with liposomal formulations of glucocorticoids and vitamin D3 have demonstrated that liposomal formulations of these immune modulators exert an altered mode of action as compared to non-liposomal formulations. For example, a recent study has demonstrated that liposomal encapsulation augments the therapeutic potency of glucocorticoids as they ameliorated experimental autoimmune encephalomyelitis (EAE) to the same extent as free glucocorticoids, but at strongly reduced dosage and application frequency. Importantly, this was accompanied by an altered mode of action. Unlike free glucocorticoids, which mainly target T lymphocytes during EAE therapy, liposomal glucocorticoids only marginally affect T cell apoptosis and function. In contrast, liposomal glucocorticoids efficiently repressed pro-inflammatory macrophage functions and upregulated anti-inflammatory genes associated with the alternatively activated M2 phenotype (Schweingruber et al., 2011).

Using PS-liposomes for liposomal formulations of vitamin D3 or derivatives thereof, glucocorticoids, and salicylates, inhibition of the NF-κB pathway in DCs and macrophages is the primary mode of action of these immune modulators due to the preferred uptake of PS-liposomes by APCs. Using PS-liposomes for liposomal formulations of tofacitinib, inhibition of the JAK1/JAK3-mediated pathways in DCs and macrophages is the primary mode of action of tofacitinib. Specific targeting of APCs by liposomal formulations of tolerance-promoting immune modulators can be further optimized by using PS-liposomes with encapsulated find-me molecules such as ATP and/or UTP.

NF-κB inhibitors are known to inhibit the maturation process of DCs, thereby generating tolerizing DCs which are capable of inducing regulatory T cells (for reviews, see Thomas, 2013; Gross and Wiendl, 2013; Van Brussel et al., 2014; Mackern-Oberti et al., 2015). If allergens or antigens are presented by tolerizing DCs, allergen- or antigen-specific Tregs are induced which have the potential to suppress allergic and autoimmune diseases. A recent study has demonstrated that phosphatidylcholine liposomes loaded with a lipophilic NF-κB inhibitor (curcumin, quercetin, or Bay11-7082) and antigen (OVA or methylated BSA) suppressed preexisting immune responses in an antigen-specific manner. By targeting antigen presenting cells (APCs) via injection of loaded liposomes into mice primed with antigen or into mice suffering from antigen-induced inflammatory arthritis, antigen-specific FoxP3 (+) regulatory T cells were induced, which suppressed effector T cell responses and the clinical signs of full-blown antigen-induced arthritis (Capini et al., 2009).

Incorporation of vitamin D3 and derivatives in the lipid bilayer of liposomes. In one preferred embodiment of the present invention, liposomal formulations of vitamin D3 and derivatives thereof are prepared by the addition of vitamin D3 or derivatives thereof to various lipid mixtures including those with or without cholesterol. Preferred lipid mixtures contain phosphatidyl-L-serine. Vitamin D3 and derivatives are incorporated into the lipid bilayer via intercalation between the hydrocarbon chains of phospholipid molecules (Merz and Sternberg, 1994).

Suitable vitamin D3 molecules and derivatives for liposomal formulations include but are not limited to a) active vitamin D3 (calcitriol; 1,25-(OH)₂D3), b) its inactive form (calcidiol; 25(OH)D3), c) vitamin D3 analogues such as 19-nor-vitamin D analogues, 24-hydroxy vitamin D derivatives, and 1α-hydroxyvitamin D3, and d) non-secosteroidal vitamin D receptor (VDR) modulators. In a preferred embodiment, liposomal formulations are prepared with the vitamin D3 derivative calcipotriol.

Liposomal encapsulation of glucocorticoids. In another preferred embodiment of the present invention, liposomal formulations of water-soluble glucocorticoids are prepared by the addition of aqueous solutions of glucocorticoids to various lipid mixtures including those with or without cholesterol. Preferred lipid mixtures contain phosphatidyl-L-serine.

Several protocols for the encapsulation of water-soluble glucocorticoids in liposomes have been described in the literature (e.g., Metselaar et al., 2003; Anderson et al., 2010; Hegeman et al., 2011; Schweingruber et al., 2011).

Preferred water-soluble glucocorticoids include but are not limited to prednisolone phosphate, methylprednisolone phosphate, and dexamethasone phosphate.

Liposomal formulations of glucocorticoids and vitamin D3 or derivatives thereof. In another preferred embodiment of the present invention, liposomal formulations containing water-soluble glucocorticoids and vitamin D3 or derivatives thereof are prepared by the addition of vitamin D3 or a derivative thereof to various lipid mixtures including those with or without cholesterol, followed by encapsulation of a water-soluble glucocorticoid. Preferred lipid mixtures contain phosphatidyl-L-serine.

Glucocorticoids and vitamin D3 modulate DCs via distinct and additive signaling pathways (Xing et al., 2002). For example, combined treatment of DCs with dexamethasone and vitamin D3 resulted in significant additive inhibition of pro-inflammatory cytokines, T-cell stimulation, chemokines, chemokine receptors, and NF-κB components (Xing et al., 2002).

Both vitamin D3-treated DCs and dexamethasone-treated DCs have the capacity to convert CD4(+) T cells into IL-10-secreting Treg potently suppressing the proliferation of responder T cells (Unger et al., 2009; Xing et al., 2002). However, only Treg induced by vitamin D3-treated DCs have been shown to express upon activation significant high levels of PD-L1 (programmed death-1 ligand) (Unger et al., 2009).

Liposomal formulations of JAK inhibitors. Suitable JAK inhibitors include but are not limited to tofacitinib.

In a preferred embodiment of the present invention, liposomal formulations containing water-soluble tofacitinib citrate (solubility in water: 2.9 mg/mL; 5.7 mM; MW 504.5) are prepared by the addition of aqueous solutions of tofacitinib citrate to various lipid mixtures including those with or without cholesterol. Preferred lipid mixtures contain phosphatidyl-L-serine.

In another preferred embodiment of the present invention, liposomal formulations containing the free base of tofacitinib are prepared by the addition of the free base of tofacitinib in ethanol to various lipid mixtures including those with or without cholesterol. Preferred lipid mixtures contain phosphatidyl-L-serine. The free base of tofacitinib (MW 312,4) is very poorly soluble in water (maximum solubility in plain water is estimated to be about 10-20 *µ*M; 3.12-6.24 *µ*g/ml), but very soluble in ethanol (100 mg/ml) or DMSO (100 mg/ml).

Hydrogel-, alum- and micro crystalline tyrosine-based compositions containing PS-liposome-tofacitinib formulations, offer the possibility to further reduce the therapeutically effective dose of tofacitinib in said compositions due to specific targeting of antigen-presenting cells such as DCs and macrophages.

### 9. Matrix-based compositions for immunotherapy

In one embodiment of the present invention, compositions for subcutaneous immunotherapy of allergic and autoimmune diseases are disclosed comprising a matrix which contains allergens or autoantigens (or fragments thereof), one or two tolerance-promoting adjuvants selected from PLGA particles or PS-liposomes, both optionally loaded with one or more low molecular weight find-me molecules, and optionally one or more additional tolerance-promoting immune modulator as liposomal or non-liposomal formulation.

Compositions based on alum or micro crystalline tyrosine. In one embodiment of the present invention, compositions for subcutaneous immunotherapy of allergic and autoimmune diseases are disclosed comprising a matrix based on alum or micro crystalline tyrosine, which contains allergens or autoantigens (or fragments thereof), one or two tolerance-promoting adjuvants selected from PLGA particles or PS-liposomes, both optionally loaded with one or more low molecular weight find-me molecules, and optionally one or more additional tolerance-promoting immune modulator as liposomal or non-liposomal formulation.

Aluminum-containing adjuvants, typically aluminum phosphate or aluminum hydroxide gels (generally referred to as alum), are still used for allergen- or autoantigen-specific immunotherapy, although alum adjuvants are known to trigger Th2-type immune responses and, therefore, are not optimal for the induction of tolerance.

Micro crystalline tyrosine (MCT) is a patent-protected depot providing adjuvant formulation of the biodegradable amino acid tyrosine that enhances Th1-like and associated immune responses (Bullimore et al., 2015), and is also not optimal for the induction of tolerance.

However, PLGA particles have been shown to mediate a shift from Th2 to Th1 immune responses and then to induce an IL-10-mediated suppression (Jilek et al., 2004). Therefore, PLGA particles can neutralize alum-mediated Th2-type or MCT-mediated Th1-like immune responses and the addition of PLGA particles to alum- or MCT-based compositions will mediate tolerance-promoting effects.

This assumption is supported by a study with Bet v 1-sensitized mice in which the therapeutic efficacy of Bet v 1-loaded anionic PLGA nanoparticles was compared to that of a composition comprising both alum and Bet v 1-loaded anionic PLGA nanoparticles (Schöll et al., 2004). Both treatments resulted in the development high levels of Bet v 1-specific IgG2a antibodies (reflecting Th1 bias) and a significant decrease of Bet v 1-specific IgG1 levels (Th2 marker). Furthermore, addition of alum to the PLGA preparation resulted in an immediate and stronger immune response already after the first injection. Possibly, released allergen from the PLGA particles was adsorbed to alum leading to a better presentation to the immune system. In addition, polyvalent Al³⁺ cations may have neutralized the negatively charged released protein molecules, thereby making them more hydrophobic which improves the hydrophobicity of particulate antigen delivery systems (Raghuvanshi et al., 2002). Most important, analysis of proliferative T cell responses upon stimulation with Bet v 1 revealed that T cells after immunotherapy with a composition comprising both alum and Bet v 1-loaded anionic PLGA nanoparticles were significantly less responsive to stimulation with Bet v 1 (stimulation index: 16.5) than those after immunotherapy with a composition comprising alum and Bet v 1 (stimulation index: 59.4) (Schöll et al., 2004). These data indicate that the addition of allergen-loaded anionic PLGA nanoparticles to alum-based compositions mediates neutralization of alum-mediated Th2-type immune responses and, thereby, induces tolerogenic effects which are superior to those of alum-based compositions without PLGA nanoparticles.

PS-liposomes are likely to exert similar effects in alum- or MCT-based compositions. For example, after uptake of PS-liposomes in vitro and in vivo macrophages have been shown to secrete high levels of the anti-inflammatory cytokines TGF-β and IL-10, concomitant with downregulation of pro-inflammatory markers such as TNF-α and the surface marker CD86 which provides costimulatory signals necessary for T cell activation and survival (Harel-Adar et al., 2011).

Hydrogel-based compositions. In one preferred embodiment, the present invention provides compositions and methods for conventional allergen-specific SCIT and immunotherapy of autoimmune diseases which are based on hydrogels containing embedded allergens or autoantigens (or fragments thereof), one or two tolerance-promoting adjuvants selected from PLGA particles or PS-liposomes, both optionally loaded with one or more low molecular weight find-me molecules, and optionally one or more additional tolerance-promoting immune modulator as liposomal or non-liposomal formulation.

For immunotherapy of allergic diseases and autoimmune diseases, the hydrogel-based compositions of the present invention provide a significant increase in therapeutic efficacy in comparison to conventional immunotherapies including conventional allergen-specific SCIT using alum or micro crystalline tyrosine as depot providing adjuvant. This results from the lack of tolerance-interfering Th2-inducing effects of alum or the tolerance-interfering Th1-inducing effects of micro crystalline tyrosine (MCT) and the tolerance-inducing effects of PLGA particles and/or PS-liposomes. Furthermore, adsorption of at least some of the allergenic or auto-immunogenic proteins or fragments thereof onto the surface of PLGA particles or PS-liposomes facilitates direct targeting of the hydrogel-embedded allergens or autoantigens or fragments thereof to local antigen-presenting cells (APCs), and presentation of non-adsorbed allergens or autoantigens to APCs occurs in an increasingly tolerogenic micro-environment.

Application of PLGA particles and/or PS-liposomes loaded with one or more low molecular weight find-me molecules provides an additional increase in therapeutic efficacy in that APCs are attracted to subcutaneously injected hydrogel compositions. A further substantial increase in therapeutic efficacy is possible by the addition of one or more low or medium molecular weight tolerance-promoting active immune modulators capable of inducing tolerogenic APCs including DCs and macrophages, and tolerance-promoting Tregs at the site of autoantigen or allergen presentation.

As important is the potential improvement of safety. Due to the improved therapeutic efficacy, the tolerogenic allergen or autoantigen dose will be lower than that required for conventional immunotherapies, which reduces potential adverse side effects.

An additional benefit of the novel compositions is the potential improvement of patient compliance. Depending on the achieved increase in therapeutic efficacy, the number of subcutaneous injections for the induction of tolerance may be reduced accordingly. Especially hydrogel compositions containing tolerance-promoting immune modulators are likely to induce an acceptable degree of tolerance after a few subcutaneous injections which provides a major relief for patients.

### 10. Disease-specific compositions for the treatment of allergic and autoimmune diseases

In one embodiment, the present invention discloses fields of applications for which disease-specific compositions according to the method of the present invention are beneficial. Fields of applications include but are not limited to allergic and autoimmune diseases.

Allergic diseases include but are not limited to allergic conjunctivitis, allergic rhinitis, and allergic asthma. Autoimmune diseases include but are not limited to type I diabetes, rheumatoid arthritis, multiple sclerosis, and autoimmune uveitis. For such diseases, the present invention discloses methods for restoring lasting immunological tolerance by allergen- or autoantigen-specific immunotherapy in combination with tolerogenic adjuvants selected from those listed in sections 1 and 2, optionally in combination with find-me molecules selected from those listed in section 3, and optionally in combination with additional tolerance-promoting immune modulators selected from those listed in sections 6 and 7.

### 10.1. Treatment of allergy

Current subcutaneous immunotherapy (SCIT) of allergy involves regular subcutaneous injections of allergens using incremental regimes, with the induction of tolerance taking from several days to several months depending on the regime used. Once tolerance is induced it can last for several years without further treatment. The limiting factor in SCIT is anaphylactic side effects, which vary in incidence from 0.1-5% of individuals depending on severity. Therefore, improved efficacy with decreased side effects has been the aim of recent approaches to SCIT including chemically modified allergens (allergoids), hypoallergenic recombinant allergens, and T-cell-reactive peptides. However, further improvements are required to revitalize the acceptance of allergen-specific immunotherapy by patients and physicians.

Based on current knowledge, allergen tolerance by subcutaneous immunotherapy is mediated by peripherally induced regulatory T cells as evidenced by a deficit of allergen-specific IL-10 producing T cells in the peripheral blood of allergic patients (for reviews, see Schmidt-Weber et al., 2005; Schmidt-Weber et al., 2006). Therefore, the main principle of specific immunotherapy in atopic patients is to generate allergen-specific tolerogenic antigen-presenting cells and, thereby, to increase the number of Tregs capable of controlling allergen-specific effector T cells.

The method of the present invention provides several concepts for significant improvement of the efficacy of subcutaneous allergen-specific immunotherapy of allergy. All concepts aim for immune intervention by targeting the molecular mechanisms of allergen tolerance and reciprocal regulation of effector T cells via regulatory T cells induced by allergen-specific tolerogenic antigen-presenting cells.

Concept A: Improvement of clinically approved therapeutic approaches. Currently, clinically approved subcutaneous immunotherapeutic approaches utilize alum or micro crystalline tyrosine (MCT) as depot-providing adjuvant and allergen extracts, allergoids or depigmented allergoids as allergen source.

In one embodiment of the present invention, one or two tolerance-promoting adjuvants selected from PLGA particles or PS-liposomes, both optionally loaded with one or more low molecular weight find-me molecules, are added to such compositions. Both tolerance-promoting adjuvants will enhance the induction of tolerance and, thereby, increase the therapeutic efficacy of these approaches. For example, PLGA particles can neutralize alum-mediated Th2-type or MCT-mediated Th1-like immune responses, since PLGA particles have been shown to mediate a Th2 to Th1 immune deviation and then to induce an IL-10-mediated suppression (Jilek et al., 2004). As demonstrated in the animal study of Schöll et al. (2004), tolerogenic effects induced with alum-based compositions containing allergen-loaded PLGA nanoparticles are superior to those of alum-based allergen-containing compositions without PLGA nanoparticles. PS-liposomes are likely to exert similar effects in alum- or MCT-based compositions, since after uptake of PS-liposomes rat macrophages have been shown to secrete high levels of the anti-inflammatory cytokines TNF-β and IL-10, concomitant with downregulation of pro-inflammatory markers such as TNF-α (Harel-Adar et al., 2011).

In another embodiment of the present invention, one or more tolerance-promoting adjuvants selected from PLGA particles or PS-liposomes, both optionally loaded with one or more low molecular weight find-me molecules, and one or more additional tolerance-promoting immune modulators selected from those listed in sections 6 und 7, are added to such compositions. In a preferred specific embodiment, calcipotriol is added as additional tolerance-promoting immune modulator to such compositions. In another preferred specific embodiment, a glucocorticoid (e.g., dexamethasone phosphate) is added as additional tolerance-promoting immune modulator to such compositions. In still another preferred specific embodiment, dexamethasone phosphate and calcipotriol are added as additional tolerance-promoting immune modulators to such compositions.

Concept B: Hydrogel-based approaches using allergen extracts. For immunotherapy of allergy, hydrogel-based compositions of the present invention provide a significant increase in therapeutic efficacy in comparison to conventional allergen-specific SCIT due to the lack of tolerance-interfering Th2-inducing effects of alum or the Th1-mediating effects of micro crystalline tyrosine (MCT).

Preferred for the method of the present invention are injectable in situ-forming gel systems which are biodegradable. Most preferred are biodegradable PLGA-PEG-PLGA hydrogels. Compared to other biodegradable hydrogels, injectable thermo-gelling PLGA-PEG-PLGA polymers possess several advantages including easy preparation, a formulation process which is free of harmful organic solvents, application of building blocks which are approved for parenteral use in humans by the FDA, excellent biocompatibility, and well established procedures for the production of composits comprising liposomes and PLGA particles.

In one embodiment, the present invention provides compositions and methods for allergen-specific SCIT which are based on PLGA-PEG-PLGA hydrogels containing a clinically approved allergen preparation selected from allergen extracts, allergoids or depigmented allergoids, and one or two tolerance-promoting adjuvants selected from PLGA particles or PS-liposomes, both optionally loaded with one or more low molecular weight find-me molecules.

In another embodiment, the present invention provides compositions and methods for allergen-specific SCIT which are based on PLGA-PEG-PLGA hydrogels containing a clinically approved allergen preparation selected from allergen extracts, allergoids or depigmented allergoids, one or two tolerance-promoting adjuvants selected from PLGA particles or PS-liposomes, both optionally loaded with one or more low molecular weight find-me molecules, and one or more additional tolerance-promoting immune modulators selected from those listed in sections 6 and 7. In a preferred specific embodiment, calcipotriol is added as additional tolerance-promoting immune modulator to such compositions. In another preferred specific embodiment, a glucocorticoid (e.g., dexamethasone phosphate) is added as additional tolerance-promoting immune modulator to such compositions. In still another preferred specific embodiment, dexamethasone phosphate and calcipotriol are added as additional tolerance-promoting immune modulators to such compositions.

Concept C: Hydrogel-based approaches using recombinant proteins. As compared to allergen extracts and allergoids, compositions of recombinant allergens provide the important advantage of reproducibility. In addition, recombinant allergens allow site-directed modifications of their surface structure in order to decrease the allergenicity while retaining its immunogenicity.

In one embodiment, the present invention provides compositions and methods for allergen-specific SCIT which are based on PLGA-PEG-PLGA hydrogels containing one or more recombinant allergens or derivatives thereof, and one or two tolerance-promoting adjuvants selected from PLGA particles or PS-liposomes, both optionally loaded with one or more low molecular weight find-me molecules.

In another embodiment, the present invention provides compositions and methods for allergen-specific SCIT which are based on PLGA-PEG-PLGA hydrogels containing one or more recombinant allergens or derivatives thereof, one or two tolerance-promoting adjuvants selected from PLGA particles or PS-liposomes, both optionally loaded with one or more low molecular weight find-me molecules, and one or more additional tolerance-promoting immune modulators selected from those listed in sections 6 and 7. In a preferred specific embodiment, a recombinant antagonistic IL-4/IL-13 mutein is added as additional tolerance-promoting immune modulator to such compositions. In another preferred specific embodiment, calcipotriol is added as additional tolerance-promoting immune modulator to such compositions. In another preferred specific embodiment, a recombinant antagonistic IL-4/IL-13 mutein and calcipotriol are added as additional tolerance-promoting immune modulators to such compositions. In another preferred specific embodiment, a glucocorticoid (e.g., dexamethasone phosphate) is added as additional tolerance-promoting immune modulator to such compositions. In another preferred specific embodiment, dexamethasone phosphate and calcipotriol are added as additional tolerance-promoting immune modulators to such compositions. In still another preferred specific embodiment, a recombinant antagonistic IL-4/IL-13 mutein and a glucocorticoid (e.g., dexamethasone phosphate) are added as additional tolerance-promoting immune modulators to such compositions.

Concept D: Hydrogel-based approaches using T cell peptides. Short allergen-derived T cell epitope peptides eliminate the risk of potential disease flares associated with current immunotherapies using allergen extracts or allergoids since they are unable to cross-link IgE and possess minimal inflammatory potential. For honeybee venom allergy (Müller et al., 1998) and cat allergy (Worm et al., 2011) subcutaneously or intradermally administered short allergen-derived T cell epitope peptides have been demonstrated to induce clinical and immunological tolerance with few adverse events after only a short course of treatment.

In one embodiment, the present invention provides compositions and methods for allergen-specific SCIT which are based on PLGA-PEG-PLGA hydrogels containing one or more allergen-derived T cell epitope peptides, and one or two tolerance-promoting adjuvants selected from PLGA particles or PS-liposomes, both optionally loaded with one or more low molecular weight find-me molecules.

In another embodiment, the present invention provides compositions and methods for allergen-specific SCIT which are based on PLGA-PEG-PLGA hydrogels containing one or more allergen-derived T cell epitope peptides, one or two tolerance-promoting adjuvants selected from PLGA particles or PS-liposomes, both optionally loaded with one or more low molecular weight find-me molecules, and one or more additional tolerance-promoting immune modulators selected from those listed in sections 6 and 7. In a preferred specific embodiment, calcipotriol is added as additional tolerance-promoting immune modulator to such compositions. In another preferred specific embodiment, a glucocorticoid (e.g., dexamethasone phosphate) is added as additional tolerance-promoting immune modulator to such compositions. In still another preferred specific embodiment, dexamethasone phosphate and calcipotriol are added as additional tolerance-promoting immune modulators to such compositions.

In another embodiment, the present invention provides compositions and methods for allergen-specific SCIT which are based on PLGA-PEG-PLGA hydrogels containing tolerizing PS-liposomes loaded with one or more allergen-derived T cell epitope peptides and one or more DC maturation inhibitors as described in detail in patent application EP 15001524.6 (antigen-specific immunotherapy using tolerizing liposomes), and PLGA particles as tolerance-promoting adjuvant, optionally loaded with one or more low molecular weight find-me molecules.

In still another embodiment, the present invention provides compositions and methods for allergen-specific SCIT which are based on PLGA-PEG-PLGA hydrogels containing PS-liposomes loaded with one or more allergen-derived T cell epitope peptides as described in detail in patent application EP 15001524.6 (antigen-specific immunotherapy using tolerizing liposomes), PLGA particles as tolerance-promoting adjuvant, optionally loaded with one or more low molecular weight find-me molecules, and one or more additional tolerance-promoting immune modulators selected from those listed in sections 6 and 7 and in patent application EP 15001524.6. In a preferred specific embodiment, calcipotriol is added as additional tolerance-promoting immune modulator to such compositions. In another preferred specific embodiment, a glucocorticoid (e.g., dexamethasone phosphate) is added as additional tolerance-promoting immune modulator to such compositions. In still another preferred specific embodiment, dexamethasone phosphate and calcipotriol are added as additional tolerance-promoting immune modulators to such compositions.

### 10.2. Treatment of allergic asthma

Asthma is one of the most common chronic diseases worldwide. It is estimated that 150 million people around the world suffer from asthma. Mortality has reached over 180,000 annually.

According to a review of 75 trials covering a total of 3,188 patients with asthma, SCIT (subcutaneous immunotherapy) led to a significant reduction in asthma symptom scores, medication use and airway hyper-responsiveness, with evidence of a dose-related effect (Abramson et al., 2003). SCIT and SLIT (sublingual immunotherapy) also decrease the development of sensitization to new allergens and decrease the risk of new asthma in both adults and children with rhinitis. A recent retrospective cohort study of 322 subjects with allergic rhinitis showed that 53.1% of subjects who were not treated with SCIT developed asthma, whereas only 41.6% of subjects who received SCIT were diagnosed with asthma (for a review, see Holgate and Polosa, 2008). Taken together, immunotherapy has been proven efficacious in treating mild asthma, as well as in preventing the progression to asthma in patients suffering from rhino-conjunctivitis (Moller et al., 2002; Jacobsen et al., 2007), but it is not yet recommended for the treatment of moderate to severe asthmatic patients (Rolland et al., 2009). Therefore, significantly improved approaches are needed.

For immunotherapy of allergic asthma according to the method of the present invention, hydrogel-based compositions are applied since hydrogels provide a significant increase in therapeutic efficacy in comparison to conventional allergen-specific SCIT due to the lack of tolerance-interfering Th2-inducing effects of alum or the Th1-mediating effects of micro crystalline tyrosine (MCT).

Preferred for the method of the present invention are injectable in situ-forming gel systems which are biodegradable. Most preferred are biodegradable PLGA-PEG-PLGA hydrogels. Compared to other biodegradable hydrogels, injectable thermo-gelling PLGA-PEG-PLGA polymers possess several advantages including easy preparation, a formulation process which is free of harmful organic solvents, application of building blocks which are approved for parenteral use in humans by the FDA, excellent biocompatibility, and well established procedures for the production of composits comprising liposomes and PLGA particles.

In one embodiment, the present invention discloses methods for restoring lasting immunological tolerance by allergen-specific immunotherapy using hydrogel-embedded tolerizing PS-liposomes containing one or more DC maturation inhibitors and one or more allergens or peptides derived thereof as described in detail in patent application EP 15001524.6 (antigen-specific immunotherapy using tolerizing liposomes), and PLGA particles as tolerance-promoting adjuvant, optionally loaded with one or more low molecular weight find-me molecules.

In another embodiment, the present invention discloses methods for restoring lasting immunological tolerance by allergen-specific immunotherapy using hydrogel-embedded tolerizing PS-liposomes containing one or more allergens or peptides derived thereof as described in detail in patent application EP 15001524.6 (antigen-specific immunotherapy using tolerizing liposomes), PLGA particles as tolerance-promoting adjuvant, optionally loaded with one or more low molecular weight find-me molecules, and one or more hydrogel-embedded additional tolerance-promoting immune modulators selected from those listed in sections 6 an7, and in patent application EP 15001524.6.

Preferred additional tolerance-promoting immune modulators include vitamin D3 and selected vitamin D3 analogs such as calcipotriol, glucocorticoids such as dexamethasone, salicylate-based therapeutics for the inhibition of TNFR1-mediated pathways, aptamer-based therapeutics for the inhibition of interleukins or the corresponding receptors including but not limited to IL-17A, IL-17F, IL-17E (IL-25), IL-23, and IL-33, and peptide- or peptidomimetic-based inhibitors of complement.

In a preferred specific embodiment, calcipotriol is added as additional tolerance-promoting immune modulator to such compositions. In another preferred specific embodiment, a glucocorticoid (e.g., dexamethasone phosphate) is added as additional tolerance-promoting immune modulator to such compositions. In still another preferred specific embodiment, dexamethasone phosphate and calcipotriol are added as additional tolerance-promoting immune modulators to such compositions.

### 10.3. Treatment of rheumatoid arthritis (RA)

RA affects between 0.5 and 1% of adults in the developed world with between 5 and 50 per 100,000 people newly developing the condition each year (Scott et al., 2010). In 2010 it resulted in about 49,000 deaths globally (Lozano et al., 2012).

Autoantigen-specific immunotherapy with the 15-mer synthetic peptide dnaJp1 has been proven efficacious in treating RA, but the majority of autoantigen-specific immunotherapeutic approaches showed in placebo-controlled trials little or no evidence of clinical efficacy. Therefore, improved autoantigen-specific immunotherapeutic approaches are needed.

In one embodiment, the present invention discloses methods for restoring lasting immunological tolerance by autoantigen-specific immunotherapy using hydrogel-embedded tolerizing PS-liposomes containing one or more DC maturation inhibitors and one or more autoantigens or peptides derived thereof as described in detail in patent application EP 15001524.6 (antigen-specific immunotherapy using tolerizing liposomes), and PLGA particles as tolerance-promoting adjuvant, optionally loaded with one or more low molecular weight find-me molecules.

In another embodiment, the present invention discloses methods for restoring lasting immunological tolerance by allergen-specific immunotherapy using hydrogel-embedded tolerizing PS-liposomes containing one or more autoantigens or peptides derived thereof as described in detail in patent application EP 15001524.6 (antigen-specific immunotherapy using tolerizing liposomes), PLGA particles as tolerance-promoting adjuvant, optionally loaded with one or more low molecular weight find-me molecules, and one or more hydrogel-embedded additional tolerance-promoting immune modulators selected from those listed in sections 6 and 7, and in patent application EP 15001524.6.

In another embodiment, the present invention discloses additional tolerance-promoting immune modulators suitable for the method of the present invention. Suitable additional tolerance-promoting immune modulators include JAK inhibitors such as tofacitinib, vitamin D3 and selected vitamin D3 analogs such as calcipotriol, glucocorticoids such as dexamethasone, salicylate-based therapeutics for the inhibition of TNFR1-mediated pathways, aptamer-based therapeutics for the inhibition of interleukins or the corresponding receptors including but not limited to IL-17A, IL-23, and IL-33, and peptide- or peptidomimetic-based inhibitors of complement. In a preferred specific embodiment, tofacitinib is added as additional tolerance-promoting immune modulator to such compositions. In another preferred specific embodiment, calcipotriol is added as additional tolerance-promoting immune modulator to such compositions. In another preferred specific embodiment, a glucocorticoid (e.g., dexamethasone phosphate) is added as additional tolerance-promoting immune modulator to such compositions. In still another preferred specific embodiment, dexamethasone phosphate and calcipotriol are added as additional tolerance-promoting immune modulators to such compositions.

In another embodiment, the present invention discloses autoantigens or fragments thereof which are suitable for the treatment of RA according to the method of the present invention. Suitable autoantigens include type II bovine or chicken collagen, HCgp39, lyophilised Escherichia coli extract, and citrullinated (cit) proteins including but not limited to cit-vimentin, cit-fibrinogen, cit-fibrinogen, and cit-collagen type II. Suitable are also fragments derived from these autoantigens, as well as the 15-mer synthetic peptide dnaJp1. In a preferred specific embodiment, the method of the present invention utilizes tolerizing PS-liposomes loaded with one or more fragments of autoantigens selected from the 15-mer synthetic peptide dnaJp1, and peptides derived from citrullinated proteins including but not limited to cit-vimentin, cit-fibrinogen, cit-fibrinogen, and cit-collagen type II.

### 10.4. Treatment of type 1 diabetes (T1D)

Type 1 diabetes causes an estimated 5-10% of all diabetes cases or 11-22 million worldwide.

Autoantigen-specific immunotherapy with immunomodulatory peptide DiaPep277 (derived from hsp60 protein) resulted in preservation of stimulated C-peptide for 10 months posttreatment. Furthermore, therapy with GAD-alum (glutamic acid decarboxylase formulated in alum) in subjects with T1D within 6 months from diagnosis also showed preservation of stimulated C-peptide levels. However, phase 2 and 3 trials of GAD-alum therapy failed to confirm the preliminary observation. Therefore, improved autoantigen-specific immunotherapeutic approaches are needed.

In one embodiment, the present invention discloses methods for restoring lasting immunological tolerance by autoantigen-specific immunotherapy using hydrogel-embedded tolerizing PS-liposomes containing one or more DC maturation inhibitors and one or more autoantigens or peptides derived thereof as described in detail in patent application EP 15001524.6 (antigen-specific immunotherapy using tolerizing liposomes), and PLGA particles as tolerance-promoting adjuvant, optionally loaded with one or more low molecular weight find-me molecules.

In another embodiment, the present invention discloses methods for restoring lasting immunological tolerance by allergen-specific immunotherapy using hydrogel-embedded tolerizing PS-liposomes containing one or more autoantigens or peptides derived thereof as described in detail in patent application EP 15001524.6 (antigen-specific immunotherapy using tolerizing liposomes), PLGA particles as tolerance-promoting adjuvant, optionally loaded with one or more low molecular weight find-me molecules, and one or more hydrogel-embedded additional tolerance-promoting immune modulators selected from those listed in sections 6 and 7, and in patent application EP 15001524.6.

In another embodiment, the present invention discloses additional tolerance-promoting immune modulators suitable for the method of the present invention. Suitable additional tolerance-promoting immune modulators include vitamin D3 and selected vitamin D3 analogs such as calcipotriol, glucocorticoids such as dexamethasone, peptide- or peptidomimetic-based inhibitors of complement, and salicylate-based therapeutics for the inhibition of TNFR1-mediated pathways. In a preferred specific embodiment, calcipotriol is added as additional tolerance-promoting immune modulator to such compositions. In another preferred specific embodiment, a glucocorticoid (e.g., dexamethasone phosphate) is added as additional tolerance-promoting immune modulator to such compositions. In still another preferred specific embodiment, dexamethasone phosphate and calcipotriol are added as additional tolerance-promoting immune modulators to such compositions.

In another embodiment, the present invention discloses autoantigens or fragments thereof which are suitable for the treatment of T1D according to the method of the present invention. Suitable autoantigens include insulin, proinsulin, GAD65 (glutamic acid decarboxylase), IA-2 (islet antigen 2; tyrosine phosphatase), and the ZnT8 transporter (zink transporter 8, localized on the membrane of insulin secretory granules). Suitable are also fragments derived from these autoantigens, as well as the immunomodulatory peptide DiaPep277 (derived from hsp60 protein), and other HSP60-derived peptides. In a preferred embodiment, the method of the present invention utilizes tolerizing PS-liposomes loaded with one or more autoantigens selected from proinsulin, GAD65 and HSP60-derived peptides.

### 10.5. Treatment of multiple sclerosis (MS)

As of 2010, the number of people with MS was 2-2.5 million (approximately 30 per 100,000) globally, with rates varying widely in different regions.

Autoantigen-specific immunotherapy has been proven efficacious in treating patients with relapsing-remitting MS. In one study, however, transdermal application of myelin peptides via skin patches had to be continued for a period of one year to achieve improvement (Walczak et al., 2013). In another study, myelin peptides were coupled to the surface of autologous, apoptotic peripheral blood monocytes and then reinjected into MS patients (Lutterotti et al., 2013). Although both studies showed promising results, there is a need for improved autoantigen-specific immunotherapeutic approaches.

In one embodiment, the present invention discloses methods for restoring lasting immunological tolerance by autoantigen-specific immunotherapy using hydrogel-embedded tolerizing PS-liposomes containing one or more DC maturation inhibitors and one or more autoantigens or peptides derived thereof as described in detail in patent application EP 15001524.6 (antigen-specific immunotherapy using tolerizing liposomes), and PLGA particles as tolerance-promoting adjuvant, optionally loaded with one or more low molecular weight find-me molecules.

In another embodiment, the present invention discloses methods for restoring lasting immunological tolerance by allergen-specific immunotherapy using hydrogel-embedded tolerizing PS-liposomes containing one or more autoantigens or peptides derived thereof as described in detail in patent application EP 15001524.6 (antigen-specific immunotherapy using tolerizing liposomes), PLGA particles as tolerance-promoting adjuvant, optionally loaded with one or more low molecular weight find-me molecules, and one or more hydrogel-embedded additional tolerance-promoting immune modulators selected from those listed in sections 6 and 7, and in patent application EP 15001524.6.

In another embodiment, the present invention discloses additional tolerance-promoting immune modulators suitable for the method of the present invention. Suitable additional tolerance-promoting immune modulators include vitamin D3 and selected vitamin D3 analogs such as calcipotriol, glucocorticoids such as dexamethasone, and aptamer-based therapeutics for the inhibition of interleukins or the corresponding receptors. In a preferred specific embodiment, calcipotriol is added as additional tolerance-promoting immune modulator to such compositions. In another preferred specific embodiment, a glucocorticoid (e.g., dexamethasone phosphate) is added as additional tolerance-promoting immune modulator to such compositions. In still another preferred specific embodiment, dexamethasone phosphate and calcipotriol are added as additional tolerance-promoting immune modulators to such compositions.

In another embodiment, the present invention discloses autoantigens or fragments thereof which are suitable for the treatment of MS according to the method of the present invention. Suitable autoantigens include myelin basic protein (MBP), myelin oligodendrocyte protein (MOG), and proteolipid protein (PLP), as well as fragments derived from these autoantigens. In a preferred embodiment, the method of the present invention utilizes tolerizing PS-liposomes loaded with the 7 myelin peptides including MBP₁₃₋₃₂, MBP₈₃₋₉₉, MBP₁₁₁₋₁₂₉, MBP₁₄₆₋₁₇₀, MOG₁₋₂₀, MOG₃₅₋₅₅, and PLP₁₃₉₋₁₅₄.

### 10.6. Treatment of autoimmune uveits (AU)

AU is a sight-threatening inflammation inside the eye and is the fourth most common cause of blindness among the working-age population in the developed world. The prevalence of AU is estimated at 38 cases per 100,000 people.

Clinical trials have been performed with peptide B27PD. Although in these clinical studies a decrease of the average corticosteroid dosage was observed, there is a need for improved autoantigen-specific immunotherapeutic approaches.

In one embodiment, the present invention discloses methods for restoring lasting immunological tolerance by autoantigen-specific immunotherapy using hydrogel-embedded tolerizing PS-liposomes containing one or more DC maturation inhibitors and one or more autoantigens or peptides derived thereof as described in detail in patent application EP 15001524.6 (antigen-specific immunotherapy using tolerizing liposomes), and PLGA particles as tolerance-promoting adjuvant, optionally loaded with one or more low molecular weight find-me molecules.

In another embodiment, the present invention discloses methods for restoring lasting immunological tolerance by allergen-specific immunotherapy using hydrogel-embedded tolerizing PS-liposomes containing one or more autoantigens or peptides derived thereof as described in detail in patent application EP 15001524.6 (antigen-specific immunotherapy using tolerizing liposomes), PLGA particles as tolerance-promoting adjuvant, optionally loaded with one or more low molecular weight find-me molecules, and one or more hydrogel-embedded additional tolerance-promoting immune modulators selected from those listed in sections 6 and 7, and in patent application EP 15001524.6.

In another embodiment, the present invention discloses additional tolerance-promoting immune modulators suitable for the method of the present invention. Suitable additional tolerance-promoting immune modulators include vitamin D3 and selected vitamin D3 analogs such as calcipotriol, glucocorticoids such as dexamethasone, and aptamer-based therapeutics for the inhibition of interleukins or the corresponding receptors. In a preferred specific embodiment, calcipotriol is added as additional tolerance-promoting immune modulator to such compositions. In another preferred specific embodiment, a glucocorticoid (e.g., dexamethasone phosphate) is added as additional tolerance-promoting immune modulator to such compositions. In still another preferred specific embodiment, dexamethasone phosphate and calcipotriol are added as additional tolerance-promoting immune modulators to such compositions.

In another embodiment, the present invention discloses autoantigens or fragments thereof which are suitable for the treatment of AU according to the method of the present invention. Suitable autoantigens include S-antigen, interphotoreceptor retinoid binding protein, cellular retinaldehyde binding protein, arrestin, and peptides derived of these autoantigens such as arrestin peptide 291-310.

### 11. Pharmaceutical formulations

In one embodiment, the therapeutic compositions of the present invention are incorporated into pharmaceutical compositions suitable for administration. Such compositions typically comprise the therapeutic compositions of the present invention and a pharmaceutically acceptable carrier.

As used herein, a pharmaceutically acceptable carrier is intended to include any and all solvents, hydrogels, dispersion media, coatings, antibacterial and antifungal agents, isotonic systems, and the like, compatible with the components of the therapeutic compositions of the present invention and pharmaceutical administration. The use of such media and agents for pharmaceutically active substances is well known in the art. Supplementary active compounds can also be incorporated into the composition.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents, antioxidants such as ascorbic acid or sodium bisulfite, chelating agents such as ethylenediaminetetraacetic acid, buffers such as acetates, citrates or phosphates and agents for the adjustment of toxicity such as sodium chloride or dextrose. The pH can be adjusted with acids or bases, such as hydrochloric acid or sodium hydroxide. In many cases, it will be preferable to include isotonic agents, for example, sugars, polyalcohols such as mannitol, sorbitol, sodium chloride in the composition.

The composition should be fluid to the extent that easy syringability exists. The proper fluidity can be maintained, for example, by the use of a coating such as lecithin, by the maintenance of the required particle size in the case dispersion and by use of surfactants. The composition must be stable under the conditions of manufacture and storage and must be preserved against the contaminating action of microorganisms such as bacteria and fungi. Prevention of the action of microorganisms can be achieved by various antibacterial and antifungal agents such as parabens, chlorobutanol, phenol, ascorbic acid, thimoseral, and the like. In all cases, the composition must be sterile. Sterile injectable solutions can be prepared by filtered sterilization. The preparation can be enclosed in ampoules, disposable syringes or multiple dose vials made of glass or plastic.

In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

### 12. Therapeutically effective doses

In one embodiment, the present invention discloses therapeutic methods including suitable therapeutically effective doses of PLGA spheres (selected from those listed in section 1), PS-liposomes (selected from those listed in section 2), find-me signals for attraction of peripheral antigen-presenting cells to the injection site of therapeutic compositions (selected from those listed in section 3), allergens or autoantigens or peptides derived thereof (selected from those listed in section 5), and additional tolerance-promoting immune modulators (selected from those listed in sections 6 and 7).

Determination of a therapeutically effective dose is well within the capability of those skilled in the art. The therapeutically effective dose can be estimated initially in animal models, usually mice, rats, rabbits, dogs, pigs, or non-human primates. The animal model also can be used to determine the appropriate concentration range and route of administration. Such information can then be used to determine useful doses and routes for administration in humans.

Dosage regimens may be adjusted to provide the optimum therapeutic response. The quantity of the matrix-embedded components depends on the release kinetics of the depot-providing matrices and is adjusted to a level that guarantees the continuous release of therapeutically effective doses over a period of at least 2 to 5 days. The quantity of embedded components will vary according to factors such as the weight and the age of the individual, and the ability of the composition to induce an effective immune response in the individual.

The following data of this section provide useful guidelines for the determination of a therapeutically effective dose for those skilled in the art.

### 12.1. Therapeutic effective doses of tolerizing PLGA particles

PLGA particles have been studied in a variety of animal models in the recent past. Results obtained from these studies provide useful information for the application of PLGA nanoparticles in humans.

Studies with plain PLGA particles. In the study of Jilek et al. (2004), 5 mg of plain biodegradable PLGA microspheres (size range: 1-10 *µ*m) in 100 *µ*l 0.25% methocel in nanopure water were administered subcutaneously to mice once a week for three consecutive weeks. Three weeks later, mice were sensitized subcutaneously with 6 doses of 100 ng bee venom phospholipase A2 combined with 1 mg alum given at 2-week intervals. Over a period of 8.5 months , the resulting immune responses were analyzed. The data demonstrate that plain PLGA microspheres can induce tolerance in mice for as long as 6 months post-sensitization, based on a dual mechanism that does initially rely on a TH2 to TH1 immune deviation and then on IL-10-mediated suppression.

Studies with allergen/antigen-loaded PLGA particles. Allergen-or antigen-loaded PLGA particles have been studied in several animal models. For example, in the study of Schöll et al. (2004) Bet v 1-sensitized mice were treated once by subcutaneous administration of PLGA nanoparticles (size range: 470-750 nm; 90% value) containing 20 *µ*g Bet v 1 (16.5 *µ*g Bet v 1/mg dry weight of polymer) in 170 *µ*l PBS/1% Pluronic (11.7 *µ*g Bet v 1/100 *µ*l). Pluronic F-68 was used as stabilizer of the PLGA nanoparticles. Treated mice developed high levels of Bet v 1-specific IgG2a antibodies (Th1 response), whereas IgG1 levels (Th2 marker) decreased significantly. Moreover, T cells from treated mice showed IFN-γ (Th1 response) and IL-10 production (tolerance-promoting interleukin).

Hydrogel-PLGA particle compositions. US 6,287,588 B1 provides an example of a composition comprising 10 mg of PLGA microspheres loaded with Zn-human growth hormone in 100 *µ*l of a 20% PLGA-PEG-PLGA hydrogel in 10 mM HEPES buffer, pH 7.0. This formulation can be injected smoothly using a 24-gauge needle due to excellent wetting and suspending ability of the PLGA-PEG-PLGA hydrogel.

### 12.2. Therapeutic effective doses of tolerizing PS-liposomes.

PS-liposomes have been studied in a variety of animal models in the recent past. Results obtained from these studies provide useful information for the application of PS-liposomes in humans.

Studies with empty PS-liposomes. For example, using BALB mice (body weight approx. 20 g), the effect of subcutaneously (s.c.) administered PS-liposomes on immune responses upon subsequent injection of ovalbumin (OVA) or keyhole limpet hemocyanine (KLH) in complete Freund's adjuvant (CFA) has been investigated (Hoffman et al., 2005). In this study, PS-containing liposomes comprising a 30:30:40 molar ratio of PS to PC to cholesterol, were first injected s.c. in one flank of 6-8 weeks old BALB mice (100 *µ*l, corresponding to 0.5 mg of total lipid), followed after one hour by another s.c. injection of 150 *µ*l of an emulsion containing 50 *µ*g of OVA or 150 *µ*g of KLH in CFA in the same region. As evident from the data, subcutaneously administered PS-liposomes specifically inhibited responses to antigens. Numbers of total leukocytes and antigen-specific CD4+ T cells were reduced as well as the level of antigen-specific IgG in blood. There was also a decrease of draining lymph node tissue mass and the size of germinal centers in spleen and lymph nodes (Hoffman et al., 2005).

Using a rat model of acute myocardial infarction (MI) in another study, the effects of intraveneously (i.v.) administered PS-liposomes on cardiac macrophages has been investigated (Harel-Adar et al., 2011). In this study, 150 *µ*l of a 0.06 M solution of PS-liposomes comprising a 30:30:40 (1:1:1.33) molar ratio of PS to PC to cholesterol (9 *µ*mol lipid or approx. 5.4 mg lipid; compare Example 1.1), were injected i.v. through the femoral vein of female Sprague-Dawley rats (body weight approx. 150 g) 48 hours after MI induction. As evident from the data, i.v. administered PS-containing liposomes promoted angiogenesis, preservation of small scars, and prevented ventricular dilatation and remodeling. Following uptake of PS-liposomes by macrophages, the cells secreted high levels of the anti-inflammatory cytokines TGF-β and IL-10 and upregulated the expression of the mannose receptor CD206, concomitant with downregulation of proinflammatory markers such as TNF-α and the surface marker CD86 (Harel-Adar et al., 2011).

Studies with allergen/antigen-loaded liposomes. Methods for encapsulation of proteins or fragments thereof in liposomes are known to the person skilled in the art. Various formulations of liposomes containing encapsulated allergens (including allergen extracts), autoantigens or fragments thereof have been prepared and studied in a variety of animal models (e.g., Ishii et al., 2010; Meechan et al., 2012; Belogurov et al., 2013) and in clinical trials (e.g., Basomba et al., 2002). Furthermore, clinical trials have demonstrated that subcutaneously administered liposomes containing encapsulated allergen extracts are safe and well tolerated (e.g., Galvain et al., 1999; Basomba et al., 2002).

### Studies with liposomes loaded DC maturation inhibitors.

Methods for encapsulation of DC maturation inhibitors such as dexamethasone phosphate (DexP), vitamin D3 (VD3) and the VD3 derivative calcipotriol (VD3c), curcumin, quercetin and Bay11-7082 in liposomes are known to the person skilled in the art. Liposomal dexamethasone phosphate (DexP).

In the study of Hegeman et al. (2011), liposomal DexP has been administered i.v. at a concentration of 11.2 *µ*g DexP/20-g mouse (adult male C57BL/6 mice with a body weight of 20-24g). The DexP to lipid ratio was 28 *µ*g DexP/*µ*mole lipid (comprising PC, cholesterol and PE at a molar ration of 55:40:5).

In another study (Anderson et al., 2010), liposomal DexP has been administered i.v. at a concentration of 1 mg DexP/kg body weight for 3 days, corresponding to three injections of 20 *µ*g DexP/20-g mouse. The DexP to lipid ratio was 40 *µ*g DexP/*µ*mole lipid (comprising DPPC, DPPG and cholesterol at a molar ration of 50:10:40).

A more than three-fold higher amount of liposomal DexP (3.75 mg liposomal DexP/kg body weight, corresponding to 75 *µ*g/20-g mouse or 563 *µ*g/150-g female rat) has been administerd i.v to rats 6, 24 and 48 hours after induction of antigen-induced arthritis (US20060147511A1).

Liposomal calcipotriol.

Using VD3 or VD3c for incorporation into liposomes made of DMPPC or egg-PC in a molar ratio of calcipotriol (MW 412.6) to lipd of 0.03 to 1, incorporation rates of more than 80% have been reported (Merz and Sternberg, 1994).

Liposomal curcumin, quercetin and Bay11-7082.

In the study of Capini et al. (2009), the lipohilic DC maturation inhibitors (NF-κB inhibitors curcumin, quercetin and Bayll-7082) have been encapsulated in phosphatidylcholine liposomes in the presence of methylated BSA (mBSA) used as model antigen. The final concentration of liposomal mBSA was 2.5 mg/ml, of liposomal Bay11-7082 0.5 mM (103.6 ug/ml; MW 207.3), of liposomal curcumin 2 mM (736.8 ug/ml; MW 368.4), and of liposomal quercetin also 2 mM (604.4 ug/ml; MW 302.2). In subsequent experiments, liposomal preparations containing mBSA and one of the NF-κB inhibitors were used for the treatment of established antigen-induced inflammatory arthritis (methylated BSA in CFA) in C57BL/6 mice. After a single s.c. injection of 0.1 ml of each liposomal preparation at the tail base, the mean clinical disease score was reduced within four days to approximately 50% as compared to control mice (Capini et al., 2009).

### 12.3. Therapeutic effective doses of vitamin D3.

Preferred vitamin D3 molecules include 25-OH-D3 (calcidiol), its biologically active metabolite 1,25-(OH)₂D3 (calcitriol), and vitamin D3 analog calcipotriol. These molecules have been studied in a variety of animal models and evaluated in clinical trials (for reviews, see Plum and DeLuca, 2010; Fletcher et al., 2012).

### Calcidiol

Under normal physiological conditions, calcidiol circulates at a concentration up to 200 nM (deficiency: <50 nM). According to a study of Jones (2008), plasma calcidiol concentrations above 0.75 *µ*M, (approx. 300 ng/ml) can produce vitamin D toxicity, although according to a previous study of Shephard and DeLuca (1980), toxicity occurs at calcidiol levels of 500 ng/ml (approx. 1.25 *µ*M) or above. According to the study of Shephard and DeLuca (1980), rodents can tolerate plasma calcidiol concentrations in the range of 250-1000 nM with normo-calcemia.

### Calcitriol

The dose-limiting hypercalcemic effects of calcitriol are more pronounced than those of calcidiol. As a consequence, amounts of calcitriol that can be administered are lower than those of calcidiol. Mice can become hypercalcemic on day 2 or day 3 at calcitriol doses higher than 750 ng/mouse when administered as a single bolus i.p. (Muindi et al., 2004). According to the study of Muindi et al. (2004) however, calcitriol can be administered as a single bolus i.p. injection up to 500 ng/mouse (in 0.2 ml of normal saline). Unlike daily calcitriol treatments, single calcitriol doses pose a lower hypocalcemia risk and, therefore, allow administration of higher doses. In other animal studies, mice were treated by a single i.p. injection of 0.1 ml propylene glycol containing 300 ng calcitriol (Cantorna et al., 1998b), or by a single i.p. injection of 0.1 ml scafflower oil containing up to 400 ng calcitriol (Nashold et al., 2013).

In clinical trials, the tolerability and pharmacokinetics of intermittent and single administrations of calcitriol have been evaluated (e.g. Beer et al., 2005; Fakih et al., 2007). In the dose escalation study of Beer et al. (2005) a single oral dose of calcitriol up to 165 *µ*g (approx. 2 *µ*g/kg body weight) was relatively well tolerated with generally only grade 1 to 2 adverse events. As described by Muindi et al. (2004), a dose of 2.1 *µ*g/kg in humans is equivalent to a dose of 500 ng/mouse (calculated according to the conversion tables of Freireich et al. (1966).

### Calcipotriol

Calcipotriol (or calcipotriene) is a synthetic derivative of calcitriol, which has similar VDR binding properties as compared to calcitriol, but has low affinity for the vitamin D binding protein (DBP) (for a review, see Trémezaygues and Reichrath; 2011). In vivo studies in rats showed that effects of calcipotriol on calcium metabolism are 100-200 times lower as compared to calcitriol while the tolerance-promoting effects of calcipotriol are comparable to those of calcitriol (e.g., Al-Jaderi et al., 2013).

The half-life of calcipotriol in circulation is measured in minutes (Kragballe, 1995). The rate of clearance (serum half-life of 4 min in rats) is approximately 140 times higher for calcipotriol than for calcitriol. Furthermore, calcipotriol is rapidly metabolized and effects of the metabolites have been demonstrated to be 100 times weaker than those of the parent compound (Kissmeyer and Binderup, 1991).

Single dose toxicicity studies of calcipotriol administered to rats by subcutaneous injection revealed LD₅₀ values of 2.19 mg/kg in males (approx. 440 *µ*g/200-g male rat) and 2.51 mg/kg in females (approx. 380 *µ*g/150-g female rat) (Imaizumi et al., 1996). Rats died probably due to the circulatory and renal disturbance. According to this study, no death occurred up to a single s.c. dose of 540 *µ*g/kg body weight (approx. 81 *µ*g/150-g female rat), and no loss of body weight could be observerd two weeks after administration.

Calcipotriol has been used clinically for more than 10 years for topical treatment of psoriasis without systemic toxicity (for a review, see Plum and DeLuca, 2010). Clinical studies with radiolabeled ointment indicate that approximately 6% of the applied dose of calcipotriene is absorbed systemically when the ointment is applied topically to psoriasis plaques or 5% when applied to normal skin.

### Animal studies

In animal studies, vitamin D molecules and analogs thereof have been used for the treatment of OVA-induced allergy in mice (Ghoreishi et al., 2009), OVA-induced allergic asthma in mice (Taher et al., 2008), insulin-dependent diabetes mellitus in NOD mice (Zella et al., 2003), Lyme arthritis and collagen-induced arthritis in mice (Cantorna et al., 1998b), and experimental autoimmune encephalomyelitis (EAE) in mice (Branisteanu et al., 1995).

In the allergy model, mice were treated on their shaved dorsal skin with 30 mg/day of calcipotriol ointment (contains 50 µg calcipotriol/g; 1.5 µg calcipotriol/30 mg) (Donovex, Leo Pharma) for three days followed by transcutaneous immunization with OVA in the presence of CpG adjuvant. This treatment abolished antigen-specific CD8+ T cell priming and induced CD4+CD5+ Tregs, thereby promoting antigen-specific tolerance (Ghoreishi et al., 2009).

In the allergic asthma mode, OVA-sensitized mice were subjected to allergen-specific immunotherapy by three s.c. injections of 100 µg OVA in the presence of 10 ng calcitriol. This treatment significantly inhibited airway hyper-responsiveness and caused a significant reduction of serum OVA-specific IgE levels (Taher et al., 2008).

In the murine model of type 1 diabetes, a diet containing 50 ng calcitriol/mouse/day was administered three times/week. This treatment prevented diabetes onset in NOD mice as of 200 days (Zella et al., 2003).

In the arthritis models, mice received a daily diet supplemented with 20 ng calcitriol/mouse/day. This dose was found to be effective in inhibiting the progression of arthritis without producing hypercalcemia (Cantorna et al., 1998b).

In the EAE model, i.p. injection of 5 µg of calcitriol/kg body weight (200 ng calcitriol/20-g mouse) every 2 days prevented the appearance of paralysis in 70% of the treated mice (Branisteanu et al., 1995).

### Clinical trials

Evaluation of vitamin D supplementation in clinical trials support some observations made in animal models. It should be noted, however, that only low doses of vitamin D3 or analogs thereof were administered to avoid adverse side effects such as hypercalcemia.

A recent prospective randomized, double-blind study including 48 children from 5 to 18 years of age with newly diagnosed asthma (only sensitive to house dust mites) has demonstrated that after six months of treatment with the glucocorticoid budesonide (800 *µ*g/d, administered as dry powder) and cholecalciferol (500 IU) a significantly lower number of children experienced asthma exacerbation (17%) as compared to the group receiving only glucocorticoids (46%) (Majak et al., 2011).

For the prevention of type 1 diabetes, vitamin D supplementation in infants with 10 µg/day (400 IU/d) does not appear to be sufficient, but doses of 50 µg/day (2000 IU/d) and higher may have a strong protective effect (for a review, see Harris, 2005). A prospective study of vitamin D supplementation in infants and type 1 diabetes including 12,055 pregnant women in Northern Finland showed that regular vitamin D supplements during infancy at doses of over 50 µg/day (2000 IU/d) reduced the relative risk of type 1 diabetes over the subsequent 30 years to 0.14, and at doses of exactly 50 µg/day to 0.22 (Hyppönen et al., 2001). Current U.S. recommendations are in the range of 5-25 µg/day (200-1000 IU/d).

In one open label study, 19 patients with rheumatoid arthritis on methotrexate therapy were treated with 2 µg/day of alfacalcidiol (1(OH)D3) for a period of 3 months. After three months of therapy, 89% of patients experienced an improvement of disease activity, with 45% (9/19) going into remission (Andjelkovic et al., 1999).

Until 2012 seven vitamin D intervention studies have been performed in patients with multiple sclerosis, six of them being performed with inactive 25-OH-D3 and one with active 1,25-(OH)₂D3 (for a review, see Fletcher et al., 2012). Some of these explorative trials did show a trend to improvement, but significant clinical effects were not reported. For example, after oral administration of 4,000-40,000 IU/day of 25-OH-D3 for 28 weeks, a significant decrease in the number of new lesions as assessed by magnetic resonance imaging (MRI) was observed, but disease progression and relapse activity were not affected (Kimball et al., 2007). However, despite the limited clinical efficacy of published vitamin D intervention studies, the studies indicate that systemic vitamin D therapy is well tolerated since the trials (most of which administered doses of 1,000 to 40,000 IU/day of 25-OH-D3) recorded no adverse effects (for a review, see Fletcher et al., 2012). Only the study performed with active 1,25-(OH)₂D3 reported hypercalcemia in those individuals who did not adhere to the recommended dietary restriction of 2.5 µg/day (Wingerchuk et al., 2005).

### 12.4. Therapeutic effective doses of glucocorticoids.

Most preferred glucocorticoids for the method of the present invention are those which exhibit a high anti-inflammatory potency which is proportional to their glucocorticoid potency established for their capacity to elevate glycemia. Glucocorticoids with a high anti-inflammatory potency include but not limited to dexamethasone and betamethasone (for a review, see Longui, 2007). However, glucocorticoids with a moderate anti-inflammatory potency such as prednisone, prednisolone, methylprednisolone, and triamcinolone, as well as those with a lower anti-inflammatory potency such as cortisone and hydrocortisone are also applicable for the method of the present invention.

A dexamethasone dose of 0.25 mg/m²/day corresponds to 2.5 mg/m²/day of prednisolone and hydrocortisone 10 mg/m²/day (for a review, see Gupta and Bhatia, 2008).

All of these glucocorticoids have been studied in a variety of animal models and evaluated in clinical trials. For example, in mice dexamethasone has been administered by i.p. injection at doses of 10-40 *µ*g/20-g mouse (0.5-2.0 mg/kg) for 7 days, leading to a 30% decrease in the number of intestinal VDRs (Hirst and Feldman, 1982a). In rats, dexamethasone has been administered at doses of 0.15-7.5 mg/150-g female rat (1.0-50.0 mg/kg) for 7 days (Hirst and Feldman, 1982b).

In clinical trials, different glucocorticoids and varying combinations thereof have been evaluated. For example, several randomized controlled trials comparing dexamethasone with prednisolone in the treatment of acute asthma exacerbations in children have been published. One study compared emergency department (ED) treatment with an initial dose of oral prednisolone 2 mg/kg (max. 60 mg) followed by 1 mg/kg daily for four days with oral dexamethasone 0.6 mg/kg (max. 16 mg) daily for two days (Qureshi et al., 2001).

Another study compared ED treatment with an initial dose of oral prednisolone 1 mg/kg (max. 30 mg) followed by 1 mg/kg twice daily for five days with a single dose of oral dexamethasone 0.6 mg/kg (max. 18 mg) (Altamimi et al., 2006).

Still another study compared ED treatment with a single dose of prednisolone 2 mg/kg (max. 80 mg) followed by 1 mg/kg (max. 30 mg) twice daily for five days with a single dose of 0.6 mg/kg oral dexamethasone (max. 16 mg) followed by one dose of 0.6 mg/kg oral dexamethasone to take the next day (Greenberg et al., 2008).

### 12.5. Therapeutic effective doses of tofacitinib.

Tofacitinib has been approved by FDA to treat adults with moderately to severely active rheumatoid arthritis (RA) who have had an inadequate response to, or who are intolerant of, methotrexate.

Using the method of the present invention, the application of tofacitinib as tolerance-promoting immune modulator is restricted to a few days until its release from injected hydrogels is completed. Therefore, short-term clinical studies with tofacitinib provide valuable information about therapeutically effective doses of tofacitinib.

### Phase II study of Kremer et al. (2009) over 6 weeks.

Patients (n = 264) were randomized equally to receive placebo, 5 mg, 15 mg or 30 mg of tofacitinib twice daily for 6 weeks, and were followed up for an additional 6 weeks after treatment. By week 6, the American College of Rheumatology 20% improvement criteria (ACR20) response rates were 70.5%, 81.2% and 76.8% in the 5 mg, 15 mg and 30 mg twice-daily groups, respectively, compared with 29.2% in the placebo group. However, the infection rate in both the 15 mg and the 30 mg twice daily groups was 30.4% (versus 26.2% in the placebo group).

### Phase II study of Fleischmann et al. (2012b) over 24 weeks.

In this 24-week, double-blind, phase IIb study, patients with RA (*n* = 384) were randomized to receive placebo, tofacitinib at 1, 3, 5, 10 or 15 mg administered orally twice a day. Treatment with tofacitinib at a dose of ≥3 mg twice a day resulted in a rapid response with significant efficacy compared with placebo, as indicated by the primary end point (ACR20 response at week 12), achieved in 39.2% (3 mg), 59.2% (5 mg), 70.5% (10 mg) and 71.9% (15 mg) in the tofacitinib group compared with 22.0% of patients receiving placebo.

### Preferred concentration of tofacitinib at the site of allergen or autoantigen presentation.

On oral administration of tofacitinib 5 or 10 mg twice a day, serum levels of approximately 100-300 nM are achieved, and such therapeutic levels are known to last for 4-6 h (Kubo et al., 2014).

Based on the different inhibitory potency of tofacitinib for the four members of the Janus kinase family in enzyme assays(see section 2), lower concentrations of tofacitinib inhibit signalling via JAK1 and JAK3 (IL-2, IL-4, IL-7, IL-9, IL-15, IL-21, and IFN-γ), whereas higher concentrations of tofacitinib inhibit also signalling via JAK1 and TYK2 (IL-10, IL-12, IL-20, IL-22, IL-23 and IFNs), and via JAK1, JAK2 and TYK2 (IL-6, IL-11, IL-27 and G-CSF).

For the induction of tolerance according to the method of the present invention it is important, to inhibit JAK1/JAK3-mediated signalling, but to avoid inhibition of JAK1/TYK2-mediated signalling since IL-10 is essential for the induction of tolerance.

Since partial inhibition of JAK1/TYK2-mediated signaling is likely to occur at a concentration of 100-300 nM, for the method of the present invention a lower therapeutic level of tofacitinib is preferred.

### 12.6. Therapeutic effective doses of salicylates.

In a preferred specific embodiment, sodium salicylate, salicylamide or choline magnesium trisalicylate are used as inhibitors of TNFR1-mediated effects according to the methods of the present invention, since these salicylates are applicable for patients suffering from aspirin-induced asthma (also known as aspirin-triad or aspirin-intolerant asthma, AIA) (for a review, see Gohil et al., 2010).

The various inhibitory effects of acetylsalicylic acid (ASA; MW 180.2) and related salicylates (e.g., sodium salicylate; SA; MW 160.11) are concentration dependent. A 50% inhibition by ASA requires a concentration of approx. 2 x 10⁻⁶ M for COX-1, appprox. 3 x 10⁻⁴ M for COX-2, approx. 1 x 10⁻³ M for IL-4 gene transcription, and approx. 3 x 10⁻³ M for NF-kappa B translocation (Cianferoni et al., 2001). Selective inhibition of TNFR1-mediated activation of NF-kappa B requires even higher doses of salicylates in the range of 1-2 x 10⁻² M (Thommesen and Laegreid, 2005).

The therapeutic range for ASA and SA has been restricted to 0.8-1.7 x 10⁻³ M, corresponding to a serum salicylate concentration of 150-300 mg/liter. Restriction of the therapeutic range is necessary to avoid salicylate-related toxicity such as tinnitus (Furst et al., 1987). However, the locally restricted delivery of salicylates at the site of antigen or allergen presentation according to the method of the present invention allows the application of higher salicylate concentrations, since upon diffusion away from the delivery site the salicylate concentration is rapidly decreased to systemically tolerable levels.

Using choline magnesium trisalicylate (CMS) for the treatment of patients with rheumatoid arthritis (RA), a strategy for reaching therapeutic salicylate levels has been developed (Furst et al., 1987). Despite the complex salicylate kinetics, the authors used a simplified weight adjusted dose of 45 mg CMS/kg/day as an initial dose. After one to two weeks of 45 mg CMS/kg/day in two divided doses, 51 of 71 patients with RA had steady state serum salicylate levels between 150 and 300 mg/liter (mean value: 213 ± 10 mg/L). Seventeen patients required dose adjustment using the formula: dosing rate = total clearance x concentration. Since all salicylate preparations are metabolized similarly once they are adsorbed and broken down to the parent salicylate molecule, the results of this study apply to all salicylates.

### 12.7. Therapeutic effective doses of inhibitory aptamers.

DNA and RNA aptamers have been studied in a variety of animal models in the recent past and several of these aptamers have been evaluated in clinical trials (for reviews, see Thiel and Giangrande, 2009; Keefe et al., 2010; Germer et al., 2013). Results obtained from these studies provide useful information for the application of inhibitory aptamers according to the method of the present invention.

Preferred inhibitory aptamers are DNA-based aptamers with a half-life of approximately 60 min (White et al., 2000). DNA-based aptamers have been developed for therapeutic applications as non-modified and modified aptamers. For example, NU172 is a 26-mer oligodeoxynucleotide that is not modified, capped or conjugated and binds to and inhibits thrombin by an interaction with exo-site 1. NU172 was discovered within a degenerate DNA oligonucleotide library using SELEX and was subsequently truncated to 26 nucleotides. This aptamer is currently being evaluated in Phase II clinical trials (for a review, see Keefe et al., 2010).

AS1411, another example of non-modified DNA aptamers, is a G-rich 26-mer oligodeoxynucleotide that contains only guanines and thymines and exists in solution as a guanine-quartet-mediated dimer. AS1411 is thought to elicit its therapeutic effects through its interaction with the cell surface protein nucleolin. AS1411 is currently in Phase II clinical trials for acute myeloid leukaemia (for a review, see Keefe et al., 2010).

ARC1779 is a modified DNA-based aptamer and binds to the A1 domain of von Willebrand factor. ARC1779 was discovered in a degenerate DNA library using SELEX and then truncated to 39 nucleotides. It was also substituted with a single phosphorothioate linkage to increase target affinity and 5'-conjugated to 20 kDa PEG to reduce the rate with which it is subject to renal filtration. Additional alterations included capping at the 3'-terminus with an inverted nucleotide, and 26 2'-O-methyl modifications were introduced to increase nuclease resistance and thermal stability. ARC1779 binds to von Willebrand factor with a Kd value of 2 nM and inhibits platelet function with an EC₉₀ value of 196 nM. ARC1779 is currently in Phase II clinical trials for thrombotic microangiopathies and in patients with carotid artery disease undergoing carotid endarterectomy (for a review, see Keefe et al., 2010).

In a recent animal study, DNA aptamers have been demonstrated to inhibit IL-17RA-mediated synovial inflammation in an experimental mouse model of osteoarthritis (OA), induced by medical meniscectomy (Chen L. et al., 2011). A DNA aptamer termed RA10-6 that could efficiently block IL-17 binding to IL-17RA in a dose-dependent manner in vitro, was administered via intra-articular injections (i.a.) at concentrations of 2 *µ*g, 4 *µ*g or 8 *µ*g in equal volumes once a week for 6 weeks to Balb/C mice one day after meniscectomy. Histological examination and quantitative RT-PCR results showed after injection of RA10-6 inhibition of synovial thickening and reduction in IL-6 levels in the synovial tissue.

### 12.8. Therapeutic effective doses of IL-4 muteins.

Preferred inhibitors of IL-4/IL-13-mediated effects include the human IL-4 double mutant R121D/Y124D (Grunewald et al., 1998; Tony et al., 1994). This double mutant has been evaluated in clinical trials (Wenzel et al., 2007). For animal experiments, an analogous murine double mutant (Q116D/Y119D) has been developed capable of inhibiting IL-4/IL-13-mediated effects in mice.

In a murine model of allergy, the analogous double mutant (Q116D/Y119D) has been administered 2 hours pre- and post-OVA immunization as a 50 µg dose each. Thereafter, treatment was continued from day 1 to 8 with 30 µg double mutant Q116D/Y119D per injection twice a day. This treatment completely abrogated humoral immune responses to OVA and development of allergic symptoms (Grunewald et al., 1998).

In a murine model of OVA-induced allergic airway inflammation, immunotherapeutic treatment (SIT) of the mice was performed by intranasal administration of 10 µg of the double mutant (Q116D/Y119D) together with increasing amounts of OVA (1 µg-1 mg) every fourth day over a 3-week period. In this study, however, mice treated with the IL-4/IL-13 inhibitor during SIT did not produce significantly different results as compared to those treated with increasing amounts of OVA only (Gogishvili et al., 2006). Most likely, intranasal adsorption of the double mutant (Q116D/Y119D) was too inefficient to generate therapeutically effective levels of the double mutant.

### 13. Administration routes of therapeutic compositions

Routes of administration of the compositions of the present invention by injection or by implantation include but are not limited to subcutaneous, intradermal, intramuscular, nasal, transbucal, transmucosal, sublingual, rectal, vaginal, intraocular, or topical administration.

For mucosal immunization, the physicochemical characteristics of the administered components and the delivery vehicle have to be adjusted to stimulate their uptake through the various mucosal routes. For example, alum salts are ineffective when administered by the oral or nasal route. In contrast, cationic chitosan derivatives are of special interest in nasal delivery because of their excellent biocompatibility and muco-adhesive nature (Hagenaars et al., 2010). For example, a thermal-sensitive hydrogel which was formulated as intranasal vaccine with N[(2-hydroxy-3-trimethylammonium)propyl] chitosan chloride (HTCC) and α,β-glycerophosphate, was shown to significantly prolong the antigen residence time in the nasal cavity and to enhance the transepithelial transport via the paracellular routes (Wu et al., 2012).

For allergen-specific immunotherapy the subcutaneous route represents the gold standard. The sublingual-swallow route has emerged as a promising alternative, although less effective than the subcutaneous route. Local side effects of itching and swelling in the mouth are common although, in general, trivial and require no treatment and only rarely result in discontinuation of therapy. The intranasal route has also been shown to be effective, although this route is less attractive for patients and local side effects may require pre-treatment with antihistamines or cromoglycate. For these reasons, the intranasal route has not been widely taken up. The oral route is not recommended currently, although there has been a recent resurgence of interest in the use of microencapsulated extracts which may avoid gastric digestion and facilitate uptake within the small bowel. The inhaled route is not recommended on account of unacceptable side effects.

For autoantigen-specific immunotherapy the subcutaneous route represents also one of the most promising routes. As demonstrated in a recent study, the s.c. route of administration proved to be more effective than the intranasal route for administration of the nine-residue N-terminal peptide of MBP (MBP Ac1-9) for the treatment of EAE, with a 1,000-fold lower dose of antigen being effective for anergy induction when compared with previous studies (Burton et al., 2014).

The preferred route of administration of the compositions of the present invention is subcutaneous administration. This route allows administration of therapeutically sufficient quantities of hydrogel-embedded components and targets peripheral dendritic cells (DCs) including dermal DCs and potentially also epidermal Langerhans cells (LCs) which provide a superior ability to generate Tregs in vivo as compared to lymphoid-resident DCs (Idoyaga et al., 2013).

The DC lineage is heterogeneous and can be classified on the basis of phenotype and origin. Lymphoid tissues, i.e., spleen and tissue-draining lymph nodes (LNs), contain lymphoid-resident DCs that arise from blood-borne precursors and can be loosely categorized as CD8⁺ and CD8⁻ DCs, expressing DEC205 (DEC) and DCIR2, respectively. These lymphoid-resident DCs rapidly take up antigens from the lymph and bloodstream for presentation to T cells. A second group of DCs are migratory DCs, which traffic from peripheral tissues to the draining LN charged with tissue auto-antigens. The nature of migratory DCs depends on the site of LN drainage. In skin draining LNs (sLNs), migratory DCs include epidermal Langerhans cells (LCs) and dermal DCs, which consist of two main subsets, CD103⁺ and CD11b⁺ DCs. Lymphoid-resident DCs and migratory DCs have different roles in the induction of immune responses (for a review, see Villadangos and Schnorrer, 2007). As demonstrated in a recent study (Idoyaga et al., 2013), Langerin⁺ migratory DCs (both dermal migratory CD103⁺ DCs and LCs co-express Langerin) induce antigen-specific Foxp3⁺ Tregs more potently than lymphoid-resident DCs in vivo. Furthermore, in vivo experiments in bone marrow chimeric mice have demonstrated that LCs and dermal CD103⁺ migratory DCs generate Tregs to an equivalent degree. It should be noted, however, that the Treginducing ability of Langerin⁺ migratory DCs is not restricted to skin DCs and is not determined by any particular receptor, as delivery of an auto-antigen, myelin oligodendrocyte glycoprotein, to lung Langerin⁺ migratory DCs also generated antigen-specific Tregs. Most important, the study also demonstrates that targeting an auto-antigen to Langerin⁺ migratory DCs, but not lymphoid-resident DCs, provides a very effective strategy for the treatment of autoimmune diseases.

### 14. Therapeutic protocols

In one embodiment, the present invention discloses therapeutic protocols including dose escalation protocols and injection protocols.

### Dose escalation protocols.

Allergen-specific immunotherapy typically involves administration of escalating doses of allergen in the early phase of treatment, before a high maintenance dose is reached, resulting in allergic desensitization. It is widely accepted that use of dose escalation strategies minimizes the risk of adverse effects associated with allergen-specific immunotherapy, which may range from mild symptoms to anaphylaxis.

Same considerations apply to autoantigen-specific immunotherapy of autoimmune diseases, since dose escalation permits administration of larger antigen doses. Antigen dose plays a critical role in determining the efficacy of immunotherapy, and a dose escalation protocol is imperative to allow safe s.c. administration of the high antigenic doses required for efficient tolerance induction.

In light of these considerations, a dose escalation strategy for efficient auto-antigen-specific tolerance induction has been developed recently using the subcutaneous (s.c.) route (Burton et al., 2014), since mucosal routes of administration have proven safe and effective in animal models of allergy and autoimmunity, but have not translated well to the clinic.

Tolerance induced by escalating dose immunotherapy (EDI) was effective in EAE models whether administered prophylactically or therapeutically (Burton et al., 2014). Using the Tg4 T-cell receptor (TCR) transgenic model of EAE (where >90% of CD4⁺ T cells recognize the nine-residue N-terminal peptide of MBP; MBP Ac1-9), the authors show that self-antigen-specific tolerance can be effectively induced via the subcutaneous (s.c.) route, eliciting IL-10-secreting CD4⁺ T cells with an anergic, regulatory phenotype. At each consecutive stage of EDI the sequential modulation of CD4⁺ T-cell phenotype has been characterized. EDI minimized CD4⁺ T-cell activation and proliferation during the early stages of immunotherapy, preventing excessive systemic cytokine release. Furthermore, the s.c. route of administration proved to be more effective than the intranasal route, with a 1,000-fold lower dose of antigen being effective for anergy induction when compared with previous studies. The ability of cells to secrete IL-10, which serves as a promising mediator of effective antigen-specific immunotherapy (Sabatos-Peyton et al., 2010), and to suppress EAE increased in a dose-dependent manner (Burton et al., 2014).

Most importantly, the dose escalating approach has been successfully used in recent clinical trials (for a review, see Garber, 2014). In one trial, nine patients with MS received a single injection of manipulated immune cells, in escalating doses. The four patients receiving the highest doses showed a reduction in the number of T cells targeting self-antigens (Lutterotti et al., 2013). The most promising trial included 30 patients with relapsing-remitting MS (Walczak et al., 2013), randomized in 1 of 3 arms to receive placebo (n=10 patients), a mixture of 1 mg of PLP₁₃₉₋₁₅₁, 1 mg of MOG₃₅₋₅₅, and 1 mg of MBP₈₅₋₉₉ (n=16), or a mixture of 10 mg of PLP₁₃₉₋₁₅₁, 10 mg of MOG₃₅₋₅₅, and 10 mg of MBP₈₅₋₉₉ (n=4). Myelin antigens were dissolved in phosphate-buffered saline and were applied transdermally in an adhesive skin patch placed on the right upper arm that was changed once per week for 4 weeks and then once per month for 11 months.The effect of myelin peptides was detected at as early as 3 months of treatment, as demonstrated by the reduction in the cumulative number of Gd⁺ lesions vs the placebo group at this point of the study. In this trial, however, the higher dose of myelin peptides (10 mg) was less efficacious than the lower dose, but this result may be due to the low number of patients in the high-dose myelin peptide treatment group.

### Injection protocols

Allergen-specific immunotherapy protocols, in general, involve weekly injections via the subcutaneous route 8-16 weeks during an up-dosing phase, followed by monthly maintenance injections (empirically this has been extended in some centers to 6-8 weeks) for a period of 3-5 years. Cluster immunotherapy up-dosing schedules may involve repeated injections at each clinic visit. Rush protocols which may involve repeated up-dosing injections in order to achieve maintenance doses within several hours are applicable to venom sensitive patients, although are unsuitable for patients with inhalant allergies in view of the marked increased occurrence of side effects.

Autoantigen-specific immunotherapy protocols, in general, involve also repeated administrations of autoantigens. For example, immunotherapy of RA patients with recombinant human cartilage glycoprotein-39 (HC gp-39) has been performed by intranasal administration of 30, 150, 300 or 600 *µ*g of HC gp-39 once a week for 13 weeks (Landewe et al., 2010). In another study, immunotherapy of MS patients has been performed by transdermal administration of 3 myelin antigens, each at a dosage of 1 or 10 mg in an adhesive skin patch placed on the right upper arm that was changed once per week for 4 weeks and then once per month for 11 months (Walczak et al., 2013).

The method of the present invention aims for shorter treatment periods, but the number of required s.c. injections needs to be determined by analysis (combination of microarray analyses and real-time PCR including RT-PCR) of the sequential modulation of CD4⁺ T-cell phenotype towards IL-10-secreting CD4⁺ T cells with an anergic, regulatory phenotype after each injection step (Burton et al., 2014).

In one embodiment, hydrogel-based compositions of the present invention for the treatment of allergic and autoimmune diseases are administered subcutaneously by at least three injections (at one-week intervals up to four-weeks intervals) during an up-dosing phase (escalating dosing phase), followed by monthly maintenance injections. Preferred are 3- to 4-weeks intervals to allow for a complete release of hydrogel-embedded components. The allergen or autoantigen dose continually increases in compositions, being lowest in the composition used for the first injection. At each consecutive stage of the escalating dose immunotherapy the sequential modulation of CD4⁺ T-cell phenotype towards IL-10-secreting CD4⁺ T cells with an anergic, regulatory phenotype is analyzed as described in section 15.

In another embodiment, hydrogel-based compositions of the present invention for the treatment of allergic and autoimmune diseases are administered subcutaneously (s.c.) by at least one injection using one therapeutically optimal dose based on the results of a prior dose escalation study as described (Burton et al., 2014). Subsequent s.c. injections are performed at one-week or two-weeks intervals. Preferred are two-weeks intervals to allow for a complete release of hydrogel-embedded components.

The number of required s.c. injections is determined for each patient individually by analysis (combination of microarray analyses and real-time PCR including RT-PCR) of the sequential modulation of CD4⁺ T-cell phenotype towards IL-10-secreting CD4⁺ T cells with an anergic, regulatory phenotype after each injection step (Burton et al., 2014). In some cases, a single injection of a hydrogel-based composition of the present invention may also be sufficient to induce IL-10-secreting CD4⁺ T cells with an anergic, regulatory phenotype to an extent that allows suppression of allergic or autoimmune disease symptoms. For example, a single s.c. injection of tolerizing liposomes loaded with antigen and NF-κB inhibitors into mice suffering from antigen-induced inflammatory arthritis has been demonstrated to reduce the mean clinical score by approximately 50% within four days (Capini et al., 2009). However, in the majority of cases repeated s.c. injections of a hydrogel-based composition of the present invention may be necessary to induce tolerance via a gradual establishment of a regulatory CD4⁺ T-cell phenotype.

### 15. Surrogate markers for allergen- or autoantigen-specific tolerance induction.

The gradual establishment of a regulatory CD4⁺ T-cell phenotype is characterized by expression of specific negative costimulatory molecules and transcription factors, in addition to the regulatory cytokine IL-10, all of which are used surrogate markers for allergen/autoantigen-specific tolerance induction according to the method of the present invention. Transcription factors previously associated with IL-10 expression include Maf, Ahr and Nfil3 (Pot et al., 2009; Motomura et al., 2011; Apetoh et al., 2010). The induction of IL-21 expression is also noteworthy, as IL-21 contributes to the IL-27-driven production of IL-10 in murine T cells (Pot et al., 2009). The most notable correlation with effective immunotherapy is the induction of a set of negative costimulatory molecules including PD-1, LAG-3, TIM-3 and TIGIT. Some of these have previously been associated with T cell exhaustion (Wherry, 2011), while others have been described as markers of IL-10-secreting Tr1 cells (Gagliani et al., 2013; Okamura et al., 2009). Recently, a positive correlation between IL-10 production and the expression of LAG-3, TIGIT, PD-1 and TIM-3 was demonstrated (Burton et al., 2014). However, expression of these markers is not uniquely restricted to the IL-10⁺ population; only 11% of LAG-3⁺ cells are IL-10⁺ and ∼50% of Tim-3⁺ or TIGIT⁺ cells are IL-10⁺ (Burton et al., 2014). These results suggest that while LAG-3 and PD-1 are good markers of the anergic CD4⁺ T-cell population induced by EDI, TIGIT and TIM-3 are better discriminators of IL-10-secreting cells induced by immunotherapy (Burton et al., 2014). CD49b was also found to correlate with IL-10 expression in CD4⁺ T cells from autoantigen-treated mice; however, within the LAG-3⁺CD49b⁺ population, only 33% of cells were found to express IL-10 (Burton et al., 2014).

Although the necessary extent of established regulatory CD4⁺ T-cell phenotypes for the induction of tolerance may vary for different patient and also for different allergic and autoimmune diseases, a recent successful, escalating dose immunotherapeutic approach for the treatment of EAE provides valuable cornerstones (Burton et al., 2014). In this study, mice were treated every 3-4 days six times s.c. with an escalating dose of autoantigen (escalating from 0.08 *µ*g to 0.8 *µ*g and then to 4 x 8 *µ*g, or escalating from 0.08 *µ*g to 0.8 *µ*g and then to 8 *µ*g and finally to 3 x 80 *µ*g). Induction of tolerance was associated with a percentage of CD4⁺ T cells expressing IL-10, c-Maf or LAG-3 in at least 50% of the cells. A rising percentage of TIGIT⁺ cells also accumulated during autoantigen-specific immunotherapy (20% of activated CD4⁺ T cells). The proportion of cells expressing TIM-3 remained relatively stable throughout the treatment, while the percentage of PD-1⁺ cells increased upon initial CD4⁺ T-cell activation and further increased during the later stages of the treatment.

### EXAMPLES

### 1. PLGA-PEG-PLGA HYDROGELS

### 1.1. SYNTHESIS AND CHARACTERIZATION OF PLGA-PEG-PLGA HYDROGELS

This example describes the synthesis and chacterization of thermogelling PLGA-PEG-PLGA hydrogels.

The biodegradable triblock polymer described in this example has a PLG/PEG weight ratio of 2.3 (70/30), and a lactide/glycolide molar ratio of approx. 15/1. Synthesis of the triblock copolymer is performed according to published protocols (Qiao *et al*., 2005).

### 1.1.1. Copolymer synthesis

7.8 g PEG 1500 (Fluka Munich) are melted at 120°C and dried under vacuum and gentle stirring for 2 hours in a two-headed glass reaction flask. The vaccuum is replaced by nitrogen atmosphere. 16.6 g glycolide (Sigma-Aldrich Munich) and 0.89 g D,L-lactide (ABCR GmbH Karlsruhe) are added against a slow nitrogen stream. After all educts are melted, 40 *µ*l Tin(II)-ethylhexanoate (Sigma-Aldrich Munich) are added as catalyst. The temperature is increased to 150°C and the reaction is continued for 16 hours under gentle stirring and nitrogen atmosphere. Thereafter the polymer reaction mix is cooled down to room temperature.

### 1.1.2. Purification of PLGA-PEG-PLGA triblock polymer

The polymer reaction mix is dissolved in 25 ml acetonitrile (Roth Karlsruhe) at room temperature with stirring for approx. 30 minutes. Then 150 ml of water (MilliQ grade destilled water) is added to the organic polymer solution and stirred at 60°C until the pre-dissolved polymer is completely dissolved. Thereafter the solution is heated to 90°C without stirring in order to precipitate the polymer. To remove impurities the reaction is cooled down to room temperature and the aqueous phase is discarded. Solvation of the polymer and precipitation is repeated twice using 15 ml acetonitrile and 150 ml of water.

### 1.1.3. Preparation for use and storage

The purified precipitated polymer is dissolved in 15 ml acetonitrile and sterile filtered using PVDF membrane filters (200 nm, Supelco). The sterile acetonitrile-polymer solution is aliquoted in 2 ml flat bottom tubes and lyophilized. The lyophilized polymer is stored at 4-8°C. Prior to use the freeze-dried polymer is dissolved at 4°C in the required aqueous solution for 72h.

### 1.1.4. Molecular weight determination

The molecular weight of the copolymer is determined by gel permeation chromatography (GPC) using polystyrene standards as described by Qiao et al. (2005).

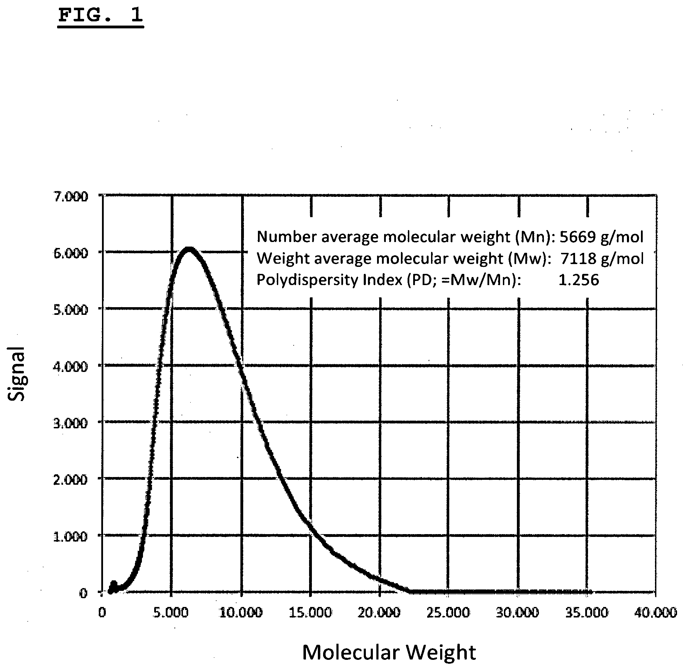

Fig. 1 shows a representative GPC analysis of the copolymer of Example 1.1.

### 1.1.5. Measurement of gelation temperature

The gelation temperature is determined as described by Qiao et al. (2005). A 2 ml transparent vial is filled with 200 *µ*l water solution of the copolymer (20% w/w and 25% w/w), is placed in a water bath. The solution is heated in 1°C steps beginning at 26°C in a thermomixing device (Eppendorf). At each temperature step the gelation is checked by careful inversion of the tube. When the solution is not free-flowing, gelation of the solution occurred, the temperature read from the thermometer is determined as gelation temperature.

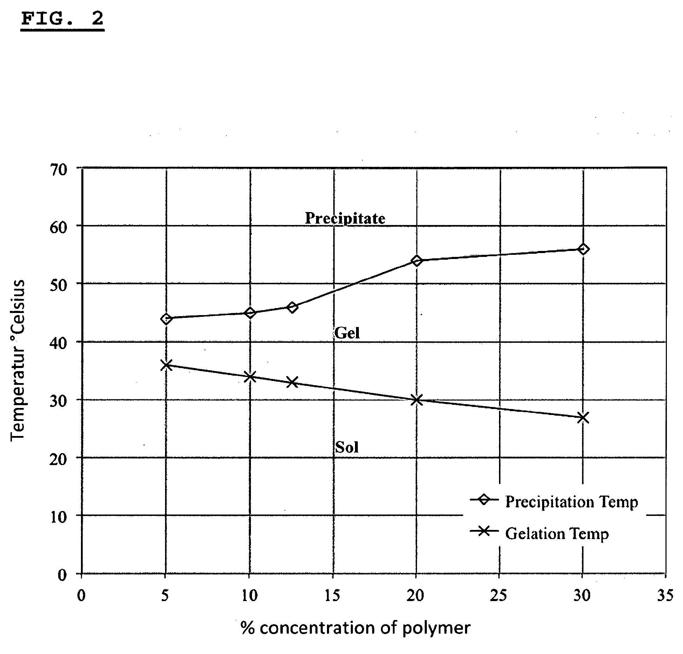

Fig. 2 shows the gelling temperature of the copolymer of Example 1.1 in dependence of the polymer concentration.

### 1.1.6. In vivo gel formation

To test the in vivo gel formation behavior of PLGA-PEG-PLGA hydrogels of Example 1.1, the hydrogel is injected subcutaneously into mice that have been anesthetized with ethyl ether.

240 *µ*l of a 18% PLGA-PEG-PLGA blockpolymer solution in water is mixed with 30 *µ*l of 10x PBS (pH 7.4) buffer and 30 *µ*l of 30 mM morpholino ethanesulfonic acid (MES) buffered solution (pH 6.0) at room temperature.

The gel sol solution is injected into hairless mouse (strain SKH1), euthanized by cervical dislocation and kept in 37°C pad, at two sites in amounts of 50 and 100 *µ*l.

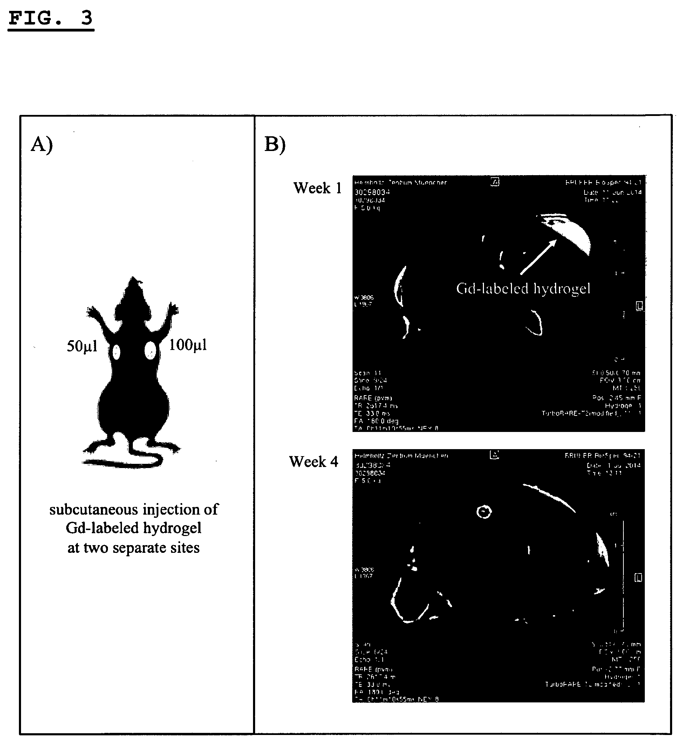

After 2 hours the gels are analyzed in the mouse by magnetic resonance imaging (MRI), using a Bruker BioSpec 9421 instrument. The position and gelled status of the hydrogel is further confirmed by chirurgical examination of the implanted hydrogel (see figure 3).

### 1.2. IN VITRO DEGRADATION OF PLGA-PEG-PLGA HYDROGELS

The in vitro degradation behavior of the copolymer of Example 1.1 is evaluated by the mass loss and/or the molecular weight reduction with time upon incubation in phosphate-buffered saline.

Samples (0.5 ml) are incubated in phosphate-buffered saline pH 7.4 at 37°C under mild agitation in a water bath. The solid residues are removed from the incubation medium at scheduled time intervals and lyophilized. The samples are weighted and the weight loss is calculated. For determination of the molecular weight reduction, the solid residues are solved in cold water and analyzed by gel permeation chromatography using polystyrene standards as described by Qiao *et al*. (2005).

### 2. SYNTHESIS OF PS-LIPOSOMES

This example describes the synthesis of unilamellar PS-liposomes from a lipid mixture of phosphatidyldserine (PS) (either 1,2-dipalmitoyl-sn-glycero-3-phospho-L-serine sodium salt (Sigma-Aldrich), 1-palmitoyl-2-oleoyl-sn-3-glycerophospho -L-serine (POP-L-S), or bovine brain phosphatidyldserin (Avanti Polar Lipids)), phosphatidylcholine (PC) (either 1,2-dipalmitoyl-sn-glycero-3-phosphocholine (DMPC; Sigma-Aldrich), 1-palmitoyl-2-oleoyl-sn-3-glycerophosphocholine (POPC; Avanti Polar Lipids), or egg phosphatidylcholine (egg-PC; Avanti Polar Lipids)), and cholesterol (CH; Avanti Polar Lipds) at a ratio of 30:30:40 (PS to PC to CH) according to Hoffmann et al. (2005).

### 2.1. SYNTHESIS OF PS-LIPOSOMES

Method for CH-rich liposomes (molar ratio of CH: 40%). A chloroform/methanol (2:1, v/v) solution containing 30 µmol PS (approx. 22.7 mg), 30 µmol PC (approx. 22.0 mg) and 40 µmol CH (approx. 15.5 mg) is placed in a conical flask and dried by rotary evaporation to prepare a thin lipd film. Thereafter, the flask is placed in a desiccator for at least one hour to completely remove the solvent. Then, 1.7 ml of phosphate-buffered saline (PBS) is added (approx. 35 mg total lipid/ml) and multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes.

The final liposomal suspension contains approx. 59 µmol (approx. 35 mg) of lipd/ml (59 mM liposomal suspension). Unilamellar PS-liposomes prepared by this procedure have been shown to disperse uniformly in physiological medium at a concentration of 60 mM total lipid due to repulsion forces (Harel-Adar et al., 2011).

The degree of PS exposure on liposomes is assessed by binding of FITC-annexin V to surface-exposed PS and subsequent analysis by FACS.

### 2.2. SYNTHESIS OF PS-LIPOSOMES CONTAINING ATP/UTP

Method A: CH-rich liposomes (molar ratio of CH: 40%). A chloroform/methanol (2:1, v/v) solution containing 30 µmol PS (approx. 22.7 mg), 30 µmol PC (approx. 22.0 mg) and 40 µmol CH (approx. 15.5 mg) is placed in a conical flask and dried by rotary evaporation to prepare a thin lipd film. Thereafter, the flask is placed in a desiccator for at least one hour to completely remove the solvent. Then, 1.7 ml of phosphate-buffered saline (PBS) containing 20 *µ*M ATP (11 *µ*g/ml; MW 551.14) and 20 *µ*M UTP (9.7 *µ*g/ml; MW 494.14) is added (lipid concentration: approx. 35 mg/ml).

Multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes.

The final liposomal suspension contains approx. 59 µmol (approx. 35 mg) of lipd/ml (59 mM liposomal suspension), and approx. per ml liposomal suspension 5 *µ*g ATP (approx. 10 *µ*M) and 5 *µ*g UTP (approx. 10 *µ*M) per ml liposomal suspension, based on incorporation rates of approx. 50% (compare Eloy et al., 2014).

Method B. CH-poor liposomes (molar ratio of CH: 10%). A chloroform/methanol (2:1, v/v) solution containing 30 µmol PS (approx. 22.7 mg), 60 µmol PC (approx. 44.0 mg) and 10 µmol CH (approx. 3.9 mg) is placed in a conical flask and dried by rotary evaporation to prepare a thin lipd film. Thereafter, the flask is placed in a desiccator for at least one hour to completely remove the solvent. Then, 1.7 ml of phosphate-buffered saline (PBS) containing 20 *µ*M ATP (11 *µ*g/ml; MW 551.14) and 20 *µ*M UTP (9.7 *µ*g/ml; MW 494.14) is added (lipid concentration: approx. 41.5 mg/ml).

Multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes.

The final liposomal suspension contains approx. 59 µmol (approx. 41.5 mg) of lipd/ml (59 mM liposomal suspension), and approx. 5 *µ*g ATP (approx. 10 *µ*M) and 5 *µ*g UTP (approx. 10 *µ*M) per ml liposomal suspension, based on incorporation rates of approx. 50% (compare Eloy et al., 2014).

Analyses. The phospholipid content is determined with a phosphate assay in the organic phase after extraction of the liposomal preparations with chloroform (Rouser et al., 1970). The ATP concentration in the liposomal suspension is determined in a bioluminescence assay (sensitivity: pmol range) with recombinant firefly luciferase and its substrate D-luciferin (Molecular Probes) after dissolution of the liposomes in ethanol. The solubility of ATP in ethanol is low (less than 1 mg/ml at 25°C) but sufficient for low liposomal ATP concentrations in the *µ*g range. The liposomal ATP content is used as measure of the liposomal UTP content.

### 2.3. SYNTHESIS OF PS-LIPOSOMES CONTAINING ATP/UTP AND CALCIPOTRIOL

Method A: CH-rich liposomes (molar ratio of CH: 40%). A chloroform/methanol (2:1, v/v) solution containing 30 µmol PS (approx. 22.7 mg), 30 µmol PC (approx. 22.0 mg) and 40 µmol CH (approx. 15.5 mg) is placed in a conical flask, mixed with a stock solution of calcipotriol in methanol (10 mg/ml) in a molar ratio of calcipotriol to lipd of 0.015 to 1.0 (620 µg calcipotriol corresponding to approx. 1.5 µmol), and dried by rotary evaporation to prepare a thin lipd film. Thereafter, the flask is placed in a desiccator for at least one hour to completely remove the solvent. Then, 1.7 ml of phosphate-buffered saline (PBS) containing 20 *µ*M ATP (11 *µ*g/ml; MW 551.14) and 20 *µ*M UTP (9.7 *µ*g/ml; MW 494.14) is added (lipid concentration: approx. 35 mg/ml).

Multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes.

The final liposomal suspension contains approx. 59 µmole (approx. 35 mg) of lipd/ml (59 mM liposomal suspension), approx. per ml liposomal suspension 5 *µ*g ATP (approx. 10 *µ*M) and 5 *µ*g UTP (approx. 10 *µ*M) per ml liposomal suspension, based on incorporation rates of approx. 50% (compare Eloy et al., 2014), and approx. 310 µg (751 nmol) calcipotriol/ml liposomal suspension, based on an incorporation rate of 85%.

Method B. CH-poor liposomes (molar ratio of CH: 10%). A chloroform/methanol (2:1, v/v) solution containing 30 µmol PS (approx. 22.7 mg), 60 µmol PC (approx. 44.0 mg) and 10 µmol CH (approx. 3.9 mg) is placed in a conical flask, mixed with a stock solution of calcipotriol in methanol (10 mg/ml) in a molar ratio of calcipotriol to lipd of 0.015 to 1.0 (620 µg calcipotriol corresponding to approx. 1.5 µmol), and dried by rotary evaporation to prepare a thin lipd film. Thereafter, the flask is placed in a desiccator for at least one hour to completely remove the solvent. Then, 1.7 ml of phosphate-buffered saline (PBS) containing 20 *µ*M ATP (11 *µ*g/ml; MW 551.14) and 20 *µ*M UTP (9.7 *µ*g/ml; MW 494.14) is added (lipid concentration: approx. 41.5 mg/ml).

Multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes. The final liposomal suspension contains approx. 59 µmol (approx. 41.5 mg) of lipd/ml (59 mM liposomal suspension), approx. 5 *µ*g ATP (approx. 10 *µ*M) and 5 *µ*g UTP (approx. 10 *µ*M) per ml liposomal suspension, based on incorporation rates of approx. 50% (compare Eloy et al., 2014), and approx. 310 µg (751 nmol) calcipotriol/ml liposomal suspension, based on an incorporation rate of 85%.

Analyses. The phospholipid content is determined with a phosphate assay in the organic phase after extraction of the liposomal preparations with chloroform (Rouser et al., 1970). The ATP concentration in the liposomal suspension is determined in a bioluminescence assay (sensitivity: pmol range) with recombinant firefly luciferase and its substrate D-luciferin (Molecular Probes) after dissolution of the liposomes in ethanol. The solubility of ATP in ethanol is low (less than 1 mg/ml at 25°C) but sufficient for low liposomal ATP concentrations in the *µ*g range. The liposomal ATP content is used as measure of the liposomal UTP content. The calcipotriol concentration in the liposomal suspensions is determined by UV absorption at 252 nm (molar extinction coefficient of 42,000; Plum et al., 2004) after dissolution of the liposomes in ethanol. Alternatively, the calcipotriol concentration in the liposomal suspensions can be determined by reversed phase HPLC using a C18-column and acetonitrile:water (77:23) as elution agent (Cirunay et al., 1998). Calcipotriol is detected by UV absorption at 263 nm.

### 2.4. SYNTHESIS OF PS-LIPOSOMES CONTAINING ATP/UTP AND DEXAMETHASONE PHOSPHATE (DexP)

Method A: CH-rich liposomes (molar ratio of CH: 40%). A chloroform/methanol (2:1, v/v) solution containing 30 µmol PS (approx. 22.7 mg), 30 µmol PC (approx. 22.0 mg) and 40 µmol CH (approx. 15.5 mg) is placed in a conical flask and dried by rotary evaporation to prepare a thin lipd film. Thereafter, the flask is placed in a desiccator for at least one hour to completely remove the solvent. Then, 1.7 ml of phosphate-buffered saline (PBS) containing 20 *µ*M ATP (11 *µ*g/ml; MW 551.14), 20 *µ*M UTP (9.7 *µ*g/ml; MW 494.14), and 20 mg DexP (Dex-ratio-pharm, Ratiopharm) is added (lipid concentration: approx. 35 mg/ml).

Multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes.

The final liposomal suspension contains approx. 59 µmol (approx. 35 mg) of lipd/ml (59 mM liposomal suspension), approx. 5 *µ*g ATP (approx. 10 *µ*M) and 5 *µ*g UTP (approx. 10 *µ*M) per ml liposomal suspension, based on incorporation rates of approx. 50% (compare Eloy et al., 2014), and and approx. 1.2 mg liposomal DexP/ml (based on 10% encapsulation efficiency).

Method B. CH-poor liposomes (molar ratio of CH: 10%). A chloroform/methanol (2:1, v/v) solution containing 30 µmol PS (approx. 22.7 mg), 60 µmol PC (approx. 44.0 mg) and 10 µmol CH (approx. 3.9 mg) is placed in a conical flask and dried by rotary evaporation to prepare a thin lipd film. Thereafter, the flask is placed in a desiccator for at least one hour to completely remove the solvent. Then, 1.7 ml of phosphate-buffered saline (PBS) containing 20 *µ*M ATP (11 *µ*g/ml; MW 551.14) and 20 *µ*M UTP (9.7 *µ*g/ml; MW 494.14) is added (lipid concentration: approx. 41.5 mg/ml).

Multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes.

The final liposomal suspension contains approx. 59 µmol (approx. 41.5 mg) of lipd/ml (59 mM liposomal suspension), approx. 5 *µ*g ATP (approx. 10 *µ*M) and 5 *µ*g UTP (approx. 10 *µ*M) per ml liposomal suspension, based on incorporation rates of approx. 50% (compare Eloy et al., 2014), and approx. 1.2 mg liposomal DexP/ml (based on 10% encapsulation efficiency).

Analyses. The phospholipid content is determined with a phosphate assay in the organic phase after extraction of the liposomal preparations with chloroform (Rouser et al., 1970). The aqueous phase after extraction is used to determine DexP content using a spectrophotometric method (Singh and Verma, 2008). The method involves oxidation of the corticosteroid by iron (III) and subsequent complexation of iron (II) with potassium hexacynoferate (III), forming a bluish green coloured complex with maximum absorbance at 780 nm (Beer's law range: 10-50 *µ*g/ml; molar absorptivity (M⁻¹cm⁻¹): for DexP 0.55 x 10⁴). Alternatively, the DexP content is determined by reversed-phase HPLC at 254 nm using a C18-column and methanol:water (1:1) as solvent (Kwak and D'Amico, 1995). Furthermore, anti-DexP polyclonal antibodies (MyBioSource) are commercially available for the determination of DexP by Elisa. The aqueous phase after extraction is also used to determine the ATP concentration in the liposomal suspension in a bioluminescence assay (sensitivity: pmol range) with recombinant firefly luciferase and its substrate D-luciferin (Molecular Probes). The liposomal ATP content is used as measure of the liposomal UTP content.

### 2.5. SYNTHESIS OF PS-LIPOSOMES CONTAINING ATP/UTP, CALCIPOTRIOL AND DEXAMETHASONE PHOSPHATE (DexP)

Method A: CH-rich liposomes (molar ratio of CH: 40%). A chloroform/methanol (2:1, v/v) solution containing 30 µmol PS (approx. 22.7 mg), 30 µmol PC (approx. 22.0 mg) and 40 µmol CH (approx. 15.5 mg) is placed in a conical flask, mixed with a stock solution of calcipotriol in methanol (10 mg/ml) in a molar ratio of calcipotriol to lipd of 0.015 to 1.0 (620 µg calcipotriol corresponding to approx. 1.5 µmol), and dried by rotary evaporation to prepare a thin lipd film. Thereafter, the flask is placed in a desiccator for at least one hour to completely remove the solvent. Then, 1.7 ml of phosphate-buffered saline (PBS) containing 20 *µ*M ATP (11 *µ*g/ml; MW 551.14), 20 *µ*M UTP (9.7 *µ*g/ml; MW 494.14), and 20 mg DexP (Dex-ratio-pharm, Ratiopharm) is added (lipid concentration: approx. 35 mg/ml).

Multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes.

The final liposomal suspension contains approx. 59 µmole (approx. 35 mg) of lipd/ml (59 mM liposomal suspension), approx. 5 *µ*g ATP (approx. 10 *µ*M) and 5 *µ*g UTP (approx. 10 *µ*M) per ml liposomal suspension, based on incorporation rates of approx. 50% (compare Eloy et al., 2014), approx. 310 µg (751 nmol) calcipotriol/ml liposomal suspension, based on an incorporation rate of 85%, and approx. 1.2 mg liposomal DexP/ml (based on 10% encapsulation efficiency).

Method B. CH-poor liposomes (molar ratio of CH: 10%). A chloroform/methanol (2:1, v/v) solution containing 30 µmol PS (approx. 22.7 mg), 60 µmol PC (approx. 44.0 mg) and 10 µmol CH (approx. 3.9 mg) is placed in a conical flask, mixed with a stock solution of calcipotriol in methanol (10 mg/ml) in a molar ratio of calcipotriol to lipd of 0.015 to 1.0 (620 µg calcipotriol corresponding to approx. 1.5 µmol), and dried by rotary evaporation to prepare a thin lipd film. Thereafter, the flask is placed in a desiccator for at least one hour to completely remove the solvent. Then, 1.7 ml of phosphate-buffered saline (PBS) containing 20 *µ*M ATP (11 *µ*g/ml; MW 551.14), 20 *µ*M UTP (9.7 *µ*g/ml; MW 494.14), and 20 mg DexP (Dex-ratio-pharm, Ratiopharm) is added (lipid concentration: approx. 41.5 mg/ml).

Multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The liposome suspension is centrifuged at 5000xg for 5 minutes and the supernatant is discarded by pipetting and replaced by 1.7 ml of PBS and vortexed to resuspend the liposomes.

The final liposomal suspension contains approx. 59 µmol (approx. 41.5 mg) of lipd/ml (59 mM liposomal suspension), approx. 5 *µ*g ATP (approx. 10 *µ*M) and 5 *µ*g UTP (approx. 10 *µ*M) per ml liposomal suspension, based on incorporation rates of approx. 50% (compare Eloy et al., 2014), approx. 310 µg (751 nmol) calcipotriol/ml liposomal suspension, based on an incorporation rate of 85%, and approx. 1.2 mg liposomal DexP/ml (based on 10% encapsulation efficiency).

Analyses. The phospholipid content is determined with a phosphate assay in the organic phase after extraction of the liposomal preparations with chloroform (Rouser et al., 1970). The aqueous phase after extraction is used to determine DexP content using a spectrophotometric method (Singh and Verma, 2008). The method involves oxidation of the corticosteroid by iron (III) and subsequent complexation of iron (II) with potassium hexacynoferate (III), forming a bluish green coloured complex with maximum absorbance at 780 nm (Beer's law range: 10-50 *µ*g/ml; molar absorptivity (M⁻¹cm⁻¹): for DexP 0.55 x 10⁴). Alternatively, the DexP content is determined by reversed-phase HPLC at 254 nm using a C18-column and methanol:water (1:1) as solvent (Kwak and D'Amico, 1995). Furthermore, anti-DexP polyclonal antibodies (MyBioSource) are commercially available for the determination of DexP by Elisa.

The aqueous phase after extraction is also used to determine the ATP concentration in the liposomal suspension in a bioluminescence assay (sensitivity: pmol range) with recombinant firefly luciferase and its substrate D-luciferin (Molecular Probes). The liposomal ATP content is used as measure of the liposomal UTP content. The calcipotriol concentration in the liposomal suspensions is determined by UV absorption at 252 nm (molar extinction coefficient of 42,000; Plum et al., 2004) after dissolution of the liposomes in ethanol. Alternatively, the calcipotriol concentration in the liposomal suspensions can be determined by reversed phase HPLC using a C18-column and acetonitrile:water (77:23) as elution agent (Cirunay et al., 1998). Calcipotriol is detected by UV absorption at 263 nm.

### 3. COMPOSITIONS OF PLGA-PEG-PLGA HYDROGELS AND PS-LIPOSOMES

This example describes the synthesis and chacterization of thermogelling PLGA-PEG-PLGA hydrogels containing phosphatidylserine (PS)-liposomes.

### 3.1. SYNTHESIS OF HYDROGEL-EMBEDDED PS-LIPOSOMES

Different concentrations of the PLGA-PEG-PLGA copolymer of Example 1 (22.5% w/w, and 30% w/w) in water are mixed with liposomal suspensions in PBS of Example 2 (2.1.-2.5.) at a ratio of two volumes hydrogel solution to one volume of liposomal suspension. The final concentration of the hydrogel is 15% (w/w) or 20% (w/w).

Hydrogels containing liposomes prepared according to Method A (cholesterol-rich PS-liposomes) provide a final concentration of approx. 20 µmole (12 mg) of lipd/ml, and combinations of approx. 1.7 *µ*g ATP (approx. 3.3 *µ*M; MW 551.14), 1.7 *µ*g UTP (approx. 3.3 *µ*M; MW 494.14), approx. 100 µg (242 nmol; MW 412.6) calcipotriol/ml, and approx. 400 *µ*g liposomal DexP (775 nmol; MW 516.4)/ml).

Hydrogels containing liposomes prepared according to Method B (cholesterol-poor PS-liposomes) provide a final concentration of approx. 20 µmole (13.8 mg) of lipd/ml, and combinations of approx. 1.7 *µ*g ATP (approx. 3.3 *µ*M; MW 551.14), 1.7 *µ*g UTP (approx. 3.3 µM; MW 494.14), approx. 100 µg (242 nmol; MW 412.6) calcipotriol/ml, and approx. 400 *µ*g liposomal DexP (775 nmol; MW 516.4)/ml).

### 3.2. GELATION CHARACTERISTICS OF HYDROGEL-PS-LIPOSOME

### COMPOSITIONS

The gelation temperature of hydrogel/PS-liposome composits of Example 3.1 is determined as described by Qiao et al. (2005). Transparent vials are filled with 200 *µ*l water containing different concentrations of the copolymer of Example 1. (22.5% w/w, and 30% w/w), cooled to 4°C and mixed with 100 *µ*l PBS containing liposomes of Example 2.1 or 100 *µ*l PBS containing no liposomes. The final concentrations of the copolymer are 15% (w/w) and 20% (w/w) containing liposomes at a concentration of approx. 20 µmole (12 mg) of lipd/ml. The vials are placed in a water bath and each solution is heated in 1°C steps beginning at 20°C in a thermomixing device (Eppendorf). At each temperature step the gelation is checked by careful inversion of the tube. When the solution is not free-flowing (gelation of the solution occurred), the temperature is determined as gelation temperature.

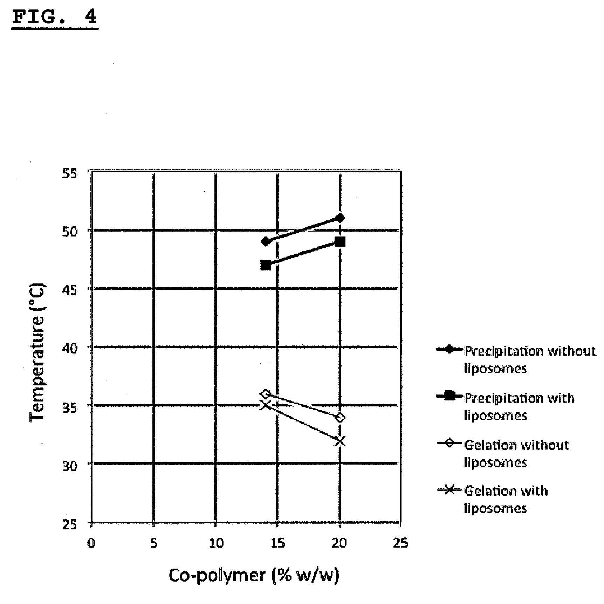

Figure 4 shows the effect of PS-liposomes on the gelation characteristics of PLGA-PEG-PLGA copolymers.

### 3.3. RELEASE KINETICS OF HYDROGEL-EMBEDDED PS-LIPOSOMES

This example describes the in vitro release characteristics of PS-Liposomes with encapsulated FITC-BSA (bovine serum albumin-fluorescein isithiocyanate conjugate) from thermogelling PLGA-PEG-PLGA hydrogels.

### 3.3.1. Synthesis of the PLGA-PEG-PLGA hydrogel

The biodegradable triblock polymer described in this example is prepared as described in Example 1.

### 3.3.2. Synthesis of FITC-BSA-containing PS-liposomes according to Example 2.1.

A chloroform/methanol (2:1, v/v) solution containing 30 µmol PS (approx. 22.7 mg), 30 µmol PC (approx. 22.0 mg) and 40 µmol CH (approx. 15.5 mg) is placed in a conical flask and dried by rotary evaporation to prepare a thin lipd film. Thereafter, the flask is placed in a desiccator for at least one hour to completely remove the solvent. Then, 1.5 ml of PBS containing 1.5 mg FITC-labeled bovine serum albumin (FITC-BSA, Sigma-Aldrich) is added and multilamellar vesicles are generated by intense vortex dispersion. For the preparation of unilamellar vesicles, the multilamellar preparation is extruded 10 times through a 1 µm pore polycarbonate membrane (Nucleopore, USA). The liposome suspension is centrifuged at 5000xg for 5 minutes and the supernatant is discarded by pipetting and replaced by 1.5 ml of PBS and vortexed to resuspend the liposomes. The final liposomal suspension contains approx. 66.7 µmol (40.1 mg) of lipd/ml.

The amount of encapsulated FITC-BSA in liposomes is determined by dissolving the lipid vesicles with 1% (v/v) Triton X-100 and monitoring the absorbance of FITC-BSA at 495 nm. Using the conditions of this example, the encapsulation efficacy is 22% (220 *µ*g FITC-BSA/ml PS-liposome suspension).

### 3.3.3. In vitro release of FITC-BSA-containing PS-liposomes from hydrogel/liposome composits

The in vitro release of FITC-BSA-containing PS-liposomes from hydrogel/PS-liposome composits is determined after gelling of the hydrogel/PS-liposome composits at 37°C by monitoring the supernatant for the development of absorbance at 495 nm in the presence of Triton X-100.

Vials are filled with 200 *µ*l water containing different concentrations of the copolymer of Example 1 (22.5% w/w, and 30% w/w), cooled to 4°C and mixed with 100 *µ*l PBS containing liposomes of Example 2.1. The final concentrations of the copolymer are 15% (w/w) and 20% (w/w) containing liposomes with encapsulated FITC-BSA at a concentration of 22.2 µmol lipid/ml (13.3 mg/ml). The reaction mixtures are incubated at 37°C under mild agitation in a water bath until gelling. Thereafter, 1.7 ml PBS, pH 7.4, is added to each sample and incubation at 37°C is continued. At specified sample collection times, 0.5 ml aliquots of the supernatant are withdrawn and replaced by an identical volume of PBS, pH 7.4, to maintain release conditions. The amount of released PS-liposomes is determined by measuring encapsulated FITC-BSA via absorbance at 495 nm in the supernatant after dissolving the lipid vesicles with 1% (v/v) Triton X-100 (Cohen et al., 1991) or by fluorescense detection in suitable detection systems.

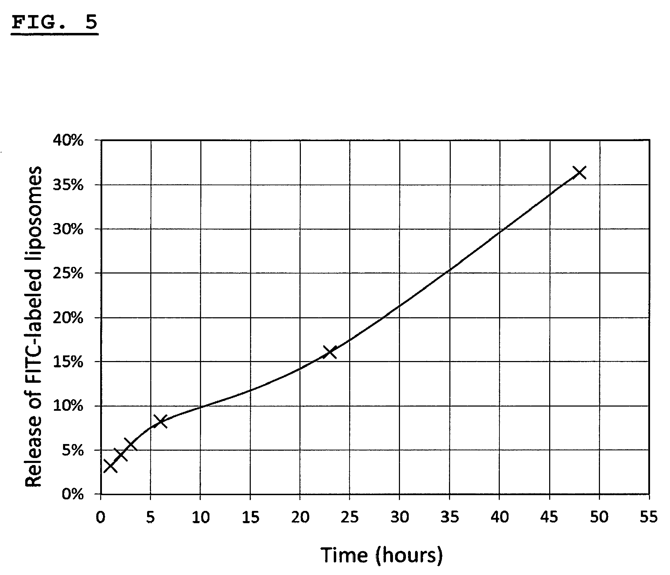

Using the experimental conditions of this example, approx. 7% of the PS-liposomes are released from the hydrogel within the first 5 hours, approx. 15% after 24 hours and approx. 35% after 48 hours (see Figure 5).

### 4. COMPOSITIONS OF PLGA-PEG-PLGA HYDROGELS AND

### PLGA PARTICLES

This example describes the synthesis and chacterization of thermogelling PLGA-PEG-PLGA hydrogels containing PLGA nanoparticles.

### 4.1. SYNTHESIS OF PLGA NANOPARTICLES CONTAINING ATP AND UTP

ATP/UTP-loaded PLGA nanoparticles are prepared according to a procedure described for the preparation of allergen-loaded tolerance-promoting immune modulators (Schöll et al., 2004). The PLGA nanospheres are prepared from Resomer RG 503H (lactide/glycolide ratio 50:50, inherent viscosity 0.32-0.44 dL/g, acid number >3 mg KOH/g; Boehringer Ingelheim, Germany) by the water-in-oil-in-water technique. PLGA is dissolved in ethyl acetate (200 mg/ml) and emulsified with 100 *µ*l water containing 10 mM ATP (551 *µ*g; MW 551.14) and 10 mM UTP (494 *µ*g; MW 494.14) by sonication for 30 s (sonifier: Bandelin electronic UW 70/HD, Germany). Then, 3 ml of 10% aqueous Pluronic solution (Pluronic F-68, Sigma) is added to the water/oil-emulsion and sonified for 15 s. Pluronic F68 is a difunctional block copolymer non-ionic surfactant that is relatively nontoxic. Thereafter, the resulting double-emulsion is poured into 50 ml of 1% aqueous Pluronic solution containing 2% isopropanol and srirred for 1 h. The residual ethyl acetate is evaporated under vacuum and the PLGA nnospheres are collected by centrifugation, washed twice in water and stored at -20°C in phosphate-buffered saline (PBS), pH 7.4, containing 1% Pluronic.

### 4.1.1. Determination of particle size

For analysis of the diameter and particle size distribution by laser diffraction (Shimadzu Laser Diffraction, Type Particle Size Analyzer SALD-1100; Shimadzu, USA), PLGA nanospheres are collected by centrifugation and suspended in an aqueous solution of 0.2% Tween 20 (Merck, Germany) to minimize agglomeration. After sonication for 1 min, the PLGA nanospheres are analysed in duplicate under continuous stirring. Using comparable conditions for the preparation of PLGA nanospheres, a mean diameter ranging between 270 and 360 nm (50% value) was determined (Schöll et al., 2004).

### 4.1.2. Encapsulation efficiency of AT and UTP

The encapsulation efficiency is determined after dissolving lyophylized PLGA nanospheres in 0.2 M NaOH until a clear solution is obtained. After neutralization with 1 M HCl, the ATP and UTP concentration is determined by absorption at 260 nm. Alternatively, the ATP concentration can be determined with a bioluminescence assay (sensitivity: pmol range) using recombinant firefly luciferase and its substrate D-luciferin (see Example 2.2). Based on an encapsulation efficiency of 30 to 50% (for comparison, the encapsulation efficiency of short CpG oligodeoxynucleotides is in the range of 30%: Joshi et al., 2013), the PLGA nanspheres contain approx. 160-280 *µ*g ATP/200 mg PLGA particles (800-1400 ng/mg) and approx. 150-250 *µ*g UTP/200 mg PLGA particles (750-1250 ng/mg).

### 4.2. SYNTHESIS OF HYDROGEL-EMBEDDED PLGA NANOPARTICLES

Different concentrations of the PLGA-PEG-PLGA copolymer of Example 1 (22.5% w/w, and 30% w/w) in water are mixed with PLGA particle suspensions in phosphate-buffered saline (PBS), pH 7.4, 1% Pluronic of Example 4.1 containing 40 mg PLGA particles/ml at a ratio of two volumes hydrogel solution to one volume of PLGA particle suspension. The final concentration of the hydrogel is 15% (w/w) or 20% (w/w).

The final concentration of ATP and UTP in 1.0 ml of the hydrogel-PLGA particle composition (13.3 mg PLGA particles/ml composition) is approx. 10.6-18.6 *µ*g/ml ATP (19.2-33.7 nmol/ml; MG 551.14) and approx. 10.0-16.6 *µ*g/ml UTP (20.2-33.6 nmol/ml; MG 494.14).

For immunotherapy, 100 *µ*l of the hydrogel-PLGA particle composition is injected subcutaneously which contains approx. 1.1-1.9 *µ*g ATP (1.9-3.4 nmol) and approx. 1.0-1.7 *µ*g UTP (2.0-3.4 nmol). Assuming a 10% release of PLGA particles (loaded with ATP and UTP) from the injected hydrogel within 6 hours (based on the in vitro release kinetics of PS-liposomes from PLGA-PEG-PLGA hydrogels), the maximal amount of releasable ATP and UTP from released PLGA particles is in the range of 200-340 pmol. Assuming a volume of approx. 1 ml in direct proximity of the injected hydrogel composition under in vivo conditions, this amount correlates to a maximal concentration of approx. 200-340 nM. However, not all of the PLGA-encapsulated ATP and UTP will be released within this period of time. Therefore, the concentrations of ATP and UTP in the proximity of subcutaneously injected hydrogel compositions are likely to be significantly lower.

### 5. RELEASE KINETICS OF HYDROGEL-EMBEDDED FIND-ME MOLECULES

This example describes the release of the find-me molecule ATP from the hydrogel of Example 1.1.

### 5.1. SYNTHESIS OF ATP-HYDROGEL COMPOSITIONS

The PLGA-PEG-PLGA triblock copolymer of Example 1.1 is dissolved at room temperature in phosphate buffered saline (PBS) at pH 7.4, containing 25% or 20% w/v polymer. Varying volumes (10 *µ*l, 20 *µ*l, 40 *µ*l, 80 *µ*l) of ATP (10 mM in PBS, pH 7.4,) are added to 200 *µ*l gel solution.

### 5.2. RELEASE KINETICS OF HYDROGEL-EMBEDDED ATP

The hydrogel composition is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

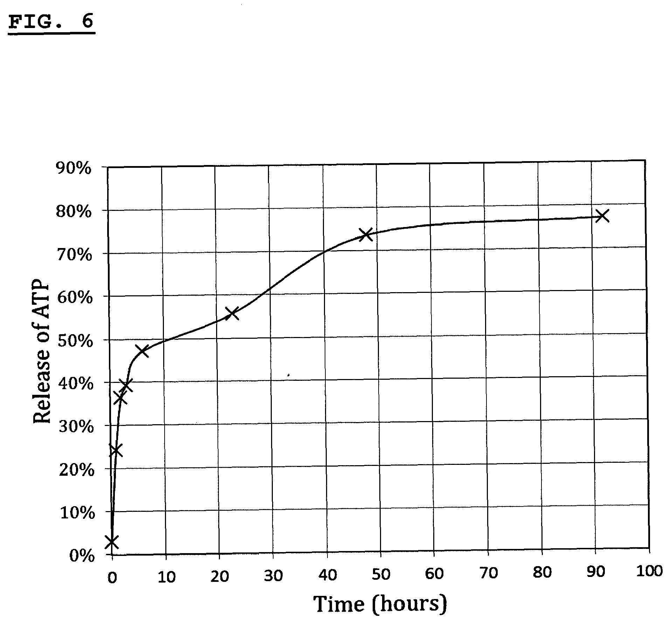

After 48 hours the samples are measured spectrometrically at 260 nm and the amount of released ATP is calculated as percentage of a reference sample containing a concentration of ATP equalling 100% release. Figure 6 shows the release kinetics of ATP from a PLGA-PEG-PLGA hydrogel under in vitro conditions.

### 6. RELEASE KINETICS OF HYDROGEL-EMBEDDED TOLERANCE-PROMOTING IMMUNE MODULATORS

This example describes the release characteristics of selected hydrogel-embedded immune-modulators suitable for generating tolerogenic antigen-presenting cells and enhancing the suppressive function of regulatory T cells.

### 6.1. RELEASE KINETICS OF HYDROGEL-EMBEDDED VITAMIN D3

This example describes the release of calcitriol (1α,25-dihydroxyvitamin D3; 1,25-(OH)₂D3) from the hydrogel of Example 1.1. Calcitriol (MW 416.65; solubility: approx. 50 mg/ml of ethanol or approx. 0.15 mg/ml in a 1:5 solution of ethanol:PBS, pH 7.2) is purchased from BioVision Inc.

The PLGA-PEG-PLGA triblock copolymer of Example 1.1. is dissolved at room temperature in 200 *µ*l PBS pH 7.4 containing different concentrations of calcitriol (10-50 µg) in a 1:5 solution of ethanol:PBS, pH 7.2, to make a 20% w/w or 25% w/w solution. Then the formulation is placed in a 2 ml vial, incubated at 37°C for 2 min until gelling, and 1.8 ml of PBS pH 7.4 is added. The vial is incubated at 37°C. At specified sample collection times, a sample is withdrawn and replaced by an identical volume of PBS pH 7.4 to maintain release conditions.

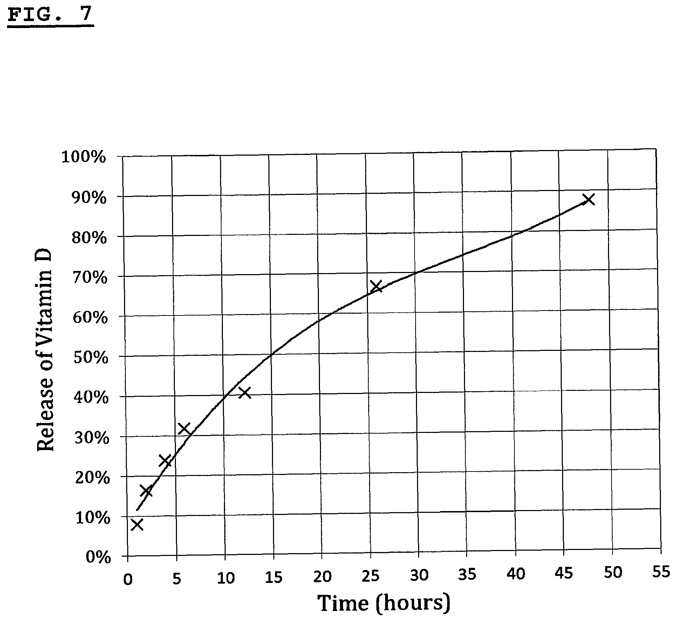

The amount of released calcitriol is determined by solid-phase ELISA (Life Science Market). Figure 7 shows the release kinetics of calcitriol from a PLGA-PEG-PLGA hydrogel under in vitro conditions.

### 6.2. RELEASE KINETICS OF HYDROGEL-EMBEDDED DEXAMETHASONE

This example describes the release of dexamethasone sodium phosphate (DexP) from the hydrogel of Example 1.1.

The PLGA-PEG-PLGA triblock copolymer of Example 1.1 is dissolved at room temperature in phosphate buffered saline (PBS) at pH 7.4, containing 25% or 20% w/v polymer. A 20 mM stock solution of DexP in PBS (10.3 mg/ml; MW 516.4; Sigma-Aldrich) is diluted 1:40 in PBS (final concentration: 0.5 mM), and 20 µl of the diluted stock solution is combined with 160 µl of 25% gel solution and 20 µl of 10x PBS (final concentration of DexP: 0.05 mM).

The mixture of is incubated for 2 minutes at 37°C to induce gelling and overlayed with 200 µl of 1x PBS. At frequent time points the supernatant is removed by pipetting and stored at 4°C. The removed supernatant is replaced by fresh 200 µl of 1x PBS. Controls of the same concentration of DexP in 1x PBS without gel are incubated and sampled in parallel.

Released DexP is determined with a spectrophotometric method (Singh and Verma, 2008). The method involves oxidation of the corticosteroid by iron (III) and subsequent complexation of iron (II) with potassium hexacynoferate (III), forming a bluish green coloured complex with maximum absorbance at 780 nm (Beer's law range: 10-50 *µ*g/ml; molar absorptivity (M⁻¹cm⁻¹): for DexP 0.55 x 10⁴).

Approx. 30-50% of hydrogel-embedded DexP is released within the first 6 hours, followed by a continuously decreasing release per hour, until 80-90% of embedded DexP is released after 2 days.

### 6.3. RELEASE KINETICS OF HYDROGEL-EMBEDDED SALICYLATE

This example describes the release of sodium salicylate (SA) from the hydrogel of Example 1.1.

The PLGA-PEG-PLGA triblock copolymer of Example 1.1 is dissolved at room temperature in phosphate buffered saline (PBS) at pH 7.4, containing 25% or 20% w/v polymer. Varying volumes (10 *µ*l, 20 *µ*l, 40 *µ*l, 80 *µ*l) of 10 mM sodium salicylate (SA) in PBS, pH 7.4, are added to 200 *µ*l gel solution. The solubility of SA in water is 2g/liter at 20°C (14.5 mM). Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

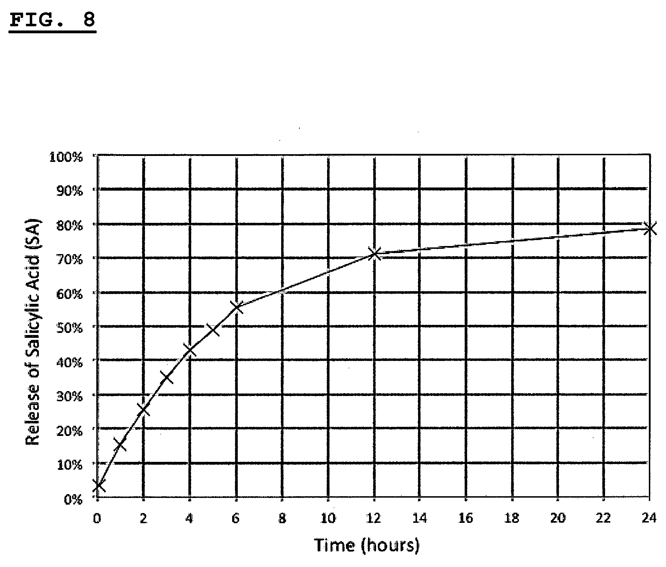

The amount of released SA is determined by derivatization with Fe(III) and quantification of the violet coloured tetraaquosalicylatroiron (III) complex at 530 nm (www.jenway.com; Application note A09-009A; The quantitative determination of the aspirin content of tablets using UV and visible wavelength spectroscopy). Figure 8 shows the release kinetics of sodium salicylate (SA) from a PLGA-PEG-PLGA hydrogel under in vitro conditions.

### 6.4. RELEASE KINETICS OF HYDROGEL-EMBEDDED IL-4/IL-13 MUTEIN

This example describes the release of murine IL-4 antagonist QY (Q116D/Y119D) from the hydrogel of Example 1.1.
6.4.1. Coning and expression of the murine IL-4 antagonist QY. First, the gene sequence murine IL-4 antagonist QY (Q116D/Y119D) is optimized for expression in mammalian CHO-cells and subcloned into a pIRES-DHFR expression vector. This GMP-compatible system offers the advantage of gene amplification. The gene of interest, the IL-4 mutein, is linked to a vector-coded dihydrofolate reductase by an IRES-site, resulting in a coupled co-expression of both proteins. Using a DHFR-negative CHO cell line and the addition of increasing concentrations of methotrexate (up to 1250 nM), a selection towards higher expression cell clones can be achieved. For affinity purification of the murine IL-4 mutein, a 10x His-tag is fused to the N-terminus.
   The final expression vector is transfected into CHO(-DHFR) cells and selected with increasing concentrations of methotrexate for improved expression and protein yield. After the selection, cell cultivation for expression is performed in a CO₂-controlled incubator in 1L roller flasks.
6.4.2. Release of murine IL-4 antagonist QY from PLGA-PEG-PLGA hydrogels. The PLGA-PEG-PLGA triblock copolymer of Example 1.1. is dissolved at room temperature in PBS pH 7.4 containing different concentrations of the murine IL-4 antagonist QY (0.5 mg/ml up to 1.0 mg/ml) to make a 20% w/w or 25% w/w solution. Then 200 *µ*l of the formulation is placed in a 2 ml vial, incubated at 37°C for 2 min until gelling, and 1.8 ml of PBS pH 7.4 is added. The vial is incubated at 37°C. At specified sample collection times, a sample is withdrawn and replaced by an identical volume of PBS pH 7.4 to maintain release conditions.
6.4.3. Analysis of released IL-4 antagonist QY. The amount of released murine IL-4 antagonist QY is determined by bicinchoninc acid (BCA) assay using BCA™ Protein Assay Kit (Pierce, USA) and by an ELISA-type assay as described above. The structural integrity of released murine IL-4 antagonist QY is determined by a cellular binding assay. The functionality of released murine IL-4 antagonist QY is determined by different in vitro assays. The first assay uses mouse spleen B cells and the determination of their proliferation behavior and cytokine secretion pattern after stimulation with IL-4 in the absence or presence of different concentrations of the mutein. The second assay uses the mouse T cell line CTLL-2 which can be used for the quantification of mouse as well as of human IL-4 activity and thereby of the blocking effect of the mutein. The blocking activity of the mutein will be determined by a proliferation assay, the quantification of the activation of IL-4 regulated genes by RT-PCR, as well as by quantification of IL-4-regulated marker proteins by FACS analyses. For all assays an anti-IL-4 blocking antibody serves as positive control.

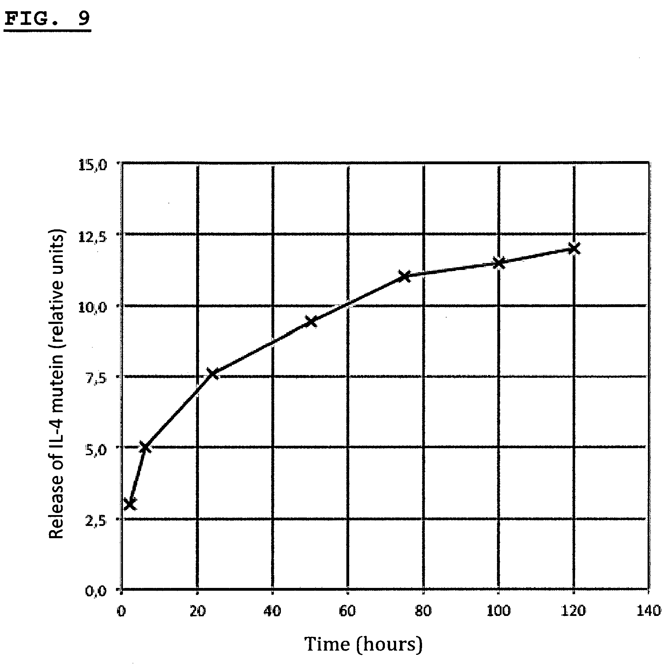

Figure 9 shows the release kinetics of murine IL-4 antagonist QY from a PLGA-PEG-PLGA hydrogel under in vitro conditions.

### 7. IMMUNOTHERAPY STUDIES WITH HYDROGEL-BASED

### COMPOSITIONS

### 7.1. IMMUNOTHERAPY WITH HYDROGEL-EMBEDDED OVALBUMIN (OVA)

This example describes the application of subcutaneously injected OVA-containing PLGA-PEG-PLGA hydrogels for immunotherapy of OVA-sensitized mice.

### 7.1.1. Animals and materials

Female wild-type C57BL/6 mice 6 to 10 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgG1, and goat antisera against murine IgG2a, IgG2b, and IgG3 from BD Pharmigen.

### 7.1.2. Preparation of hydrogel compositions

This example describes the synthesis of PLGA-PEG-PLGA hydrogel compositions comprising hydrogel-embedded OVA.

The PLGA-PEG-PLGA triblock copolymer of Example 1.1., dried under vacuum at room temperature until constant weight, is dissolved at 4°C in PBS at a concentration of 30% w/v polymer, and then mixed with OVA in PBS (either 3.0 mg/ml or 1.5 mg/ml) at a ratio of 2 volumes hydrogel and 1 volume OVA in PBS. The final concentration of the hydrogel is 20% w/v, containing OVA at a concentration of 1.0 mg/ml or 0.5 mg/ml.

### 7.1.3. Allergic sensitization procedure

Mice are immunized four times by i.p. injection of 200 µl alum-adsorbed OVA (30 µg OVA adsorbed to 2 mg alum). The second i.p. injection is performed 7 days after the first injection, the third injection 14 days after the first injection, and the fourth injection 28 days after the first injection.

### 7.1.4. Immunotherapy of OVA-sensitized mice with hydrogel-embedded OVA.

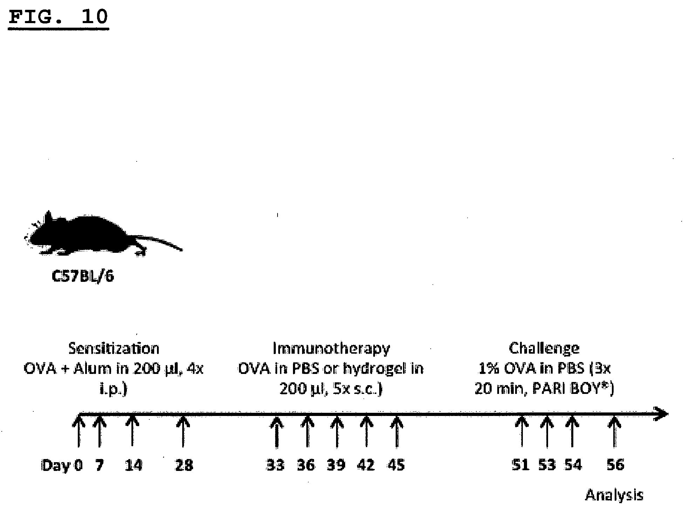

Figure 10 shows the experimental setting for the induction of tolerance in OVA-sensitized mice with PLGA-PEG-PLGA hydrogel-embedded OVA.

Four groups of wild-type C57BL/6 mice (each group: 15 mice) are used for this experiment. Group 1: 5x sc. injections of 200 *µ*l hydrogel composition containing 200 *µ*g OVA, group 2: 5x sc. injections of 200 *µ*l hydrogel composition containing 100 *µ*g OVA, group 3: 5x sc. injections of 200 *µ*l PBS containing 100 *µ*g OVA, and group 4: no treatment.

The five subcutaneous (s.c.) injections (in 4x 50 µl portions in the rear flank) are performed in three-day intervals, starting 5 days after the last i.p. sensitization (day 33) and ending at day 45.

### 7.1.5. Challenge procedure

One week after the last immunotherapeutic treatment (day 52), mice are exposed on three consecutive days to a nebulized 1% OVA Grade V solution for 20 min in a challenge chamber. Two days after the last challenge (day 54) mice are euthanized and subjected to various analyses.

### 7.1.6. Analysis of serum levels of OVA-specific antibodies

Mice are bled at day 0 before immunization with OVA, 4 days after immunization with OVA (1 day before starting immunotherapy), and two days after the last challenge. Analyses include the determination of serum levels of OVA-specific antibodies and cytokines.

Murine anti-OVA IgE and IgG subclasses are determined by ELISA. Plates are coated with 10 µg OVA in 100 µl 0.1 M NaHCO₃ for 6 h at 37°C, followed by blocking with 200 µl 3% BSA in PBS, pH 7.4, for 2 h at 37°C. After washing, 100 µl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgG1, IgG2a, IgG2b, IgG3). Analysis is performed at 405 nm in a microplate autoreader.

### 7.1.7. Bronchoalveolar lavage (BAL) analysis

The lungs of euthanized animals are lavaged in situ with five 1 ml aliquots of sterile saline, with 3 to 4 ml BAL fluid recovered from each animal. The BAL is centrifuged and resulting cell pellets are suspended in 250 µl saline. BAL protein concentrations are measured in the supernatants by the bicinchoninc acid (BCA) assay using the BCA™ Protein Assay Kit (Pierce, USA) and bovine serum albumin as standard. Total leukocytes are counted in a hemocytometer using trypan blue dye exclusion as a measure of viability. Cytospin slides are made and stained with May-Grunwald/Giemsa to determine the BAL cell differential. The remaining cells are analyzed by fluorescence flow cytometry. For these analyses, BAL samples are washed in phosphate-buffered saline (PBS) containing 0.2% bovine serum albumin and 0.1% NaN₃. Aliquots containing 10⁴ to 10⁵ cells are incubated with 100 µl of appropriately diluted antibodies for 30 min at 4°C. After staining, the cells are washed twice with the above PBS solution, and relative fluorescence intensities are determined on a 4-decade log scale by flow cytometric analysis using a FACScan (Becton Dickinson).

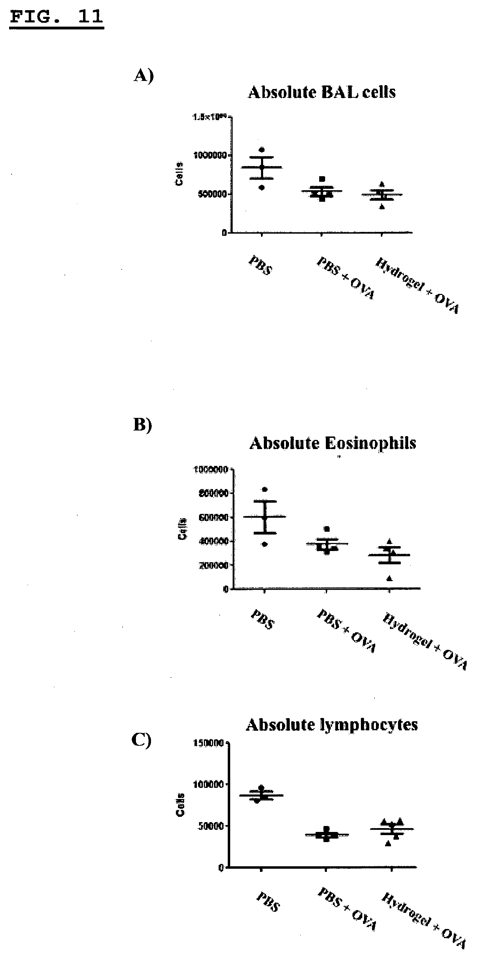

Figure 11 shows that subcutaneously injected hydrogel-embedded OVA is as efficient as s.c. injected OVA in PBS in decreasing the number of BAL cells including lymphocytes and eosinophils.

### 7.1.8. Summary

The data of this experiment demonstrate that PLGA-PEG-PLGA hydrogels are suitable depot matrices for the induction of tolerance via allergen- or autoantigen-specific immunotherapy.

### 7.2. THERAPY WITH HYDROGEL-EMBEDDED PS-LIPOSOMES PRIOR TO IMMUNIZATION WITH OVA IN CFA

This example describes the application of subcutaneously (s.c.) injected PS-liposome-containing PLGA-PEG-PLGA hydrogels for modulation of the immune response of mice to subsequently s.c. injected ovalbumin in Complete Freund's Adjuvant (CFA).

### 7.2.1. Animals and materials.

Female wild-type BALB/c mice 6 to 10 weeks of age (body weight approx. 20 g) are purchased from Charles River (Sulzfeld, Germany). The animals are kept under specific pathogen-free conditions and maintained on OVA-free diets. Ovalbumin (OVA) grade V is purchased from Sigma, rat monoclonal antibodies against murine IgE and IgG1, and goat antisera against murine IgG2a, IgG2b, and IgG3 from BD Pharmigen.

### 7.2.2. Preparation of PS-liposomes.

This example describes the synthesis of unilamellar PS-liposomes from a lipid mixture of bovine brain phosphatidylserine (PS; Avanti Polar Lipids), egg phosphatidylcholine (PC; Avanti Polar Lipids), and cholesterol (CH; Avanti Polar Lipds) at a ratio of 30:30:40 PS to PC to CH according to Hoffmann et al. (2005).

A chloroform/methanol (2:1, v/v) solution containing 30 µmol PS (approx. 24.4 mg), 30 µmol PC (approx. 22.8 mg) and 40 µmol CH (approx. 15.5 mg) is placed in a conical flask and dried by rotary evaporation to prepare a thin lipd film. Thereafter, the flask is placed in a desiccator for at least one hour to completely remove the solvent. Then, 1.5 ml of phosphate-buffered saline (PBS) is added and multilamellar vesicles are generated by intense vortex dispersion. For the preparation of unilamellar vesicles, the multilamellar preparation is extruded 10 times through a 1 µm pore polycarbonate membrane (Nucleopore, USA). PS-liposomes with a particle size of approx. 1 µm are suitable for efficient uptake by macrophages (Harel-Adar et al., 2011). The final liposomal suspension contains approx. 66.6 µmol (39.2 mg) of lipd/1.0 ml. Unilamellar PS-liposomes prepared by this procedure have been shown to disperse uniformly in physiological medium due to repulsion forces (Harel-Adar et al., 2011).

The degree of PS exposure on liposomes is assessed by binding of FITC-annexin V to surface-exposed PS and analysis by FACS.

### 7.2.3. Preparation of control liposomes.

This example describes the synthesis of unilamellar control liposomes from a lipid mixture of egg phosphatidylcholine (PC; Avanti Polar Lipids), and cholesterol (CH; Avanti Polar Lipds) at a ratio of 60:40 PC to CH according to Hoffmann et al. (2005). The synthesis is performed as described in 7.2.2.

### 7.2.4. Preparation of liposome-hydrogel compositions.

This example describes the synthesis of different compositions comprising hydrogel-embedded unilamellar PS-liposomes or unilamellar control liposomes.

The PLGA-PEG-PLGA triblock copolymer of Example 1.1., dried under vacuum at room temperature until constant weight, is dissolved at 4°C in PBS at a concentration of 30% w/v polymer, and then mixed with PS-liposomes, or control liposomes, or PBS at a ratio of 2 volumes hydrogel and 1 volume liposomes or PBS. The final concentration of the hydrogel is 20% w/v, containing liposomes at a concentration of 22.2 µmol (approx. 13 mg) of total lipd/1.0 ml.

Using the experimental conditions of Example 3.3, approx. 7% of the PS-liposomes are released from the hydrogel within the first 5 hours, approx. 15% after 24 hours and approx. 35% after 48 hours (see Figure 5). Accordingly, upon subcutaneous injection of 200 µl of the liposome-hydrogel composition containing approx. 2.6 mg of total lipid, approx. 0.4 mg of PS-liposomes or control liposomes are released within the first 24 h. This is comparable to the experimental conditions of the study of Hoffmann et al. (2005) in which PS-liposomes or control liposomes were subcutaneously injected into mice at a concentration of 0.5 mg total lipid.

### 7.2.5. Treatment of mice with hydrogel-embedded PS-liposomes prior to immunization with OVA in CFA.

Four groups of wild-type BAL/c mice (each group: 15 mice) are used for this experiment. Group 1: treatment with hydrogel-embedded PS-liposomes prior to immunization, group 2: treatment with hydrogel-embedded control liposomes prior to immunization, group 3: treatment with hydrogel prior to immunization, and group 4: no treatment.

BALB/c mice are injected 200 µl of the liposome-hydrogel compositions (or hydrogel without liposomes') subcutaneously (s.c.) in the rear flank (in four 50 µl portions). After 24 hours, 150 µl of an emulsion containing 50 µg ovalbumin in CFA are injected s.c. in the same region.

In a similar study (Hoffmann et al., 2005), PS-containing liposomes comprising a 30:30:40 molar ratio of PS to PC to cholesterol (or control liposomes comprising a 60:40 molar ratio of PC to cholesterol), were first injected s.c. in one flank of 6-8 weeks old BALB/c mice (100 *µ*l, corresponding to 0.5 mg of total lipid), followed after one hour by another s.c. injection of 150 *µ*l of an emulsion containing 50 *µ*g of OVA in CFA in the same region.

### 7.2.6. Determination of tissue mass.

Seven days after immunization, 5 mice of each group are euthanized, spleens and lymph nodes (including superficial inguinal, axillary and lateral axillary nodes) are removed and dissected free from surrounding fat, followed by determination of tissue mass.

As observed in the study of Hoffmann et al. (2005), immunization with OVA in CFA results in a large increase in both spleen and draining lymph node mass. Mice in which PS-liposomes are injected first, have only minimal reduction in spleen mass, but significantly reduced lymph node mass.

### 7.2.7. Determination of serum IgG levels.

Nine days after immunization, another 5 mice of each group are euthanized and whole blood is collected by cardiac puncture. The whole blood is allowed to clot at room temperature for 1 h and centrifuged for 15 min. The serum supernatant is used for ELISA analyses.

As observed in the study of Hoffmann et al. (2005), mice in which PS-liposomes are injected first have lower serum levels of OVA-specific IgG antibodies in comparison to controls.

### 7.2.8. Stimulation of spleen and lymph node cells.

Ten days after immunization, another 5 mice of each group are euthanized and spleens and lymph nodes are removed. Thereafter, the tissues are homogenized in RPMI 1640 and passed through a 40 *µ*m cell strainer. The obtained cell suspensions are the washed once with RPMI 1640. One portion of the cells is resuspended in serum-free medium, incubated for 24 h in the absence or presence of ovalbumin (final concentration of 100 *µ*g/ml), and then assayed for IL-2, IL-4, IL-10, TNF-β and IFN-γ in the supernatants by ELISA.

Another portion of the cells is resuspended in RPMI 1640 containing 10% FCS, 1% penicillin/streptomycin/L-glutamine, 1 mM sodium pyruvate, 0.2% sodium bicarbonate, 0.1 mM essential and nonessential amino acids, 14.4 mM 2-mercaptoethanol, and 2.92 mg/ml L-glutamine. Aliquots of 1x 10⁶ and 5x 10⁵ cells are placed in each well of 96-well plates and OVA is added to each well at a final concentration of 100 *µ*g/ml. After incubation of the cells at 37°C and 5% CO₂ for 3 days, IL-2, IL-4, IL-10, TGF-β and IFN-γ are determined in the supernatants by ELISA.

As observed in the study of Hoffmann et al. (2005), mice in which PS-liposomes are injected first show decreased levels of IL-2 and IFN-γ in the supernatants, even after stimulation with OVA. In cell cultures obtained from these mice, IL-4 levels are low and IL-10 is not detectable, whereas levels of TGF-β are signicantly increased.

### 7.2.9. Summary.

Subcutaneously administered PS-liposomes specifically inhibit responses to antigens. As demonstrated by Hoffmann et al. (2005), numbers of total leukocytes and antigen-specific CD4+ T cells are reduced as well as the level of antigen-specific IgG in blood. There is also a decrease of draining lymph node tissue mass and the size of germinal centers in spleen and lymph nodes. Apparently, dendritic cells (DCs) are not involved in the tolerance-promoting effects of PS-liposomes, since PS-liposomes are not capable of inhibiting phenotypical and functional maturation of DCs in response to proinflammatory stimuli. Most likely, PS-liposomes inhibit immune responses by acting on other cell types at the site of immunization including macrophages, fibroblasts, epithelial cells and endothelial cells. Since the subcutaneous administration of a mouse monoclonal antibody against TGF-β at the time of liposome injection and again at the time of immunization with an OVA-CFA emulsion is able to reverse the inhibitory effects of PS-liposomes, TGF-β apparently plays an important role for the tolerance-promoting effects of PS-liposomes.

### 7.3. IMMUNOTHERAPY WITH HYDROGEL-EMBEDDED PLGA MICROSPHERES

This example describes the application of subcutaneously (s.c.) injected PLGA microsphere-containing PLGA-PEG-PLGA hydrogels for modulation of the immune response of mice to subsequently s.c. injected bee venom phospholipase A2 (PLA2).

### 7.3.1. Animals and materials.

Female wild-type BALB/c mice 6 to 10 weeks of age (body weight approx. 20 g) are purchased from Charles River (Sulzfeld, Germany). The animals are kept under specific pathogen-free conditions. Poly(D,L-lactide-co-glycolide microspheres (PLGA microsphere, average diameter 3.5 *µ*m; ratio lactide: glycolide 50:50; molecular weight 30,000) are purchased from Phosphorex (USA), native bee venom phospholipase A2 from Latoxan (France), rat monoclonal antibodies against murine IgE and IgG1, and goat antisera against murine IgG2a, IgG2b, and IgG3 from BD Pharmigen, and Imject Alum from Pierce/KMF.

### 7.3.2. Preparation Hydrogel-PLGA microsphere compositions.

This example describes the synthesis of compositions comprising hydrogel-embedded PLGA microspheres.

The PLGA-PEG-PLGA triblock copolymer of Example 1.1., dried under vacuum at room temperature until constant weight, is dissolved at 4°C in PBS at a concentration of 30% w/v polymer, and two volumes of hydrogel are mixed with one volume of PLGA microspheres in 0.3% methocel (water-soluble methylcellulose and hydroxypropyl methylcellulose polymer) in nanopure water (150 mg PLGA microsperes/ml). The final concentration of the hydrogel is 20% w/v, containing 50 mg PLGA microsperes and 0.1% methocel per ml hydrogel composition.

Assuming comparable release kinetics of PLGA microspheres from PLGA-PEG-PLGA hydrogels as observed for hydrogel-embedded PS-liposomes, approx. 5-10% of the PLGA microspheres are released from the hydrogel within the first 6 hours, approx. 15% after 24 hours and approx. 35% after 48 hours. Accordingly, upon subcutaneous injection of 200 µl of the liposome-hydrogel composition containing approx. 10 mg of PLGA microspheres, approx. 1.5 mg of PLGA microspheres are released within the first 24 h and approx. 3 to 4 mg of PLGA microspheres with 48 hours. This is comparable to the experimental conditions of the study of Jilek et al. (2004) in which 5 mg plain PLGA microspheres (size range: 1-10 *µ*m) in 100 *µ*l 0.25% methocel in nanopure water was subcutaneously injected into mice once a week for 3 consecutive weeks.

7.3.3. Treatment of mice with hydrogel-embedded plain PLGA microspheres prior to immunization with bee venom phospholipase A2 (PLA2) in alum.

Four groups of wild-type BAL/c mice (each group: 15 mice) are used for this experiment. Group 1: treatment with PLGA-PEG-PLGA hydrogel containing PLGA microspheres and 0.1% methocel prior to immunization with PLA2, group 2: treatment with PLGA-PEG-PLGA hydrogel containing 0.1% methocel prior to immunization with PLA2, group 3: treatment with 0.1% methocel prior to immunization with PLA2, and group 4: no treatment. BALB/c mice are injected subcutaneously (s.c.) in the rear flank three times in two-week intervals a) 200 µl of the PLGA-PEG-PLGA hydrogel composition containing 10 mg of PLGA microspheres (average diameter 3.5 *µ*m; ratio lactide: glycolide 50:50; molecular weight 30,000), and 0.1% methocel (group 1), or b) 200 µl of the PLGA-PEG-PLGA hydrogel composition containing 0.1% methocel (group 2), or c) 200 µl 0.1% methocel (group 3) (injections are performed in four 50 µl portions).

Three weeks after the last s.c. injection of hydrogel compositions, mice are sensitized subcutaneously in the rear flank with 6 doses of 100 ng of native bee venom phospholipase A2 adsorbed to 1 mg alum given at two-week intervals (this has been shown by Seeger et al., (1998) to favour the induction of anti-PLA2 IgE responses).

Four weeks after the last sensitization with PLA2/alum, mice are boosted subcutaneously with 100 ng of native bee venom PLA2 adsorbed to 1 mg alum. Three days later, mice are challenged intraperitoneally with 30 *µ*g of native bee venom PLA2 in PBS, a dose that kills 100% of mice within 30 min in the absence of treatment (von Garnier et al., 2000). After the challenge, mice are killed.

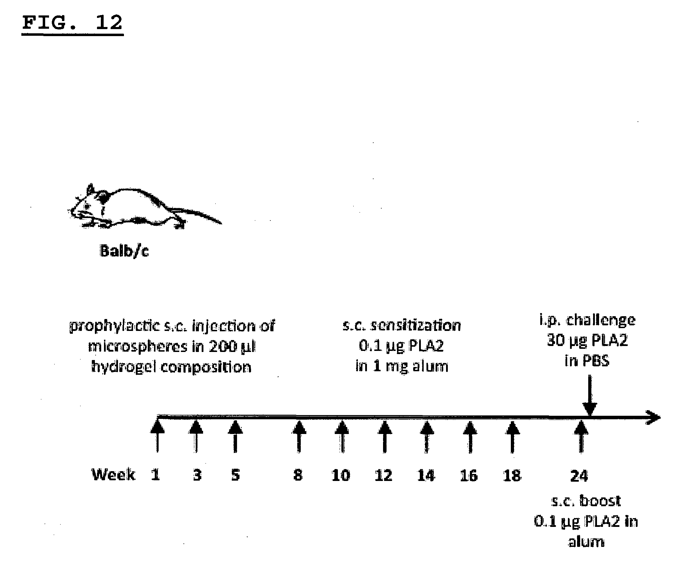

Figure 12 shows the experimental setting for the induction of tolerance towards PLA2 in mice with PLGA-PEG-PLGA hydrogel-embedded PLGA microspheres.

### 7.3.4. Measurement of serum antibody titers.

Antibody titers (total IgG, IgG1, IgG2a, IgE) are determined by commercially available ELISA systems. For the determination of PLA2-specific antibody titers, immunoplates are coated with 50 *µ*l of 5 *µ*g/ml PLA2 (Latoxan) in 50 mM carbonate-bicarbonate, pH 9.6, followed by blocking with 200 *µ*l of PBS-0.05% Tween 20-1% BSA for 1 h at 37°C. After three washes with PBS-0.05% Tween 20, serial dilution of mouse sera are tested according to standard procedures.

### 7.3.5. Detoxification of PLA2 for T cell proliferation assays.

Since native bee venom PLA2 PLA2 is toxic to most cells in culture, native PLA2 is inactivated by reduction-alkylation for these experiments. PLA2 in PBS is incubated overnight at 37°C in the presence of dithiothreitol (100-fold molar excess), followed by alkylation with a 100-fold molar excess of N-ethylmaleimide as described by Jilek et al. (2001), and subsequent purification by gel chromatography on Sephadex G25.

### 7.3.6. Stimulation of spleen and lymph node cells.

Three days after intraperitoneal challenge with PLA2, mice are killed and spleens and lymph nodes are removed. Thereafter, the tissues are homogenized in RPMI 1640 and passed through a 40 *µ*m cell strainer. The obtained cell suspensions are the washed once with RPMI 1640.

Cytokine release assays. One portion of the cells is resuspended in serum-free medium (1x 10⁶ and 5x 10⁵ cells are placed in each well of 96-well plates), incubated for 48 h in the absence or presence of alkylated PLA2 (final concentration 10 *µ*g/ml), and then assayed for IL-2, IL-4, IL-10, TNF-β and IFN-γ in the supernatants by commercially available ELISA assays. For control of cell viabilitry, cells are also incubated in the presence of 2 *µ*g/ml concanavalin A.

Proliferation assays. Another portion of the cells is resuspended in RPMI 1640 containing 10% FCS, 1% penicillin/streptomycin/L-glutamine, 1 mM sodium pyruvate, 0.2% sodium bicarbonate, 0.1 mM essential and nonessential amino acids, 14.4 mM 2-mercaptoethanol, and 2.92 mg/ml L-glutamine. Aliquots of 1.5-2 x 10⁵ cells in 200 *µ*l medium are placed in each well of 96-well plates and alkylated PLA2 is added to each well at a final concentration of 10 *µ*g/ml. Concanavalin A (2.5 *µ*g/ml) is used as positive control and OVA (10 *µ*g/ml) as negative control. After incubation of the cells at 37°C and 5% CO₂ for 4 days, cells are pulsed overnight with [³H] thymidine (1 *µ*Ci/well) and then analyzed for nuclear incorporation of radioactivity using a scintillation beta counter. Proliferation responses are calculated as stimulation index dividing geometric mean antigen-stimulated cpm by background cpm.

### 7.3.7. Summary.

Subcutaneously administered PLGA microspheres protect mice against anaphylaxixs induced by PLA2 challenge. As demonstrated by Jilek et al. (2004), microspheres alone protect most mice against anaphylaxis through transient induction of the TH1-type antibody IgG2a, and on production of IL-10. The recall challenge with PLA2 is associated with a combined expression of both IL-4 and IFN-γ, together with sustained expression of IL-10 that can explain the protective effect against anaphylaxis. Apparently, microspheres are somehow sufficient in a prophylactic therapeutic setting to instruct the immune system not to overreact at the time the antigen is given during the sensitization procedure. The TH1 bias at the level of the immunoglobulin in the initial period of the treatment is sustained transiently, followed by a mixed TH1/TH2 response supported by a similar expression of IL-4 and IFN-γ underlining the plasticity of the modulation in vivo.

### REFERENCES

Abe M., et al., J. Immunol. 167: 4651-4660; 2001.
Abramson M.J., et al., Cochrane Database Syst. Rev. CD001186; 2003.
Adler A., et al., Comb. Chem. High Throughput Screen 11: 16-23; 2008.
Albani S., et al., Nat. Med. 1 : 448-452 ; 1995.
Alexis F., Polymer Int. 54: 36-46; 2005.
Alhalaweh A., et al., Eur. J. Pharm. Sci. 38: 206-214; 2009.
Al-Jaderi Z., et al., Toxins 5 : 1932-1947 ; 2013.
Altamimi S., et al. Pediatr. Emerg. Care 22: 786-793; 2006.
Ames R.S., et al., J. Immunol. 166: 6341-6348; 2001.
Anderson P.H., et al., Mol. Cell. Endocrinol. 285: 1-9; 2008.
Anderson R., et al., Arthr. Res. Ther. 12 : R147 ; 2010.
Andreau K., et al., Immunopharmacology 40 : 67-76 ; 1998.
Anzilotti C, et al., Autoimmun Rev 9:158-160; 2010.
Andjelkovic Z., et al. Clin. Exp. Rheumatol. 17: 453-456; 1999.
Apetoh L. et al., Nat. Immunol. 11: 854-861; 2010.
Arumugam T.V., et al., Kidney Int. 63:134-142; 2003.
Arumugam T.V., et al., J. Hepatol. 40: 934-941; 2004.
Assier E., et al., Ann. Rheum. Dis. 69:A46-A47; 2010
Baelder R., et al., J. Immunol. 174: 783-789; 2005.
Bal S.M., et al., Vaccine 29: 1045-1052; 2011.
Ballantyne, S.J., et al., J. Allergy Clin. Immunol. 120: 1324-1331; 2007.
Bao L., et al., J. Immunol. 175: 1947-1955; 2005a.
Bao L., et al., Eur. J. Immunol. 35: 2496-2506; 2005b.
Barlow J.L., McKenzie A.N., J. Mol. Biol., vol. 35: 178-182; 2009.
Barnes P.J., Allergy 56: 928-936; 2001.
Basomba A., et al. J. Allergy Clin. Immunol. 109: 943-948; 2002.
Bautsch W., et al., J. Immunol. 165: 5401-5405; 2000.
Beer T.M., et al. Clin. Cancer Res. 11: 7794-7799; 2005.
Belogurov Jr. A.A., et al. FASEB J. 27: 222-231; 2013.
Bereshchenko O., et al., Cell Rep.7: 464-475; 2014.
Biesecker G., et al., Immunopharmacol. 42: 219-320; 1999.
Bikle D.D., Curr. Osteoporos. Rep. 7: 58-63; 2009.
Blagg J., et al., Bioorg. Med. Chem. Lett. 18: 5601-5604; 2008.
Blass S, et al., Arthritis Rheum 44:761-771.; 2001.
Bonté F., Juliano R.L., Chem. Phys. Lipids 40 : 359-372 ; 1986.
Bouillon R., et al., Endocrinol. Rev. 29: 726-775; 2008.
Branisteanu D.D., et al. J. Neuroimmunol. 61: 151-160; 1995.
Brodbeck R.M., et al., J. Pharmacol. Exp. Ther.327 : 898-909 ; 2008.
Bullimore A.D., et al., XXIV World Allergy Congress, Seoul, abstract 3127 ; 2015.
Burton B.R., et al. Nature Communications 5, article 4741; 2014.
Cantorna M.T., et al. J. Immunol. 160: 5314-5319; 1998a.
Cantorna M.T., et al. J. Nutr. 128: 68-72; 1998b.
Capini C., et al. J. Immunol. 182: 3556-3565; 2009.
Carrol M.C., Nat. Immunol. 5: 981-986 ; 2004.
Caspi R.R., et al., J. Immunol. 140: 1490-1495; 1988.
Catley M.C., et al., Pharmacol. Therap. 132: 333-351; 2011.
Chambers E.S., Hawrylowicz C.M., Curr. Allergy Asthma Rep. 11: 29-36; 2011.
Champion J.A., Mitragotri S., Proc. Natl. Acad. Sci. USA 103: 4930-4944; 2006.
Changelian P.S., et al., Science 302: 875-878; 2003.
Chen A., et al., J. Immunol. 193: 35-39; 2014.
Chen L., et al. Osteoarthr. Cartilage 19: 711-718; 2011.
Chen X., et al., J. Immunol. 173 : 2985-2994 ; 2004.
Chen X., et al., Eur. J. Immunol. 34: 859-869; 2004b.
Chen X., et al., J. Immunl. 179: 154-161; 2007.
Chen X., Oppenheim J.J., In: TNF pathophysiology. Molecular and cellular mechanisms. Curr. Dir. Autoimmun., pp. 119-134, Karger, Basel; 2010.
Chen Y., et al., J. Clin. Chem. 116: 1317-1326; 2006.
Chemnitz, J.M., et al., J. Immunol. 173 : 945-954 ; 2004.
Chiu H.W., et al., Nanoscale 7: 736-746; 2014.
Chorny A., et al., Proc. Natl. Acad. Sci. USA 102: 13562-13567; 2005.
Cianferoni A., et al., Blood 97: 1742-1749; 2001.
Cirunay J.J.N., et al. J. Chromatogr. Sci. 36: 417-421; 1998.
Clare-Salzler M.J., et al., J. Clin. Invest. 90: 741-748; 1992.
Corrigan C.J., et al., J. Allergy Clin. Immunol. 128: 116-124; 2011.
Cutolo M., Ther. Adv. Musculoskel. Dis. 5: 3-11; 2013.
Danhier F., et al., J. Control. Release 161: 505-522; 2012.
Deeg C.A., et al., Clin. Develop. Immunol. 2007 ; Article ID 39245.
Denonne F., et al., Bioorg. Med. Chem. Lett. 17: 3258-3261; 2007a.
Denonne F., et al., Bioorg. Med. Chem. Lett. 17: 3262-3265; 2007b.
De Souza Reboucas J., et al., J. Biomed. Biotech., article ID 474605; 2012.
Dong Z., et al., J. Biol. Chem. 272: 9962-9970; 1997.
Drouin S.M., et al., J. Immunol. 169: 59-26-5933; 2002.
Drouin S.M., et al., Am. J. Respir. Crit. Care Med. 173: 852- 857; 2006.
Duan L., et al., Clin. Develop. Immunology. Article ID 924363; 2013
Elliott, M.R., et al., Nature 461 : 282-286 ; 2009. Eloy J.O., et al., Colloids Surfaces B : Biointerfaces 123 : 345-363 ; 2014.
Fadok V.A., et al., J. Immunol. 148 : 2207-2216 ; 1992.
Fadok V.A., et al., J. Biol. Chem. 276 : 1071-1077 ; 2001.
Fakih M.G., et al. Clin. Cancer Res. 13: 1216-1223; 2007.
Fang C., et al., Blood 114: 1005-1015; 2009.
Farahani R., et al., Adv Biomed Res. 2014; 3: 127. doi: 10.4103/2277-9175.133249.
Fiane A.E., et al., Xenotransplantation 6: 52-65; 1999.
Finch A.M., et al., J. Med. Chem. 42: 1965-1974; 1999.
Flanagan M.E., et al., J Med Chem. 53:8468-8484; 2010.
Fleili-Hariri M., et al., Diabetes 48: 2300-2308; 1999.
Fleischmann R., et al., N. Engl. J. Med. 367: 495-507; 2012a.
Fleischmann R., et al., Arthritis Rheum. 64: 617-629; 2012b.
Fletcher J.M., et al., Recent Pat. Inflamm. Allergy Drug Discov. 6: 22-34; 2012.
Francois C., et al., Patent WO2009046198. Freireich E.J., et al. Cancer Chemother. Rep. 50: 219-244; 1966.
Fromen C.A., et al., Proc. Natl. Acad. Sci. USA 112: 488-493; 2015.
Fujita J., et al., Allergy 67 : 744-750 ; 2012.
Furst D.E., et al., J. Rheumatol. 14: 342-347; 1987. Gaffen S.L., Nature Rev. Immunol. 9: 556-567; 2009.
Gagliani N., et al. Nat. Med. 19: 739-746; 2013.
Gale E.A., et al., Lancet, 363: 925-931; 2004.
Galvain S., et al. Current Therap. Res. 60 : 278-294 ; 1999.
Garber K. Nature 507: 418-420; 2014
Garcia Z., et al., Proc. Natl. Acad. Sci. USA 104: 4553-4558; 2007
Geelen T., et al., J. Nanobiotechnology 10 : 37-48 ; 2012.
Gentile P., et al., Int. J. Mol. Sci. 15: 3640-3659; 2014.
Gerard N.P., Gerard C., Curr. Opin. Immunol. 14: 705-708; 2002.
Germer K., et al. Int. J. Biochem. Mol. Biol. 4: 27-40; 2013.
Getts D.R., et al., J. Immunol. 187 : 2405-2417 ; 2011.
Getts D.R., et al., Sci. Transl. Med. 6: 219ra217; 2014.
Getts D.R., et al., Trends Immunol. 36: 419-427; 2015.
Ghoreschi K., et al., J. Immunol. 186: 4234-4243; 2011.
Ghoreishi M., et al., J. Immunol. 182: 6071-6078; 2009.
Gibeon D., Menzies-Gow A.N., Expert Rev. Respir. Med. 6: 423-39; 2012.
Gilbert J.C., et al., J. Control. Release 5: 113-118; 1987.
Gilbreath M.J., et al., J. Immunol. 134 : 3420-3425 ; 1985.
Gogishvili T., et al. Int. Arch. Allergy Immunol. 142: 165-174; 2006.
Gohil U., et al., Global J. Pharmacol. 4 : 19-30 ; 2010.
Gombault A., et al., Frontiers Immunol. 3: article 414; 2013.
Gong C.Y., et al., Int. J. Pharm. 365: 89-99; 2009a.
Gong C.Y., et al., BMC Biotechnol. 9: 8; 2009b.
Grant C.R., et al., Autoimmunity Rev. 14: 105-116; 2015.
Greenberg R.A., et al. Clin. Pediatr. (Phila.) 47: 817-823; 2008.
Griffin M.D., et al., Proc. Natl. Acad. Sci. USA 98: 6800-6805; 2001.
Grit M., et al., Int. J. Pharm. 5: 1-6; 1989.
Gross C.C., Wiendl H., Curr. Opin. Rheumatol. 25: 268-274; 2013.
Grunewald S.M., et al., J. Immunol. 160: 404-4009; 1998.
Gupta P., Bhatia V. Indian J. Pediatr. 75: 1039-1044; 2008.
Hagenaars N., et al., J. Control. Release 144: 17-24; 2010.
Harel-Adar T., et al., Proc. Natl. Acad. Sci. USA 108 : 1827-1832 ; 2011.
Harkin D.W., et al., J. Vasc. Surg. 39: 196-205; 2004.
Harris S. J. Nutr. 135: 323-325, 2005.
Hart P.H., et al., Nat. Rev. Immunol. 11: 584-596; 2011.
Hashimoto M., et al., J. Exp. Med. 207: 1135-1143; 2010.
Hawang T.L., et al., Int. J. Nanomed. 10: 371-385; 2015.
Hegele R.A., et al., Lancet Diabetes Endocrinol. pii: S2213-8587 (13) 70191-8; 2013.
Hegeman M.A., et al., Brit. J. Pharmacol. 163: 1048-1058; 2011.
Hewison M., Mol. Cell. Endocrinol. 321: 103-111; 2010.
Hirst M., Feldman D. Biochem. Biophys. Res. Commun. 105: 1590-1596; 1982a.
Hirst M., Feldman D. Endocrinol. 111: 1400-1402; 1982b.
Hochreiter-Hufford A., Ravichandran K.S., Cold Spring Harb. Perspect. Biol. 5: a008748; 2013.
Hoffmann P.R., et al., J. Immunol. 174 : 1393-1404 ; 2005.
Hofstetter H.H., et al. Cell. Immunol. 237: 123-130; 2005.
Holgate S.T., Polosa R., Nature Rev. Immunol 8: 218-230; 2008.
Holgate S.T., J. Allergy Clin. Immunol. 128 : 495-505 ; 2011.
Huber-Lang M., et al., Am. J. Pathol. 161: 1849-1859; 2002.
Humbles A.A., et al., Nature 406: 998-1001; 2000.
Hyppönen E., et al. Lancet 358: 1500-1503; 2001.
Hyun H., et al., Biomacromolecules 8: 1093-1100; 2007.
Idoyaga J., et al. J. Clin. Invest. 123 : 844-854 ; 2013.
Imaizumi T., et al. J. Toxicol. Sci. 21 (supplement II): 277-285; 1996.
Ishii M., et al. Intern. Immunopharmacol.10: 1041-1046; 2010.
Jacobsen L., et al., Allergy 62: 943-948; 2007.
Jaeckel E., et al., Diabetes 54 : 306-310 ; 2005.
Jeffery L.E., et al. J. Immunol. 183: 5458-5467; 2009.
Jenkins, M.K., Schwartz, R.H., J. Exp. Med. 165 : 202-319 ; 1987.
Jeong B., et al., Nature 388: 860-862, 1997.
Jiang H.-R., et al., Eur. J. Immunol. 42: 1804-1814; 2012.
Jilek S., et al., J. Immunol. 166 : 3612-3621 ; 2001.
Jilek S., et al., J. Allergy Clin. Immunol. 114: 943-950; 2004.
Jilek S., et al., Pharm. Res. 21: 1240-1247; 2004b.
Jones G. Am. J. Clin. Nutr. 88(suppl.): 582S-586S; 2008.
Jones S.A., et al. Nature Immunol. 13: 1022-1025; 2012.
Josefowicz S.Z., et al. Annu. Rev. Immunol. 30: 531-564; 2012.
Joshi V.B., et al., Am. Assoc. Pharmaceut. Sci. J. 15 : 85-94 ; 2013.
Kang Y.M., et al., Biomaterials 31: 2453-2460; 2010.
Karagiannidis C., et al. J. Allergy Clin. Immunol. 114 : 1425-1433 ; 2004.
Karaman M.W., et al., Nat. Biotechnol. 26: 127-132; 2008.
Katragadda M., et al., . Med. Chem. 49: 4616-4622; 2006.
Kawamoto S., et al., J. Clin. Invest. 114: 399-407; 2004.
Kearley J., et al., J. Exp. Med. 202: 1539-1547; 2005.
Keefe A.D., et al., Nature Rev. Drug Discov. 9 : 537-550 ; 2010.
Keijzer C., et al., PLoS one 6 (11): e26684; 2011.
Keijzer C., et al., J. Control. Release 168: 35-40; 2013.
Kelchtermans H., et al. Ann. Rheum. Dis. 68: 744-750; 2009.
Kensil C.R., Dennis E.A., Biochemistry 20: 6079-6085; 1981.
Kim W.-U., et al., Arthritis Rheum. 46: 1109-1120; 2002.
Kimball S.M., et al. Am. J. Clin. Nutr. 86: 645-651; 2007.
Kioi M., et al., Cell. Immunol. 229: 41-51; 2004.
Kirby C., Gegoriadis G., Biochem. J. 199: 251-252; 1981.
Kissmeyer A.-M., Binderup, L., Biochem. Pharmacol.41: 1601-1606; 1991.
Köhl J., et al., J. Clin. Invest. 116: 783-796; 2006.
Köhl J., Curr. Opin. Mol. Ther. 8: 529-538; 2006b.
Koenders M.I., et al., Arthritis Rheum. 52: 3239-3247; 2005.
Koffeman E.C., et al., Arthritis Rheum. 60: 3207-3216; 2009.
Kohm A. P., et al., J. Immunol. 169, 4712-4716; 2002.
Komai-Koma M., et al., Eur J Immunol. 2007;37:2779-86; 2007.
Komiyama Y., et al. J., Immunol. 177: 566-573; 2006.
Kondo C., et al., Clin. Exp. Immunol. 124: 323-329; 2001.
Kong N., et al., Arthritis Rheum. 64: 2548-2558; 2012.
Kono H., Rock, K.L., Nature Rev. Immunol. 8 : 279-289 ; 2008.
Konteatis Z.D., et al., J. Immunol. 153: 4200-4205; 1994.
Koop E., Ghosh S., Science 265: 956-959; 1994.
Koppolu B., Zaharoff D.A., Biomaterials 34: 2359-2369; 2013.
Kornbluth R.S., Immunol. Lett.43 : 125-132 ; 1994.
Kragballe K., Pharmacol. Toxicol. 77: 241-246; 1995.
Kremer J., et al., Arthritis Rheum. 60: 1895-1905; 2009.
Kretschmer K., et al., Nat. Immunol. 6: 1219-1227 ; 2005.
Kruse N., et al., EMBO J. 11: 3237-3244; 1992.
Kruse N., et al., EMBO J. 12: 5121-5129; 1993.
Kubo S., et al., Ann. Rheum. Dis. 73: 2192-2198; 2014.
Kurowska-Stolarska M., et al., J Immunol. 181:4780-90; 2008.
Kwak H.W., D'Amico D.J. Korean J. Ophthalmol. 9 : 79-83 ; 1995.
Kyger M., et al., Trans. Vis. Sci. Tech. 2013 ; 2 : article 1.
Landewe R.B.N., et al. Ann. Rheum. Dis. 69: 1655-1659; 2010.
Lappegard K.T., et al., Ann. Thorac. Surg. 79: 917-923; 2005.
Lappegard K.T., et al., J. Biomed. Mater. Res. A87: 129-135; 2008.
Law S.C., et al., Arthritis Res Ther 14:R118; 2012.
Lee H., et al., Immunol. Cell Biol. 86 : 153-160 ; 2008.
Lee H.Y., et al., Exp. Lung Res. 40: 66-76; 2014.
Lenmark A., Larsson H.E., Nature Rev. Endocrinology 9: 92-103; 2013.
Leroux-Roels, G., Vaccine 285: C25-C36; 2010.
Letzelter F., et al., Eur. J. Biochem. 257: 11-20; 1998.
Longui C.A., J. Pediatr. (Rio J.) 83: S163-171; 2007.
Lozano, R. et al., Lancet 380: 2095-128; 2012.
Lubberts E., et al., J. Immunol. 167: 1004-1013; 2001.
Lubberts E., Curr. Opin. Investig. Drugs 4: 572-7; 2003.
Lubberts E., et al. Arthritis Rheum. 50: 650-659; 2004.
Ludvigsson J., et al. N. Engl. J. Med. 359: 1909-1910; 2008.
Ludvigsson J., et al., N. Engl. J. Med. 366: 433-442; 2012.
Lutterotti, A., et al., Sci. Translat. Med. 5 : 1-19 ; 2013.
Ma Q., et al. Bone Marrow Transplantation 49: 972-976; 2014.
Machen J., et al. J. Immunol. 173: 4331-4341; 2004.
Mackern-Oberti J.P., et al., Autoimmun. Rev. 14: 127-139; 2015.
Maeshima K., et al., Arthritis Rheum. 64: 1790-1798; 2012.
Mahon B.D., et al., J. Cell. Biochem. 89: 922-932; 2003.
Majak P., et al. J. Allergy Clin. Immunol. 127 : 1294-1296 ; 2011.
Makadia H.K,, Siegel S.J., Polymers (Basel) 3: 1377-1397; 2011.
Mantel P.-Y. et al., PLoS biology 5, e329: 2847-2861; 2007.
Mao S., et al., Int. J. Pharm. 334 : 137-148 ; 2007.
March D.A., et al., Mol. Pharmacol.65 : 868-879 ; 2004.
Markiewski M.M., et al., Nat. Immunol. 9 : 1225-1235 ; 2008.
Martin E., et al., Arthritis Rheum. 56: 2255-2266; 2007.
Masson-Bessiere C., et al., J Immunol 166:4177-4184; 2001.
Matsuda R., et al., Invest. Ophthalmol. Vis. Sci. 53: 7235-7245; 2012.
Matsuno R., et al., Biochem. Biophys. Res. Commun. 281: 614-620; 2001.
Mazzeo D., et al., Eur. J. Immunol. 28: 3205-3213; 1998.
Meechan P., et al. Int. Arch. Allergy Immunol. 61 : 351-362 ; 2013.
Merz K., Sternberg B., J. Drug Target. 2: 411-417; 1994.
Metselaar J.M., et al., Arthritis Rheum. 48: 2059-2066; 2003.
Meyer D.M., et al., J. Inflamm. (Lond) 7:41; 2010.
Meyer C., et al., J. Nucleic Acids, Article ID 904750; 2011.
Miossec P., Arthritis Rheum. 48: 594-601; 2003.
Miossec P., Kolls J.K., Nature Rev. Drug Discovery 11: 763-776; 2012.
Mizutani N., et al., J. Immunol. 188: 5694-5705; 2012.
Mizutani N., et al., J. Immunol. 192: 1372-1384; 2014.
Moller C., et al., J. Allergy Clin. Immunol. 109: 251-256; 2002.
Mollnes T.E., et al., Trends Immunol. 23: 61-64; 2002.
Monastra G., Bruni, A., Lymphokine Cytokine Res. 11 : 39-43 ; 1992.
Monastra G., et al., Neurology 43 : 153-163 ; 1993.
Morgan B.P., Gasque P., Clin. Exp. Immunol. 107: 1-7 ; 1997.
Morgan M.E., et al., Arthritis Rheum. 52: 2212-2221; 2005.
Morikis D., et al., Protein Science 7: 619-627; 1998.
Motomura Y., et al. Nat. Immunol. 12: 450-459; 2011.
Mottet C., et al., J. Immunol. 170: 3939-3943; 2003.
Muller U., et al., J. Allergy Clin. Immunol. 101:747-754; 1998.
Muindi J.R., et al. Oncology 66: 62-66; 2004.
Muller T., et al., J. Mol. Biol. 237 : 423-436, 1994.
Nakae S., et al. J. Immunol. 171: 6173-7; 2003.
Nakano Y., et al., J. Allergy Clin. Immunol. 112: 525-530; 2003.
Nashold F.E., et al. J. Neuroimmunol. 263: 64-74; 2013.
Nicholls, E.F., et al., Ann. N.Y. Acad. Sci. 1213: 46-61; 2010.
Nicolete R., et al., Int. Immunopharmacol. 11 : 1557-1563 ; 2011.
Nie S., et al., Internatl. J. Nanomed. 6: 151-166; 2011.
Nilsson B., et al., Blood 92: 1661-1667; 1998.
Obst R., et al., Proc. Natl. Acad. Sci. USA 104: 15460-15465; 2007.
Oda N., et al., Am. J. Respir. Crit. Care Med. 171 : 12-18 ; 2005.
Okamura T.; et al. Proc. Natl. Acad. Sci. USA 106: 13974-13979; 2009.
Ota K., et al., J. Immunol. Res., Article ID 602846 ; 2014.
Otsuka M., et al., Biochem. Biophys. Res. Commun. 324: 1400-1405; 2004.
Palomares O., et al., Eur. J. Immunol. 40: 1232-1240; 2010.
Papenfuss T.L., et al., J. Immunol. 186: 3346-3355; 2011.
Park T.G., Biomaterials 16: 1123-1130; 1995.
Park M.V., et al., Biomaterials 32: 9810-9817; 2011.
Peng K.-T., et al., Biomaterials 31: 5227-5236; 2010.
Peng Q., et al., J. Immunol. 176: 3330-3341; 2006.
Petersen B.C., Lukacz N.W., Future Med. Chem. 4: 833-836; 2012.
Pichler J., et al., Pediatr. Res. 52: 12-18; 2002.
Plum L.A., et al. Proc. Natl. Acad. Sci. USA 1001: 6900-6904; 2004.
Plum L.A., DeLuca H.F., Nat. Rev. Drug Discov. 9: 941-955; 2010.
Polosa R., Casale T., Drug Discovery Today 17: 591-599; 2012.
Ponzin D., et al., Immunopharmacol. 18 : 167-176 ; 1989.
Pot C., et al. J. Immunol. 183: 797-801; 2009.
Pratt J.R., et al., Nat. Med. 8: 582-587 ; 2002.
Proctor L.M., et al., Brit. J. Pharmacol. 142: 756-764; 2004.
Qiao M. et al., Int. J. Pharm. 294: 103-112; 2005.
Qu H., et al., Mol. Immunol. 47: 185-195; 2009.
Qureshi F., et al. J. Pediatr. 139 : 20-26 ; 2001.
Raedler H., et al., Am. J. Transplant. 9: 1784-1795; 2009.
Raghuvanshi R.S., et al., Int. J. Pharm. 245: 109-121; 2002.
Raich-Regué D., et al., Eur. J. Immunol. 42:771-782; 2012.
Rank M.A., et al., J Allergy Clin Immunol. 123:1047-54; 2009.
Ravichandran K.S., Immunity 35 : 445-455 ; 2011.
Raz I, et al., Lancet 358: 1749-1753; 2001.
Reichel H., et al., Proc. Natl. Acad. Sci. USA 84: 3385-3389; 1987.
Rhen T., Cidlowski J.A., N. Engl. J. Med. 353 : 1711-1723 ; 2005.
Ricklin D., Lambris J.D., Adv. Exp. Med. Biol. 632: 273-292; 2008.
Ricklin D., et al., Nat. Immunol. 11: 785-797; 2010.
Robinson D.S., J. Allergy Clin. Immunol. 126: 1081-1091; 2010.
Roep B. O., et al., Sci. Transl. Med. 5: 191ra82; 2013.
Rolland J.M., et al., Pharmacol. Ther. 121: 273-284; 2009.
Rouser G., et al. Lipids 5: 494-496; 1970.
Ruel-Gariepy, E., Leroux, J-C., Eur. J. Pharmaceutics Biopharmaceutics 58: 409-426; 2004.
Sabatos-Peyton C. A., et al. Curr. Opin. Immunol. 22: 609-615; 2010
Sahu A., et al., J. Immunol. 157: 884-891; 1996.
Scalapino K.J., et al., J. Immunol. 177: 1451-1459; 2006.
Schatz D.A., Bingley PJ., J. Ped. Endocrinol. Metabol. 14 (Suppl. 1): 619-622; 2001.
Schloot NC, et al., Diabetes Metab. Res. Rev. 23: 276-285; 2007.
Schmidt S., et al., J. Biomed. Mater. Res. A66: 491-499; 2003.
Schmidt-Weber C., Blaser K., Curr. Opin. Allergy Clin. Immunol. 5: 525-530; 2005.
Schmidt-Weber C., Blaser K., Inflamm. Allergy Drug Targets 5: 15-21; 2006.
Schmitz J., et al., Immunity 23:479-90; 2005.
Schnatbaum K., et al., Bioorg. Med. Chem. Lett. 16: 5088-5092; 2006.
Schöll I., et al., Clin. Exp. Allergy 34: 215-321; 2004.
Schweingruber N., et al., J. Immunol. 187: 4310-4318; 2011.
Scott D.L., et al., Lancet 376: 1094-108; 2010.
Seeger M., et al., Eur. J. Immunol. 28 : 2124-2130 ; 1998.
Shah SC, et al., N. Engl. J. Med., 320: 550-554; 1989.
Shakhar G., et al., Nat. Immunol. 6: 707-714; 2005.
Shephard R.M., DeLuca H.F. Arch. Biochem. Biophys. 202: 43-53; 1980.
Shi D., et al., Arch. Dermatol. Res. 299: 327-336; 2007.
Shigdar S., et al., Sensors 13: 13624-13637; 2013.
Singh D.K., Verma R. Iranian J. Pharmacol. Ther. 7: 61-65; 2008.
Sinha V.R., et al., Int. J. Pharm. 278: 1-23; 2004.
Skyler J.S., et al., Diabetes Care, 28: 1068-1076; 2005.
Smithgall M.D., et al., Int Immunol. 20:1019-30; 2008.
Snir O., et al., Arthritis Rheum 63:2873-2883; 2011.
Sokolove J., et al., PLoS One 2012, 7:e35296.
Soulika A.M., et al., Clin. Immunol. 96: 212-221; 2000.
Stoop J.N., et al., Arthritis Rheum. 62: 3656-3665; 2010.
Strainic M.G., et al., Immunity 28: 425-435; 2008.
Strainic M.G., et al., Nat. Immunol. 14: 162-171; 2013.
Sumichika H., et al., J. Biol. Chem. 277: 49403-49407; 2002.
Tagasugi Y., et al., Pharmacol. Pharmacy 4 : 248-254 ; 2013.
Taher Y.A., et al. J. Immunol. 180: 5211-5221; 2008.
Tamachi T., et al., J. Allergy Clin. Immunol. 118: 606-614; 2006.
Tan, L.J., et al., J. Immunol. 147 : 1797-1802 ; 1991.
Tang C., et al., Immunology. 135: 112-124; 2012.
Tang J., et al., J. Immunol. 182: 4624-4632; 2009.
Tang Q., et al., J. Exp. Med. 199, 1455-1465; 2004.
Tang W., et al., Int. Arch. Allergy Immunol. 63: 5-10; 2014.
Thakker P., et al., J. Immunol. 178: 2589-2598; 2007.
Thiel K.W., Giangrande P.H. Oligonucleotides 19: 209-222; 2009.
Thiele L., et al., Pharm. Res. 20: 221-228; 2003.
Thomas R., et al., Arthritis Rheum. 63: 2430; 2011.
Thomas R., Arthritis Res. Therapy 15: 204-214; 2013.
Thommesen L., Laegreid A., J. Biochem. Mol. Biol. 38: 281-289; 2005.
Thurau S.R., et al., Ann. N.Y. Acad. Sci. 1029: 408-412; 2004.
Ting E., et al., Br. J. Pharmacol. 153: 1043-1053; 2008.
Tjernberg J., et al., Transplantation 85: 1193-1199; 2008.
Tony H.P., et al., Eur. J. Biochem. 225: 659-665; 1994.
Toscano M.G., et al., Mol. Ther. 18: 1035-1045; 2010.
Trémezaygues L., Reichrath J. Dermatoendocrinol. 3: 180-186; 2011.
Tsark E.C., et al., J Immunol 169:6625-6633; 2002.
Tsvetkova-Vicheva V., et al., Israel Med. Assoc. J. 16: 358-362; 2014.
Unger W.W.J., et al., Eur. J. Immunol. 39: 3147-59; 2009.
Urry Z., et al., J. Clin. Invest. 119: 387-398; 2009.
Uyttenhove C., Van Snick J., Eur. J. Immunol. 36: 2868-2874; 2006.
van Brussel I., et al., Autoimmun. Rev. 13: 138-150; 2014.
van der Aar A.M.G., et al., J. Allergy Clin. Immunol. 127: 1532-1540; 2011.
van der Helm-van Mil A.H.M., et al., Arthritis Rheum 56:425-432; 2007.
van Duivenvoorde L.M., et al., J. Immuol. 179: 1506-1515; 2007.
Verschoor A., et al., J. Immunol. 171: 5363-5371; 2003.
Villadangos J.A., Schnorrer P. Nat. Rev. Immunol. 7 : 543-555 ; 2007.
Voll, R.E., et al., Nature 390 : 350-351 ; 1997.
von Delwig A., et al., Arthritis Rheum 62:143-149; 2010.
von Garnier C., et al., Eur. J. Immunol. 30 : 1638-1645 ; 2000.
Vossenaar E.R., Van Venrooij W.J., Arthritis Res Ther 6:107-111; 2004.
Wacker W.B., et al., J. Immunol. 119: 1949-1958; 1977.
Wagner E., Frank M.M., Nature Rev. 9: 43-56; 2010.
Walczak A., et al., J. Am. Med. Assoc. Neurol. 70: 1105-1109; 2013.
Waters S.M., et al., J. Biol. Chem. 280: 40617-40623; 2005.
Wenzel S., et al., Lancet 370: 1422-1431; 2007.
Wherrett D.K., et al., Lancet 378: 319-327; 2011.
Wherry E.J. Nat. Immunol. 12: 492-499; 2011.
White R.R., et al. J. Clin. Invest. 106: 929-934; 2000.
Wingerchuk D.M., et al. J. Neurol. Neurosurg. Psychiatry 76: 1294-1296; 2005.
Woodruff T.M., et al., J. Pharmacol. Exp. Ther. 314: 811-817; 2005.
Woodruff T.M., et al., FASEB J. 20: 1407-1417; 2006.
Worm M., et al., J. Allergy Clin. Immunol. 127 : 89-97 ; 2011.
Wu Y., et al., Biomaterials 33: 2351-3260; 2012.
Xia T., et al., ACS Nano 2: 85-96; 2008.
Xiang S.D., et al., Methods 60: 232-241; 2013.
Xiao B.C., et al., Int. Immunol. 16: 13-22; 2004.
Xing N., et al., Biochem. Biophys. Res. Commun. 297: 645-652; 2002.
Xing Y., et al., J. Liposome Res., early online 1-7 ; 2013.
Xu W., et al., PLOS ONE 7 (7): e40314, doi:10.1371/ journal.pone.00401314; 2012.
Yadav K., et al., Frontiers Immunol. 4: 232; 2013.
Yamazaki S., et al., J. Exp. Med. 198: 235-247; 2003.
Yao X.J., et al., Clin. Exp. Allergy 44: 765-777; 2014.
Yoo S.-A. et al., J Exp Med 209: 871-886; 2012.
Zaharoff, D.A., et al., Vaccine 25: 2085-2094; 2007.
Zella J.B., et al. Arch. Biochem. Biophys. 417: 77-80; 2003.
Zhang J., et al., Biomacromolecules 7: 2492-2500; 2006.
Zhang H., et al., J. Translat. Med. 12: 125; 2014.
Zhou J., Frontiers Genetics 3 : Article 234 ; 2012.
Zhou W., Immunobiology 217: 225-234; 2012b.
Zhu S., Qian Y., Clin. Sci. (Lond) 122: 487-511; 2012.

## Claims

1. Pharmaceutical composition for subcutaneous allergen- or autoantigen-specific immunotherapy of allergic and autoimmune diseases using matrix-based compositions for subcutaneous administration comprising matrix-embedded allergens or autoantigens or fragments thereof, one or more tolerance-promoting adjuvants, optionally one or more low molecular weight find-me molecules, and optionally one or more tolerance-promoting immune modulators suitable for enhancing the suppressive function of regulatory T cells and/or inhibiting the production of pro-inflammatory cytokines, and/or inhibiting the biological activity of secreted pro-inflammatory cytokines at the site of antigen or allergen presentation.

2. Composition of claim 1, wherein the matrix suitable for locally restricted sustained release of embedded therapeutics is selected from alum-, micro crystalline tyrosine-; or hydrogel-based formulations, wherein preferred hydrogel-based matrices are selected from the group consisting of polyethylene, polypropylene, polyethylene oxide (PEO), polypropylene oxide (PPO), polyurethane, polyurea, polyamides, polycarbonates, polyaldehydes, polyorthoesters, polyiminocarbonates, poly caprolactone (PCL), poly-D,L-lactic acid (PDLLA), poly-L-lactic acid (PLLA), lactides of said lactic acids, polyphosphazenes, polyglycolic acids, albumin, monomethoxypoly(ethylene glycol) (MPEG), trimethylated chitosan derivatives, or copolymers or mixtures of any of the above including poly(lactic-co-glycolic acid) (PLGA), copolymers of L-lactide and D,L-lactide, polyester copolymers, diblock copolymers consisting of MPEG and PCL, MPEG and PCL-ran-PLLA, MPEG and PLGA, PEO and PLLA, trimethylated chitosan and α,β-glycerophosphate, triblock copolymers consisting of PEO and PLLA, PLGA-PEG-PLGA, PEG-PLGA-PEG, PEG-PCL-PEG, and PEO-PPO-PEO (Poloxamers), wherein preferred hydrogel-based matrices are biodegradable or biostable polymers, more preferably biodegradable, even more preferably thermogelling, and most preferably reverse thermogelling, and wherein the gelling temperature is between 20°C and 40°C, preferably between 25°C and 35°C, and/or wherein more than 50% degradation of the polymer weight in body environment and/or more than 50% release of embedded tolerance-promoting adjuvants and/or tolerance-promoting immune modulators from the polymer is completed within 1 to 10 days, preferably within 1 to 5 days.

3. Composition of claim 1, wherein matrix-embedded tolerance-promoting adjuvants are selected from poly(lactic-co-glycolic acid) micro- and nanoparticles (PLGA particles) and phosphatidyl-L-serine-containing liposomes (PS-liposomes), wherein preferred tolerance-promoting adjuvants contain one or more encapsulated low molecular find-me molecules for enhanced peripheral phagocytosis of said tolerance-promoting adjuvants, and wherein most preferred tolerance-promoting adjuvants allow the sustained release of encapsulated low molecular find-me molecules within a period of 2 to 5 days.

4. Composition of claim 1, wherein low molecular find-me molecules for enhanced peripheral phagocytosis are selected from lysophosphatidylcholine (LPC), sphingosine-1-phosphate (S1P) and the nucleotides ATP and UTP, wherein preferred low molecular find-me molecules are ATP and/or UTP, wherein said low molecular find-me molecules are embedded in a matrix according to claim 2, preferably as PLGA-encapsulated or PS-liposome-encapsulated formulation.

5. Composition of claim 1, wherein tolerance-promoting immune modulators include but are not limited to a) vitamin D3 and selected analogs such as calcipotriol, b) glucocorticoids such as dexamethasone phosphate, c) Janus kinase (JAK) inhibitors such as tofacitinib, d) antagonistic cytokine molecules such as Il-4/IL-13 muteins, e) salicylate-based therapeutics for the inhibition of TNFR1-mediated pathways, f) peptide- or peptidomimetic-based complement inhibitors, and g) aptamer-based inhibitors of pro-inflammatory cytokines, wherein preferred tolerance-promoting immune modulators are low to medium molecular weight immune modulators which provide a relatively short serum half-life that is sufficient to be locally active upon their release from a depot at the site of autoantigen or allergen presentation, and which allows fast removal from circulation upon diffusion and transport away from the site of autoantigen or allergen presentation.

6. Composition of claim 1, wherein matrix-embedded allergens include allergen extracts, cross-linked allergen extracts (allergoids), depigmented allergoids, one or more purified natural allergens, one or more recombinant allergens or derivatives thereof, and one or more fragments of natural or recombinant allergens, wherein matrix-embedded autoantigens include one or more purified natural autoantigens, one or more recombinant autoantigens or derivatives thereof, and one or more fragments of natural or recombinant autoantigens.

7. Composition according to any of the claims 1 to 6, wherein all components are mixed as a single preparation comprising the matrix according to claim 2, one or two therapeutically effective doses of tolerance-promoting adjuvants according to claim 3, one or more therapeutically effective doses of low molecular find-me molecules for enhanced peripheral phagocytosis according to claim 4, one or more therapeutically effective doses of tolerance-promoting immune modulators according to claim 5, and one or more therapeutically effective doses of allergen or autoantigen according to claim 6, wherein the composition is galenically prepared for administration by injection or by implantation, intradermally, subcutaneously, nasally, transbucally, transmucosally, sublingually, intraocularly, intramus-cularly, or topically.

8. Composition according to claim 1, comprising matrix-embedded allergens or autoantigens or fragments thereof, wherein the tolerance-promoting adjuvant is PLGA-particles,
wherein the low molecular weight find-me molecule is ATP or UTP , and wherein the tolerance-promoting immune modulator is calcipotriol.

9. Composition according to claim 1, comprising matrix-embedded allergens or autoantigens or fragments thereof, wherein the tolerance-promoting adjuvant is PLGA-particles, wherein the low molecular weight find-me molecule is ATP or UTP , and wherein the tolerance-promoting immune modulator is dexamethasone phosphate.

10. Composition according to claim 1, comprising matrix-embedded allergens or autoantigens or fragments thereof, wherein the tolerance-promoting adjuvant is phosphatidyl-L-serine-containing liposomes (PS-liposomes),wherein the low molecular weight find-me molecule is ATP or UTP , and wherein the tolerance-promoting immune modulator is tofacitinib.

11. Use of a composition according to one of the claims 1 to 10 for modulation of antigen-presenting cell, T cell and B cell responses by allergen- aor autoantigen-specific immunotherapy in a mammalian organism, preferably a human, in need thereof, in particular for the treatment of T cell-mediated diseases, preferably selected from the group consisting of allergy, allergic asthma, type 1 diabetes, rheumatoid arthritis, and multiple sclerosis, autoimmune uveitis and wherein the pharmaceutical composition is administered in a therapeutically effective dose.

12. Methods for manufacturing a pharmaceutical composition according to one of the claims 1 to 10, wherein the components are mixed with each other in a therapeutically effective quantity, wherein optionally galenic compounds are additionally admixed to one or all of the preparations.
